# EUROPEAN PATENT SPECIFICATION

(11) **EP 3 385 361 B1**
(45) Date of publication and mention of the grant of the patent: **27.03.2019**
(21) Application number: 17164901.5
(22) Date of filing: 05.04.2017
(51) Int. Cl.: C11D 3/386

(54) **DETERGENT COMPOSITIONS COMPRISING BACTERIAL MANNANASES**
WASCHMITTELZUSAMMENSETZUNGEN MIT BAKTERIELLEN MANNANASEN
COMPOSITIONS DÉTERGENTES COMPRENANT DES MANNANASES BACTÉRIENNES

(43) Date of publication of application: 10.10.2018
(73) Proprietor: Henkel AG & Co. KGaA, 40589 Düsseldorf (DE)
(72) Inventor: HERBST, Daniela, 40237 Düsseldorf (DE); WIELAND, Susanne, 41541 Zons/Dormagen (DE); MUßMANN, Nina, 47877 Willich (DE); LEINONEN, Taija, 11100 Riihimäki (FI); VALTAKARI, Leena, 05200 Rajamäki (FI); RACHINGER, Michael, 63762 Großostheim (DE); JUNTUNEN, Kari, 02680 Espoo (FI); DOLLAK, Daniela, 64589 Stockstadt a. Rhein (DE); LORENZ, Patrick, 64653 Lorsch (DE); VEHMAANPERÄ, Jari, 00980 Helsinki (FI); OJAPALO, Pentti, 04360 Tuusula (FI); PURANEN, Terhi, 01900 Nurmijärvi (FI); JÄRVINEN, Kristiina, 02940 Espoo (FI)

(56) References cited:
- WO-A2-99/64619
- DHAWAN SAMRITIL ET AL: "Microbial mannanases: An overview of production and applications", CRC CRITICAL REVIEWS IN BIOTECHNO, CRC PRESS, BOCA RATON, FL, US, vol. 27, no. 4, 1 January 2007 (2007-01-01), pages 197-216, XP009161462, ISSN: 0738-8551, DOI: 10.1080/07388550701775919

## Description

### FIELD OF THE INVENTION

This invention relates to novel detergent compositions comprising bacterial mannanase enzymes. The detergent compositions comprising bacterial mannanases are useful in laundry and cleaning applications wherein degradation or modification of mannan is desired. The invention also relates to the use of said detergent compositions in laundry and cleaning applications as well as a method for degrading mannan.

### BACKGROUND

Mannans are mannose containing polysaccharides found in various plants. Mannans are poorly soluble in an aqueous environment and their physicochemical properties give rise to viscous dispersions. Additionally, mannans have high water-binding capacity. All of these characteristics cause problems in several industries including brewing, baking, animal nutrition, and laundry and cleaning applications.

In plant based diets different β-mannans are present and depending on their amounts and properties they can compromise nutrient digestion, microbial colonisation and growth performance. Enzymatic degradation of mannans reduces digesta viscosity of high water soluble mannans and leads to production of manno-oligosaccharides that may form water-insoluble linear mannans present in leguminoseae. Mannanase increases average daily gain, feed efficiency, weight uniformity and livability in all monogastric animals.

For animal feed applications, such as feed for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiencies.

For laundry and cleaning applications detergent compositions comprising mannanase can be used to degrade mannan. However, providing mannanases that are stable in varying storage and use conditions while still showing good mannan degrading activity is difficult.

WO 99/64619 A2 discloses and characterizes several mannanases and their presumed possible use in several industry applications.

It is an object of the present invention to provide detergent compositions comprising novel enzymes exhibiting mannanase activity when applied in these detergent compositions. It is a further object of the present invention to provide detergent compositions having increased stain removal performance on mannan containing stains.

### SUMMARY

According to the first aspect of the invention there is provided a detergent composition comprising at least one enzyme having an amino acid sequence having at least 74% sequence identity to the amino acid sequence of SEQ ID NO: 16 (Man7), wherein the at least one enzyme has mannan degrading activity.

In one embodiment of the invention the at least one enzyme has an amino acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to SEQ ID NO: 16.

The mannanases comprised in the detergent composition of the invention are suitable for degrading and modifying mannan containing material in various chemical environments, preferably in detergent compositions.

In one embodiment of the present invention the detergent composition further comprises one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase, preferably selected from the group consisting of proteases, amylases, cellulases and lipases.

In a further embodiment of the present invention the detergent composition is in form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition.

The present invention furthermore relates to the use of the detergent composition as herein disclosed for degrading mannan.

In a further embodiment the present invention relates to the use of the detergent composition as herein disclosed in a laundry process.

The present invention furthermore relates to a method for removing a stain from a surface, comprising contacting the surface with a detergent composition as herein disclosed.

The present invention also relates to a method for degrading mannan comprising applying a detergent composition as herein disclosed to mannan, preferably wherein the mannan is on the surface of a textile.

### BRIEF DESCRIPTION OF THE FIGURES

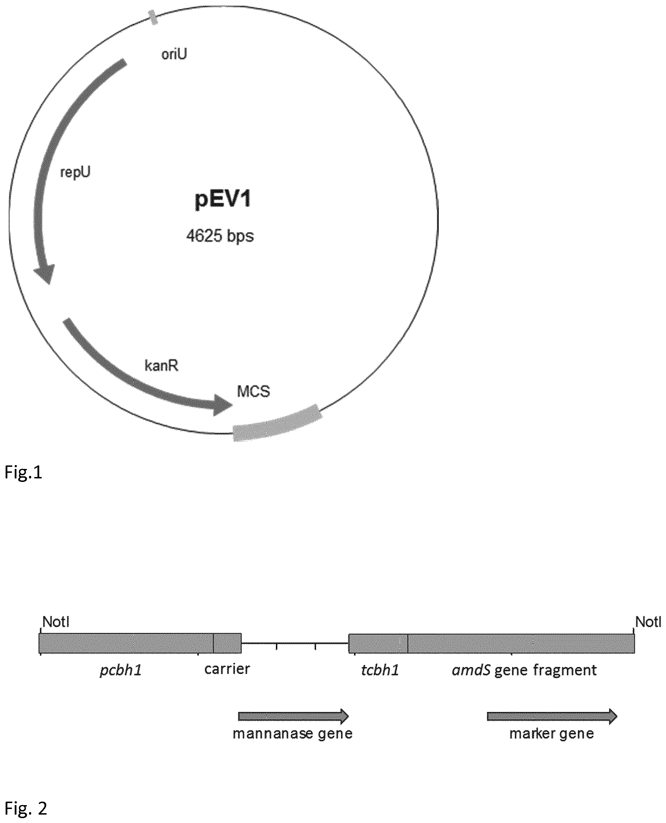

**Fig 1** shows schematic representation of vector pEV1 for replication in *Bacillus*
**Fig 2** schematically shows the expression cassettes used in the transformation of *Trichoderma reesei* protoplasts for overproducing the recombinant mannanase protein Man7 according to the present invention as well as further recombinant mannanase proteins (Man6 and Man14). The mannanase genes were under the control of *T. reesei cel7A*/*cbh1* promoter (p*cbh1*) and the termination of the transcription was ensured by using *T. reesei cel7A*/*cbh1* terminator sequence (t*cbh1*)*.* The amdS gene was included as a transformation marker.

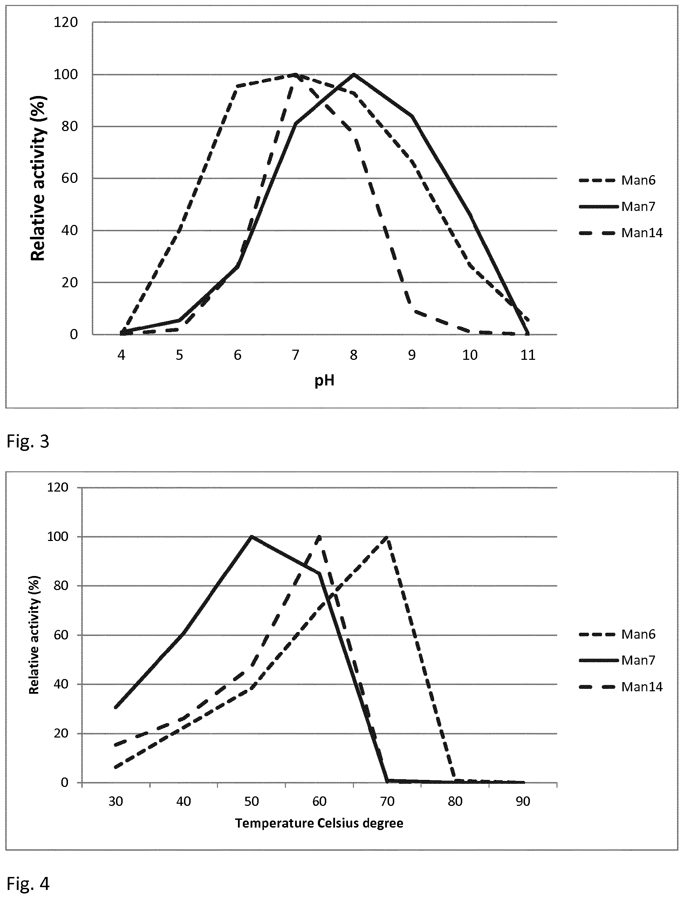

**Fig 3** shows the temperature profile of recombinant Man6, Man7 and Man14 (*Bacillus* produced) mannanase assayed in 40 mM Britton-Robinson buffer pH 7 using 10 min reaction time, Azurine-crosslinked carob galactomannan was used as a substrate. All measurements were made at least duplicates. The data points are averages of separate measurements.
**Fig 4** describes the effect of pH on the activity of recombinant Man6, Man7 and Man14 (*Bacillus* produced) mannanase protein in 40 mM Britton-Robinson buffer at pH 4 to pH 11. Reaction temperature was 50°C and the reaction time was 10 min. Azurine-crosslinked carob galactomannan was used as a substrate. All measurements were made at least duplicates. The data points are averages of separate measurements.

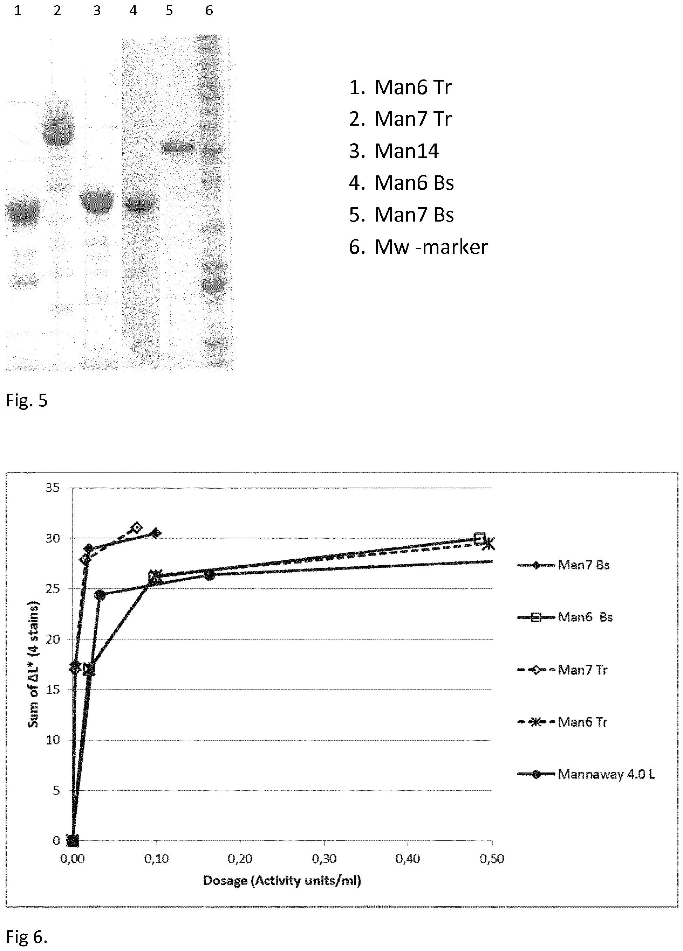

**Fig 5** shows SDS PAGE analysis of bacterial mannanases.
**Fig 6** describes the stain removal performance of Man 6 and Man7 (produced in *Bacillus* and *Trichoderma*) as an increase of lightness (sum of ΔL*of 4 stains) in the presence of 4,4 g/l of Commercial heavy duty liquid detergent A at 40°C,16 °dH, 60 min, pH approx. 8.3 and enzymes dosed as activity units. Commercial preparation Mannaway® 4,0 L was used for comparison.

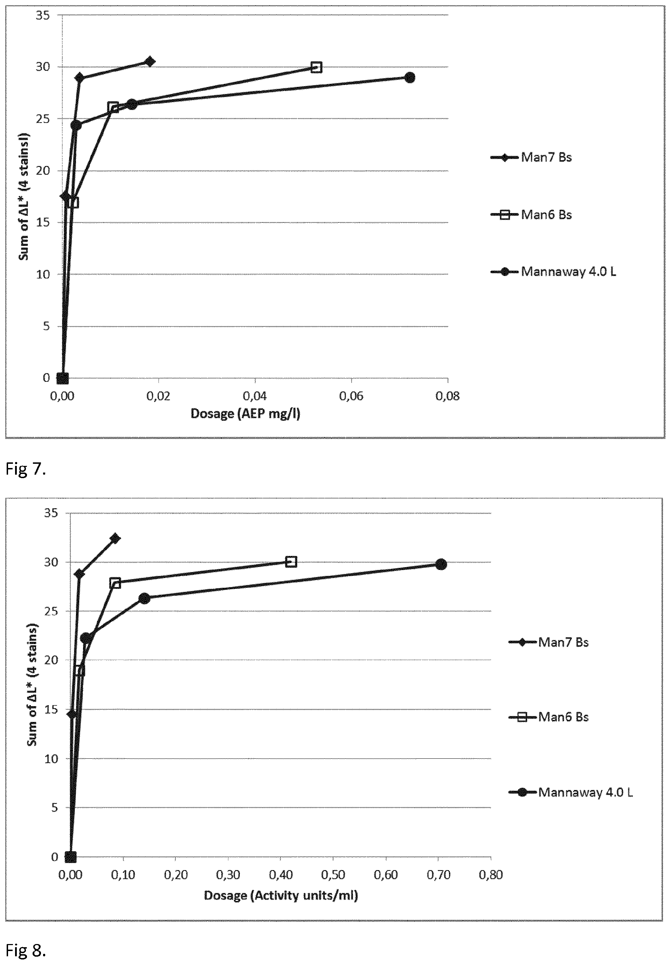

**Fig 7** describes the stain removal performance of Man 6 and Man7 (produced in *Bacillus)* as an increase of lightness (sum of ΔL*of 4 stains) in the presence of 4.4 g/l of Commercial heavy duty liquid detergent A at 40°C, 16 °dH, 60 min, pH approx. 8.3 and enzymes dosed as active enzyme protein (AEP). Commercial preparation Mannaway® 4,0 L was used for comparison.
**Fig 8** describes the stain removal performance of Man 6 and Man7 (produced in *Bacillus)* as an increase of lightness (sum of ΔL*of 4 stains) in the presence of 3.8 g/l of Commercial color detergent powder at 40°C, 16 °dH, 60 min, pH approx. 10. and enzymes dosed as activity units. Commercial preparation Mannaway® 4,0 L was used for comparison.

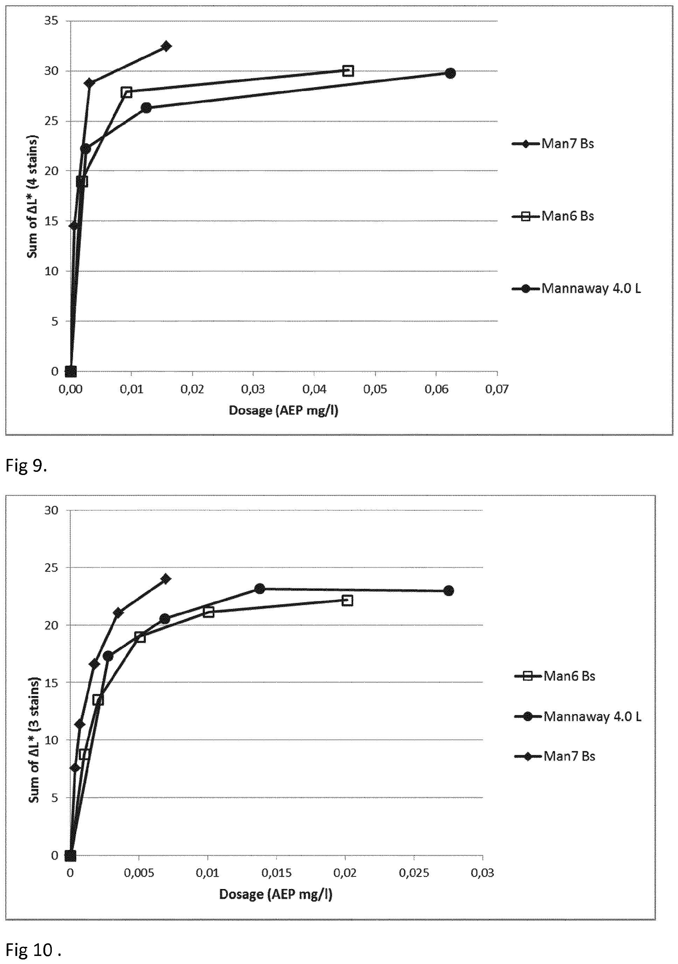

**Fig 9** describes the stain removal performance of Man 6 and Man7 (produced in *Bacillus)* as an increase of lightness (sum of ΔL*of 4 stains) in the presence of 3.8 g/l of Commercial color detergent powder at 40°C, 16 °dH, 60 min, pH approx. 10 and enzymes dosed as active enzyme protein. Commercial preparation Mannaway® 4,0 L was used for comparison.
**Fig 10** describes the stain removal performance of Man 6 and Man7 (produced in *Bacillus)* as an increase of lightness (sum of ΔL* of 3 stains) in the presence of 4,2 g/l of Commercial bleach detergent powder at 40°C, 16 °dH, 60 min, pH approx. 9.5 and enzymes dosed as active enzyme protein. Commercial preparation Mannaway® 4,0 L was used for comparison.

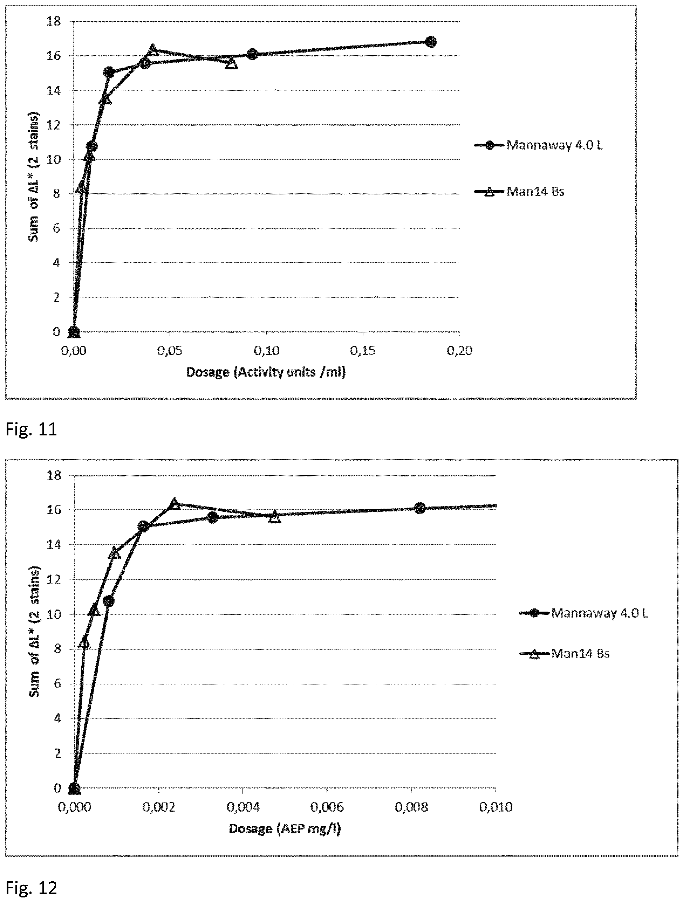

**Fig 11** the stain removal performance of Man14 (produced in *Bacillus)* as an increase of lightness (sum of ΔL*of 2 stains) in the presence of 5 g/l of Commercial heavy duty liquid detergent B at 40°C, 16 °dH, 60 min, pH approx. 8.3 and enzymes dosed as activity units. Commercial preparation Mannaway® 4,0 L was used for comparison.
**Fig 12** the stain removal performance of Man14 (produced in *Bacillus)* as an increase of lightness (sum of ΔL*of 2 stains) in the presence of 5 g/l of Commercial heavy duty liquid detergent B at 40°C, 16 °dH, 60 min, pH approx. 8.3 and enzymes dosed as active enzyme protein. Commercial preparation Mannaway® 4,0 L was used for comparison.

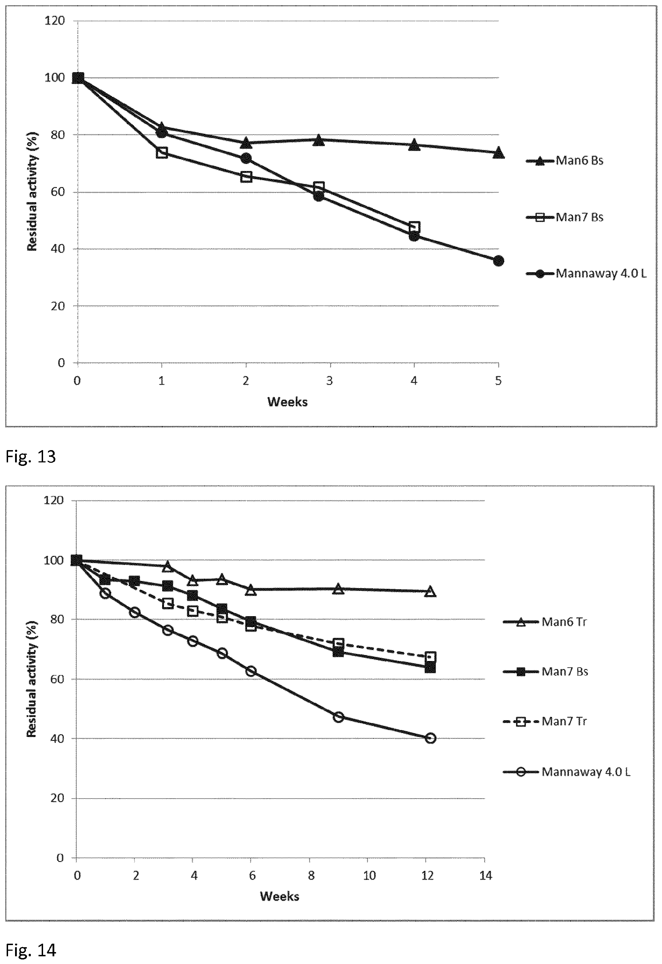

**Fig 13** describes the stability of Man 6 and Man7 (produced in *Bacillus)* in liquid detergent (OMO Color) at 37°C. Commercial preparation Mannaway® 4,0 L was used for comparison
**Fig 14** describes the stability of Man 6 (produced in *Bacillus)* in Commercial heavy duty liquid detergent A. Commercial preparation Mannaway® 4,0 L was used for comparison.

### SEQUENCE LISTINGS

SEQ ID NO: 1 Sequence of the oligonucleotide primer Man6_1
SEQ ID NO: 2 Sequence of the oligonucleotide primer Man6_2
SEQ ID NO: 3 Sequence of the oligonucleotide primer Man7_1
SEQ ID NO: 4 Sequence of the oligonucleotide primer Man7_2
SEQ ID NO: 5 Sequence of the oligonucleotide primer Man14_1
SEQ ID NO: 6 Sequence of the oligonucleotide primer Man14_2
SEQ ID NO: 7 Sequence of the oligonucleotide primer Vec_1
SEQ ID NO: 8 Sequence of the oligonucleotide primer Vec_2
SEQ ID NO: 9 The nucleotide sequence of the *Bacillus clausii man6*
SEQ ID NO: 10 The nucleotide sequence of the *Bacillus clausii man6* without signal peptide encoding sequence and with codon optimization to *Trichoderma reesei*
SEQ ID NO: 11 The deduced amino acid sequence of the *Bacillus clausii* Man6
SEQ ID NO: 12 The deduced amino acid sequence of the *Bacillus clausii* Man6 without signal peptide
SEQ ID NO: 13 The nucleotide sequence of the *Bacillus hemicellulosilyticus man7*
SEQ ID NO:14 The nucleotide sequence of the *Bacillus hemicellulosilyticus man7* without signal peptide encoding sequence and with codon optimization to *Trichoderma reesei*
SEQ ID NO: 15 The deduced amino acid sequence of the *Bacillus hemicellulosilyticus* Man7
SEQ ID NO: 16 The deduced amino acid sequence of the *Bacillus hemicellulosilyticus* Man7 without signal peptide
SEQ ID NO: 17 The nucleotide sequence of the *Virgibacillus soli man14*
SEQ ID NO: 18 The nucleotide sequence of the *Virgibacillus soli man14* without signal peptide encoding sequence and with codon optimization to *Trichoderma reesei*
SEQ ID NO: 19 The deduced amino acid sequence of the *Virgibacillus soli* Man14
SEQ ID NO: 20 The deduced amino acid sequence of the *Virgibacillus soli* Man14 without signal peptide
SEQ ID NO: 21 Sequence of the oligonucleotide primer BMAN1
SEQ ID NO: 22 Sequence of the oligonucleotide primer BMAN2
SEQ ID NO: 23 Sequence of the oligonucleotide primer BMAN3
SEQ ID NO: 24 Sequence of the oligonucleotide primer BMAN4
SEQ ID NO: 25 The nucleotide sequence of *Bacillus pumilus man31*
SEQ ID NO: 26 The deduced amino acid sequence of the *Bacillus pumilus* Man31
SEQ ID NO: 27 The nucleotide sequence of the *Bacillus amyloliquefaciens man32*
SEQ ID NO: 28 The deduced amino acid sequence of the *Bacillus amyloliquefaciens* Man32
SEQ ID NO: 29 The nucleotide sequence of the *Amphibacillus xylanus man33*
SEQ ID NO: 30 The deduced amino acid sequence of the *Amphibacillus xylans* Man33
SEQ ID NO: 31 The nucleotide sequence of the *Paenibacillus polymyxa man34*
SEQ ID NO: 32 The deduced amino acid sequence of the *Paenibacillus polymyxa* Man34
SEQ ID NO: 33 The nucleotide sequence of the *Bacillus hemicellulosilyticus man35*
SEQ ID NO: 34 The deduced amino acid sequence of the *Bacillus hemicellulosilyticus* Man35
SEQ ID NO: 35 The nucleotide sequence of the *Bacillus alcalophilus man36*
SEQ ID NO: 36 The deduced amino acid sequence of the *Bacillus alcalophilus* Man36
SEQ ID NO: 37 The nucleotide sequence of the *Bacillus* sp. *man37*
SEQ ID NO: 38 The deduced amino acid sequence of the *Bacillus* sp. Man37
SEQ ID NO: 39 The nucleotide sequence of the *Bacillus circulans man38*
SEQ ID NO: 40 The deduced amino acid sequence of the *Bacillus circulans* Man38
SEQ ID NO: 41 The nucleotide sequence of the *Paenibacillus* sp. *man39*
SEQ ID NO: 42 The deduced amino acid sequence of the *Paenibacillus* sp. Man39
SEQ ID NO: 43 The nucleotide sequence of the *Bacillus circulans man40*
SEQ ID NO: 44 The deduced amino acid sequence of the *Bacillus circulans* Man40
SEQ ID NO: 45 The nucleotide sequence of the *Bacillus nealsonii man41*
SEQ ID NO: 46 The deduced amino acid sequence of the *Bacillus nealsonii* Man41
SEQ ID NO: 47 The nucleotide sequence of the *Bacillus circulans man42*
SEQ ID NO: 48 The nucleotide sequence of the *Bacillus circulans* Man42

### DETAILED DESCRIPTION

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment a polypeptide, enzyme, polynucleotide, host cell or composition of the invention is isolated.

As used herein, the term "comprising" includes the broader meanings of "including", "containing", and "comprehending", as well as the narrower expressions "consisting of" and "consisting only of".

As used herein, "fragment" means a protein or a polynucleotide having one or more amino acids or nucleotides deleted. In the context of DNA, a fragment includes both single stranded and double stranded DNA of any length. A fragment may be an active fragment which has the biological function, such as enzyme activity or regulatory activity, of the protein or the polynucleotide. A fragment may also be an inactive fragment, i.e. it does not have one or more biological effects of the native protein or polynucleotide.

As used herein, "variant" means a fragment of sequence (nucleotide or amino acid) inserted or deleted by one or more nucleotides/amino acids or which is chemically modified.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss_needle/).

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora and Humicola;* more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;* more preferably from the group consisting of *Trichoderma reesei (Hypocrea jecorina), T. citrinoviridae, T. longibrachiatum, T. virens, T. harzianum, T. asperellum, T. atroviridae, T. parareesei,* , *Fusarium oxysporum, F. gramineanum, F. pseudograminearum, F. venenatum, Gibberella fujikuroi, G. moniliformis,* G. zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.* Non-limiting examples of a host cell are bacterial cells, preferably gram positive Bacilli (e.g. *B*. *subtilis, B. licheniformis, B. megaterium, B. amyloliquefaciens, B. pumilus),* actinomycetales (e.g. *Streptomyces* sp.) and yeasts (e.g. *Saccharomyces cerevisiae, Pichia pastoris, Yarrowia lipolytica)* In an example embodiment of the host cell is a fungal cell, preferably a filamentous fungal cell, such as *Trichoderma* or *Trichoderma reesei.* In an example embodiment of the host cell is a bacterial cell, preferably a gram positive *Bacillus* cell, such as *B*. *subtilis, B. licheniformis, B. megaterium, B. amyloliquefaciens, B. pumilus.*

A "recombinant cell" or "recombinant host cell" refers to a cell or host cell that has been genetically modified or altered to comprise a nucleic acid sequence which is not native to said cell or host cell. In an embodiment the genetical modification comprises integrating the polynucleotide in the genome of the host cell. In another embodiment the polynucleotide is exogenous compared to the host cell.

As used herein, "expression" includes any step involved in the production of a polypeptide in a host cell including, but not limited to, transcription, translation, post-translational modification, and secretion. Expression may be followed by the harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vectors are generally derived from plasmid or viral DNA, or may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The term "recombinant expressed" or "recombinantly expressed" used herein in connection with expression of a polypeptide or protein is defined according to the standard definition in the art.

The term "obtained from" and "obtainable" as used herein in connection with a specific microbial source, means that the polynucleotide and/or polypeptide is produced by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for expression of a recombinant mannanase, but which microorganism simultaneously produces other enzymes.

The term "operably linked", when referring to DNA segments, denotes that the segments are arranged so that they function in concert for their intended purposes, e.g. transcription initiates in the promoter and proceeds through the coding segment to the terminator

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" denotes a DNA sequence that encodes a polypeptide (a "secretory peptide") that, as a component of a larger polypeptide, directs the larger polypeptide through a secretory pathway of a host cell in which it is synthesized. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" denotes a domain of an enzyme which may or may not have been modified or altered, but which has retained its original activity; the catalytic domain as known in the art has remained functional.

By the term "linker" or "spacer" is meant a polypeptide comprising at least two amino acids which may be present between the domains of a multidomain protein, for example an enzyme comprising an enzyme core and a binding domain such as a carbohydrate binding module (CBM) or any other enzyme hybrid, or between two proteins or polypeptides expressed as a fusion polypeptide, for example a fusion protein comprising two core enzymes. For example, the fusion protein of an enzyme core with a CBM is provided by fusing a DNA sequence encoding the enzyme core, a DNA sequence encoding the linker and a DNA sequence encoding the CBM sequentially into one open reading frame and expressing this construct.

The term "detergent composition", includes unless otherwise indicated, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent composition" and "detergent formulation" are used in reference to mixtures which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the variants according to the invention, the term encompasses detergents that may contain, e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, enzyme inhibitors or stabilizers, enzyme activators, transferase(s), hydrolytic enzymes, oxido reductases, bluing agents and fluorescent dyes, antioxidants, and solubilizers.

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes storage stability and stability during use, e.g. during a wash process (in wash stability) and reflects the stability of the protease variant according to the invention as a function of time e.g. how much activity is retained when the protease is kept in solution, in particular in a detergent solution. The stability is influenced by many factors e.g. pH, temperature, detergent composition e.g. amount of builder, surfactants etc. The protease stability may be measured using the 'stability assay' as described in the Materials and Methods section herein. The term "improved stability" or "increased stability" is defined herein as a variant protease displaying an increased stability in solutions, relative to the stability of the parent protease. The terms "improved stability" and "increased stability" includes "improved chemical stability", "detergent stability" or "improved detergent stability.

### Detergent composition

The present invention relates to novel detergent compositions comprising bacterial mannanase enzymes. The detergent compositions comprising bacterial mannanases are useful in laundry and cleaning applications wherein degradation or modification of mannan is desired. The invention also relates to the use of said detergent compositions in laundry and cleaning applications as well as a method for degrading mannan.

As used herein, the term "mannan" refers to polysaccharides consisting of a mannose backbone linked together by β-1,4-linkages with side-chains of galactose attached to the backbone by α-1,6-linkages. Mannans comprise plant based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose sidebranches.

As used herein, the term "mannanase" or "galactomannanase" denotes a mannanase enzyme defined according to the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

"Mannanase activity" as used herein refers to the mannan degrading activity of a polypeptide. Degrading or modifying as used herein means that mannose units are hydrolyzed from the mannan polysaccharide by the mannanase. The mannan degrading activity of the polypeptides according to present invention can be tested according to standard test procedures known in the art. Example 7 provides an example of a standard method for determining mannanase activity.

According to the first aspect the detergent composition of the present invention comprises at least one enzyme having an amino acid sequence having at least 74% sequence identity to the amino acid sequence of SEQ ID NO: 16 (Man7), wherein the at least one enzyme has mannan degrading activity.

In one embodiment of the invention the at least one enzyme has an amino acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 16.

The mannanases comprised in the detergent composition of the invention are suitable for degrading and modifying mannan containing material in various chemical environments, preferably in detergent compositions.

In one embodiment of the present invention the detergent composition further comprises one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase, preferably selected from the group consisting of proteases, amylases, cellulases and lipases.

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

### Cellulases

Suitable cellulases include those of bacterial or fungal origin. Chemically modified or protein engineered mutants are included. Suitable cellulases include cellulases from the genera *Bacillus, Pseudomonas*, *Humicola*, *Fusarium*, *Thielavia*, *Acremonium*, *e*.*g*., the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259. Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299._Example of cellulases exhibiting endo-beta-1,4-glucanase activity (EC 3.2.1.4) are those having described in WO02/099091._Other examples of cellulases include the family 45 cellulases described in WO96/29397, and especially variants thereof having substitution, insertion and/or deletion at one or more of the positions corresponding to the following positions in SEQ ID NO: 8 of WO 02/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146 R._Commercially available cellulases include Celluclean™ Celluzyme™, and Carezyme™ (Novozymes A/S), Clazinase™, and Puradax HA™ (Genencor International Inc.), and KAC-500(B)™ (Kao Corporation).

### Proteases

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family. Examples of subtilases are those derived from *Bacillus* such as *Bacillus lentus*, *B*. *alkalophilus*, *B*. *subtilis*, *B*. *amyloliquefaciens*, *Bacillus pumilus* and *Bacillus gibsonii* described in; US7262042 and WO09/021867, and *subtilisin lentus*, *subtilisin Novo*, *subtilisin Carlsberg, Bacillus licheniformis, subtilisin BPN', subtilisin 309, subtilisin 147* and *subtilisin* 168 described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and WO05/040372, and the chymotrypsin proteases derived from *Cellumonas* described in WO05/052161 and WO05/052146. A further preferred protease is the alkaline protease from *Bacillus lentus* DSM 5483, as described for example in WO95/23221, and variants thereof, which are described in WO92/21760, WO95/23221, EP1921147 and EP1921148. Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering). Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime®, Prefer-enz™, Purafect MA®, Purafect Ox®, Purafect OxP®, Puramax®, Properase®, Effectenz™, FN2®, FN3® , FN4®, Excellase®, , Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

### Lipases and Cutinases

Suitable lipases and cutinases include those of bacterial or fungal origin. Chemically modified or protein engineered mutant enzymes are included. Examples include lipase from *Thermomyces*, e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P*. *alcaligenes* or *P*. *pseudoalcaligenes* (EP218272), *P*. *cepacia* (EP331376), *P*. *sp.* strain SD705 (WO95/06720 & WO96/27002), *P*. *wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S*. *pristinaespiralis* (WO12/137147).
Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™; Lipolex™ and Lipoclean™ (Novozymes A/S), Lumafast (originally from Genencor) and Lipomax (originally from Gist-Brocades).
Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M*. *smegmatis* perhydrolase in particular the S54V variant used in the commercial product Gentle Power Bleach from Huntsman Textile Effects Pte Ltd (WO10/100028).

### Amylases

Suitable amylases which can be used together with subtilase variants of the invention may be an alpha-amylase or a glucoamylase and may be of bacterial or fungal origin. Chemically modified or protein engineered mutants are included. Amylases include, for example, alpha-amylases obtained from *Bacillus*, *e*.*g*., a special strain of *Bacillus licheniformis,* described in more detail in GB 1,296,839.
Suitable amylases include amylases having SEQ ID NO: 3 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.
Different suitable amylases include amylases having SEQ ID NO: 6 in WO 02/010355 or variants thereof having 90% sequence identity thereto. Preferred variants are those having a deletion in positions 181 and 182 and a substitution in position 193.
Other amylases, which are suitable are hybrid alpha-amylase comprising residues 1-33 of the alpha-amylase derived from *B*. *amyloliquefaciens* shown in SEQ ID NO: 6 of WO 2006/066594 and residues 36-483 of the *B*. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B*. *amyloliquefaciens* shown in SEQ ID NO: 6 of WO 2006/066594 and residues 36-483 of SEQ ID NO: 4 of WO2006/066594 are those having the substitutions:
M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184. Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.
Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.
Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.
Other examples are amylase variants such as those described in WO2011/098531, WO2013/001078 and WO2013/001087.
Commercially available amylases are Duramyl™, Termamyl™, Fungamyl™, Stainzyme™, Stainzyme Plus™, Natalase™, Liquozyme X and BAN™ (from Novozymes A/S), and Rapidase™ , Purastar™/Effectenz™, Powerase and Preferenz S100 (from Genencor International Inc./DuPont).

### Peroxidases/Oxidases

Suitable peroxidases/oxidases include those of plant, bacterial or fungal origin. Chemically modified or protein engineered mutants are included. Examples of useful peroxidases include peroxidases from *Coprinus*, *e*.*g*., from *C*. *cinereus*, and variants thereof as those described in WO 93/24618, WO 95/10602, and WO 98/15257.
Commercially available peroxidases include Guardzyme™ (Novozymes A/S).
The detergent enzyme(s) may be included in a detergent composition by adding separate additives containing one or more enzymes, or by adding a combined additive comprising all of these enzymes. A detergent additive of the invention, *i*.*e*., a separate additive or a combined additive, can be formulated, for example, as a granulate, liquid, slurry, etc. Preferred detergent additive formulations are granulates, in particular non-dusting granulates, liquids, in particular stabilized liquids, or slurries.
Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

A composition for use in solid laundry detergent, for example, may include 0.000001% - 5%, such as 0.000005%-2%, such as 0.00001%-1%, such as 0.00001%-0,1% of enzyme protein by weight of the composition.

A composition for use in laundry liquid, for example, may include 0.000001% - 3%, such as 0.000005 - 1%, such as 0.00001%-0,01% of enzyme protein by weight of the composition.

A composition for use in automatic dishwash, for example, may include 0.000001% - 5%, such as 0.000005%-2%, such as 0.00001%-1%, such as 0.00001%-0,1% of enzyme protein by weight of the composition.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708.

In one embodiment, the invention is directed to detergent compositions comprising an enzyme of the present invention in combination with one or more additional cleaning composition components. The choice of additional components is within the skill of the artisan and includes conventional ingredients, including the exemplary non-limiting components set forth below.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

### 1. Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations thereof.

When included therein the detergent will usually contain from about 0% to about 10% by weight of a semipolar surfactant. Non-limiting examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, fatty acid alkanolamides and ethoxylated fatty acid alkanolamides, and combinations thereof.

When included therein the detergent will usually contain from about 0% to about 10% by weight of a zwitterionic surfactant. Non-limiting examples of zwitterionic surfactants include betaine, alkyldimethylbetaine, sulfobetaine, and combinations thereof.

### 2. Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### 3. Builders and Co-Builders

The detergent composition may contain about 0-65% by weight, such as about 5% to about 45% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-20% by weight, such as about 5% to about 10%, of a detergent co-builder, or a mixture thereof. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl-or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N'*-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra-(methylenephosphonic acid) (EDTMPA), diethylenetriaminepentakis(methylenephosphonic acid) (DTPMPA or DTMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*, *N*-diacetic acid (a-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA) , taurine-*N*, *N*-diacetic acid (TUDA) and sulfomethyl-*N*, N-diacetic acid (SMDA), *N*-(2-hydroxyethyl)-ethylidenediamine-*N*, *N*', *N*'-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

### 4. Bleaching Systems

The detergent may contain 0-50% by weight, such as about 0.1% to about 25%, of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:
(i) 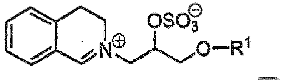
(ii) 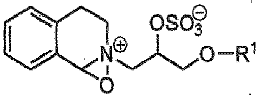
(iii) and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, e.g. in WO2007/087258, WO2007/087244, WO2007/087259 and WO2007/087242. Suitable photobleaches may for example be sulfonated zinc phthalocyanine

### 5. Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below- mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

### 6. Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

### 7. Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants: The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents: The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent: The detergent compositions of the present invention will preferably also contain additional components that may tint articles being cleaned, such as fluorescent whitening agent or optical brighteners. Where present the brightener is preferably at a level of about 0,01% to about 0,5%.. Any fluorescent whitening agent suitable for use in a laundry detergent composition may be used in the composition of the present invention. The most commonly used fluorescent whitening agents are those belonging to the classes of diaminostilbene-sulphonic acid derivatives, diarylpyrazoline derivatives and bisphenyl-distyryl derivatives. Examples of the diaminostilbene-sulphonic acid derivative type of fluorescent whitening agents include the sodium salts of: 4,4'-bis-(2-diethanolamino-4-anilino-s-triazin-6-ylamino) stilbene-2,2'-disulphonate; 4,4'-bis-(2,4-dianilino-s-triazin-6-ylamino) stilbene-2.2'-disulphonate; 4,4'-bis-(2-anilino-4(N-methyl-N-2-hydroxy-ethylamino)-s-triazin-6-ylamino) stilbene-2,2'-disulphonate, 4,4'-bis-(4-phenyl-2,1,3-triazol-2-yl)stilbene-2,2'-disulphonate; 4,4'-bis-(2-anilino-4(1-methyl-2-hydroxy-ethylamino)-s-triazin-6-ylamino) stilbene-2,2'-disulphonate and 2-(stilbyl-4"-naptho-1.,2':4,5)-1,2,3-trizole-2"-sulphonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4 anilino-s-triazin-6-ylamino) stilbene disulphonate. Tinopal CBS is the disodium salt of 2,2'-bis-(phenyl-styryl) disulphonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt % to upper levels of 0.5 or even 0.75 wt%.

Soil release polymers: The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore random graft co-polymers are suitable soil release polymers Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents: The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

In a further embodiment of the present invention the detergent composition is in form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit.

Pouches can be configured as single or multicompartments. It can be of any form, shape and material which is suitable for hold the composition, e.g. without allowing the release of the composition from the pouch prior to water contact. The pouch is made from water soluble film which encloses an inner volume. Said inner volume can be devided into compartments of the pouch. Preferred films are polymeric materials preferably polymers which are formed into a film or sheet. Preferred polymers, copolymers or derivates therof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxyprpyl methyl cellulose (HPMC). Preferably the level of polymer in the film for example PVA is at least about 60%. Preferred average molecular weight will typically be about 20,000 to about 150,000. Films can also be of blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticisers like glycerol, ethylene glycerol, Propylene glycol, sorbitol and mixtures thereof. The pouches can comprise a solid laundry detergent composition or part components and/or a liquid cleaning composition or part components separated by the water soluble film. The compartment for liquid components can be different in composition than compartments containing solids. Ref: (US2009/0011970 A1).

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

The detergent compositions of present invention may be provided in the form of laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example Na⁺, K⁺ or NH₄⁺ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, an enzyme, optionally one or more additional enzymes, a protease inhibitor, and a salt of a monovalent cation and an organic anion may be prepared and and the mixture is then plodded. The enzyme and optional additional enzymes may be added at the same time as the protease inhibitor for example in liquid form. Besides the mixing step and the plodding step, the process may further comprise the steps of milling, extruding, cutting, stamping, cooling and/or wrapping.

The present invention furthermore relates to different uses of the detergent composition as herein disclosed, such as for degrading mannan and for use in a laundry process.

The present invention furthermore relates to a method for removing a stain from a surface, comprising contacting the surface with a detergent composition as herein disclosed.

The present invention also relates to a method for degrading mannan comprising applying a detergent composition as herein disclosed to mannan, preferably wherein the mannan is on the surface of a textile. By degrading mannan attached to the textile or fabric, dirt or soil bound to mannan is released and not capable of binding again to the mannan or mannan stains.

In an embodiment of the present invention the mannanase comprised in the detergent composition of present invention has a relative activity of at least 30% in the temperature range from 45° to 65° C. Providing mannanases that retain activity in temperatures above ambient temperature and in acidic pH is advantageous for applications wherein mannan degradation is required in such conditions.

In an embodiment the mannanase comprised in the detergent compositions of present invention hydrolyses endo-beta-1,4-mannosidic linkages randomly.

In an embodiment the mannanase comprised in the detergent compositions of present invention is obtainable or derivable from a bacterial source.

In an embodiment the mannanase comprised in the detergent compositions of present invention is fused with at least one further polypeptide, thus forming a fusion polypeptide. The fusion polypeptide or the further polypeptide may have other catalytic or binding activities in addition to those of mannanase. In an embodiment the further polypeptide comprises or consists of carbohydrate binding module, which is optionally a fragment of another protein or enzyme derived from the same or different organism as the mannanase. The mannanase can be connected to the further polypeptide with a linker.

### EXAMPLES

The following examples are provided to illustrate various aspects of the present invention. They are not intended to limit the invention, which is defined by the accompanying claims.

Although the following examples are related to three different mannanase proteins, the mannanase protein Man7 is considered to be the novel mannanase according to the present invention.

### Example 1: Screening

For identification of new beta-1,4-mannanases public databases (NCBI, EBI) and selected proprietary and public genomes were screened. All proprietary and public genomes used in this work are shown in Tab. 1. All hits were grouped and finally 15 genes of bacterial origin were selected for cloning in Bacillus based on the phylogenetic distance between each other (Table 2)

**Table 1: List of proprietary and public genomes used for screening of beta-1,4-mannanases**

| Species | Strain | Source |
|---|---|---|
| *Bacillus pumilus* | MS8 | ABE |
| *Amphibacillus xylanus* | NBRC 15112 | NCBI |
| *Bacillus hemicellulosilyticus* | JCM 9152 | NCBI |
| *Bacillus clausii* | KSM-K16 | NCBI |
| *Bacillus amyloliquefaciens* | RH1330 | ABE |
| *Virigibacillus soli* | PL205 | NCBI |

**Table 2: List of genes selected for cloning in Bacillus**

| **Sequence ID** | **Species** | **GH family** | **Length** |
|---|---|---|---|
| orf2511 | *Bacillus amyloliquefaciens* | 26 | 360 aa |
| AXY_08250 | *Amphibacillus xylanus* | 5 | 497 aa |
| Man7 | *Bacillus hemicellulosilyticus* | 5 | 490 aa |
| T1Z249.2 | *Bacillus nealsonii* | 5 | 369 aa |
| Man6 | *Bacillus clausii* | 5 | 324 aa |
| Q9EYQ3 | *Clostridium cellulolyticum* | 5 | 424 aa |
| YdhT | *Bacillus cellulosilyticus* | 26 | 1183 aa |
| V5X1N9 | *Paenibacillus polymyxa* | 5 | 588 aa |
| Q9ZI87 | *Geobacillus stearothermophilus* | 5 | 694 aa |
| Q49HI4 | *Bacillus circulans* | 5 | 327 aa |
| orf0659 | *Bacillus pumilus* | 5 | 376 aa |
| JCM9152_1090 | *Bacillus hemicellulosilyticus* | 26 | 489 aa |
| D3HC62 | *Streptococcus gallolyticus* | 5 | 487 aa |
| A0LSH9 | *Acidothermus cellulolyticus* | 5 | 763 aa |
| Man14 | *Virgibacillus soli* | 5 | 482 aa |

### Example 2: Cloning of bacterial mannanases in Bacillus

Unless otherwise stated, the molecular biological methods including DNA manipulations and transformations were performed as described in Sambrook and Russell (2001) and Harwood and Cutting (1990). The genes *man6, man7* and *man14* were amplified by PCR using Pfx Accu Prime Polymerase (Invitrogen). PCRs were performed according to manufacturer's instructions. Following PCR conditions were used for construction of the expression plasmids: 120 sec initial denaturation at 94 °C, followed by 35 cycles of 15 sec at 94 °C, 30 sec annealing at one of the following 50/55 °C, 110/290 sec extension at 68 °C and the final extension at 68 °C for 10 min. For amplification of *man7* genomic DNA of *Bacillus hemicellulosilyticus* JCM 9152 was used. *man6* and *man14* were ordered as synthetic genes without codon optimization (Eurofins MWG, Germany). Sequences of primers used for cloning are shown in Table 3. Overhangs for hybridization are underlined.

**Table 3: List of primers used for amplification of man6, man7 and man14**

| **Template** | **Primer** | **bp** | **Sequence** | **Seq ID No** |
|---|---|---|---|---|
| syn. gene *man6* | Man6_1 | 39 | | 1 |
| syn. gene *man6* | Man6_2 | 39 | | 2 |
| gDNA *B*. *hemicellulosilyticus* | Man7_1 | 37 | | 3 |
| gDNA *B*. *hemicellulosilyticus* | Man7_2 | 36 | | 4 |
| syn. Gene *man14* | Man14_1 | 40 | | 5 |
| syn. Gene *man14* | Man14_2 | 39 | | 6 |
| pUB110 derivate | Vec_1 | 17 | AGCTGCAGAGGCGGTTG | 7 |
| pUB110 derivate | Vec_2 | 21 | GACAGAGATATACCGACAGTG | 8 |

Genes were cloned in a standard vector pEV1 pEV1 (Fig. 1), a pUB110 derivate including promoter PaprE from *Bacillus licheniformis* and xylanase signal peptide from *Bacillus amyloliquefaciens,* by using NEBuilder®Hifi DNA Assembly Master Mix (NEB, Frankfurt). A vector:insert ration of 1:3 was applied for cloning. The total amount of fragments was at 0.2 pmol in a total volume of 20 µl. Samples were incubated for 40 min at 50°C. For construction purposes, expression plasmids were transformed by induced competence in *Bacillus subtilis* SCK6 as described in Zhang & Zhang 2011. The transformed cells were plated onto LB (Luria-Bertani) plates supplemented with 10 mg/l Kanamycin. Plates were incubated for 20h at 37°C. Arising colonies were picked and plasmid was isolated using QiaPrep MiniPrep Kit (Qiagen, Hilden). Isolation procedure was carried out according to the manufacturers recommendations for Gram positives plasmid preparations. Inserts were sequenced via Sanger sequencing (GATC, Germany) and revealed the DNA sequences corresponding to the mature parts of the mannanases Man6, Man7 and Man14. Sequence comparisons were done using ClustalW sequence alignment (Thompson et al 1994). Finally, expression plasmids were transformed in an appropriate *Bacillus* production strain via electroporation. *Bacillus* production strain was grown in electroporation medium containing 20g/l Trypton, 10g/I yeast extract, 10g NaCl and 2M saccharose and 10 ml were harvested at an OD(600nm) of 0.4. Cells were washed with electroporation buffer containing 0,272M saccharose, 1mM MgCl₂ and 7mM KH₂PO4 and finally resuspended in 250 µl electroporation buffer. Electroporation was performed using following conditions: 1,2 kV, 150 Ω, 50 µF. 1ml electroporation medium was added afterwards and cells were incubated for 3h at 37°C. Cells were plated on LB plates supplemented with 20 mg/l Kanamycin and incubated for 18h at 37°C. Clones were verified as described above and used for generation of material for analytic tests. Therefore, strains were inoculated in a standard expression under protein inducing conditions and incubated for 30h at 37°C. Supernatants were harvested and used for analytical and application tests. Genes and enzyme characteristics are shown in Table 4 and 5.

**Table 4. The summary on the GH5 family mannanase encoding genes from Bacillus clausii KSM-K16, Bacillus hemicellulosilyticus JCM 9152 and Virgibacillus soli PL205.**

| **Gene** | **Length including SP (bp)** | **SEQ ID NO** |
|---|---|---|
| *man6* | 975 | 9 |
| *man7* | 1473 | 13 |
| *man14* | 1449 | 17 |

**Table 5. The summary of the amino acid sequences deduced from the GH5 mannanase encoding gene sequences from Bacillus clausii KSM-K16, Bacillus hemicellulosilyticus JCM 9152 and Virgibacillus soli PL205.**

| Man protein | No of AAs | Length of SS | CBM | Predicted MW (Da), ss not included | Predicted pl, ss not included | SEQ ID NO |
|---|---|---|---|---|---|---|
| Man6 | 324 | 35 | | 31.84 | 4.56 | 11 |
| Man7 | 490 | 21 | Yes | 51.36 | 4.81 | 15 |
| Man14 | 482 | 16 | Yes | 50.68 | 4.35 | 19 |

### Example 3: PCR-cloning of bacterial mannanases man6 and man7 in Trichoderma reesei

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

*Man6* and *man7* from *Bacillus clausii* and *Bacillus hemicellulosilyticus,* respectively, were also cloned for expression in *Trichoderma reesei* The genes were PCR-cloned using synthetic genes with codon optimization for *Trichoderma reesei.* DNA sequences encoding the signal peptides of *man6* and *man7* were removed using PCR and new cloning sites created. The sequences of the primers are shown in Table 6 (SEQ ID NOs: 21-24).

**Table 6. The oligonucleotides used as PCR primers to amplify Bacillus hemicellulosilyticus and Bacillus clausii mannanase genes.**

| Template, (synthetic) DNA from | Oligonucleotides | Length (bp) | Sequence^{(a} | SEQ ID NO: |
|---|---|---|---|---|
| *Bacillus hemicellulosilyticus* | BMAN1 | 60 | | 21 |
| *Bacillus hemicellulosilyticus* | BMAN2 | 46 | | 22 |
| *Bacillus clausii* | BMAN3 | 60 | | 23 |
| *Bacillus clausii* | BMAN4 | 50 | | 24 |

| | | | | |
|---|---|---|---|---|
| ^{(a} "s" in the parenthesis = sense strand, "as" = antisense strand. | | | | |

The genes were amplified by PCR with primers described in Table 6 and using synthetic DNAs as templates in the reactions. The PCR mixtures of *Bacillus clausii man6* and *Bacillus hemicellulosilyticus man7* contained each 1x HF buffer for Phusion HF Polymerase (NEB/BioNordika, Finland), 0.2 mMdNTP mix (Thermo Fisher Scientific, Finland), 1 µM each primer, 3% DMSO (Thermo Fisher Scientific), 1 unit of Phusion High-Fidelity Polymerase (NEB/BioNordika, Finland) and 50 ng of the corresponding plasmid DNA. The conditions for the PCR reactions were the following: 30 sec initial denaturation at 98 °C, followed by 28 cycles of 10 sec at 98 °C, 30 sec annealing at one of the following 45/50/55/60 °C, 45 sec extension at 72 °C and the final extension at 72 °C for 7 min.

Primer combination described in Table 6 produced specific DNA products having the expected sizes. The PCR products were isolated from agarose gel with GenJet Gel Extraction Kit (Thermo Fisher Scientific) according to manufacturer's instructions, digested with *Nrul* and BamHI restriction enzymes (Thermo Fisher Scientific) and cloned into an expression vector cleaved with *Nrul* and BamHI. Ligation mixtures were transformed into *Escherichia coli* XL1-Blue (AH Diagnostics) and plated on LB (Luria-Bertani) plates containing 50-100 µg/ml ampicillin. Several *E*. *coli* colonies were collected from the plates and DNA was isolated with GenJet Plasmid Miniprep Kit (Thermo Fisher Scientific). Positive clones were screened using restriction digestions. The genes encoding the *Bacillus clausii man6* and *Bacillus hemicellulosilyticus man7* GH5 mannanases without their own signal peptide encoding sequences were sequenced and the plasmids were named pALK4274 and pALK4273, respectively (For details see Example 6).

### Example 4: Cloning of synthetic bacterial mannanase man14

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

Mannanase gene *man14* from *Virgibacillus soli* was also cloned for *Trichoderma* expression as well. The gene encoding GH5 family mannanase Man14 from *Virgibacillus soli was* ordered from GenScript as a synthetic construct with codon optimization for *Trichoderma reesei.*

Plasmid DNA obtained from GenScript including the *man14* gene was re-suspended in sterile water, digested with *Nrul* and BamHI restriction enzymes (Thermo Fisher Scientific) according to manufacturer's instructions and cloned into an expression vector cleaved with *Nrul* and BamHI. Ligation mixture was transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions and they were shown to contain inserts of expected sizes. Fusion sites of *Virgibacillus soli man14* to the expression plasmid were sequenced and the plasmid was named pALK4414 (For details see Example 6).

### Example 5: Production of recombinant bacterial GH5 mannanase proteins in Bacillus

Expression plasmids were constructed for production of recombinant GH5 mannanase (Man6, Man7 and Man14) proteins from *Bacillus clausii, Bacillus hemicellulosilyticus* and *Virgibacillus soli.* The expression plasmids constructed are listed in Table 7. The recombinant GH5 genes (man6, *man7* and *man*14)*,* without their own signal sequences, were fused to the *Bacillus licheniformis PaprE promoter* and *B*. *amyloliquefaciens* xylanase signal peptide. The transcription termination was ensured by a strong terminator and a kanamycin resistance marker was used for selection of the transformants. The transformations were performed as described in Example 2.

**Table 7. The expression plasmids constructed to produce Man6, Man7 and Man14 recombinant proteins from Bacillus clausii, Bacillus hemicellulosilyticus and Virgibacillus soli in an appropriate Bacillus expression strain.**

| **Mannanase (GH5) protein** | **Expression plasmid** |
|---|---|
| Man6 | pEV1 Man6 |
| Man7 | pEV1 Man7 |
| Man14 | pEV1 Man14 |

The GH5 production of the transformants was analyzed from the culture supernatants of the shake flask cultivations. The transformants were inoculated from the LB plates to shake flasks containing 2% glucose, 6% corn steep powder, 1,3 % (NH4)2HPO4 , 0,05% MgSO4 x 7H2O and 0,5% CaCI2. pH was adjusted to pH 7.5. The GH5 protein production of the transformants was analyzed from culture supernatants after growing them for 30 hours at 37 °C, 180 rpm. Heterologous production of recombinant proteins was analyzed by SDS-PAGE with subsequent Coomassie staining. The best producing transformants were chosen to be cultivated in laboratory scale bioreactors. The transformants were cultivated in bioreactors at 37 °C under protein inducing conditions and additional feeding until a suitable yield was reached. The supernatants were recovered for application tests by centrifugation or filtration.

### Example 6: Production of recombinant bacterial GH5 mannanase proteins in Trichoderma reesei

Expression plasmids were constructed for production of recombinant GH5 mannanase (Man6, Man7 and Man14) proteins from *Bacillus clausii, Bacillus hemicellulosilyticus* and *Virgibacillus soli* (See Examples 3 and 4) in *Trichoderma reesei.* The expression plasmids constructed are listed in Table 8. The recombinant GH5 genes (*man6*, *man7* and *man14*), without their own signal sequences, were fused to the *T. reesei cel7A*/*cbh1* promoter with *T. reesei cel6A*/*cbh2* CBM carrier and linker followed by Kex2 protease recognition site. The transcription termination was ensured by the *T. reesei cel7A*/*cbh1* terminator and the *A. nidulans amdS* marker gene was used for selection of the transformants as described in Paloheimo *et al.* (2003). The linear expression cassettes (Fig. 2) were isolated from the vector backbones after *Not*I digestions and were transformed into *T.* reesei protoplasts. The host strains used does not produce any of the four major *T*. *reesei* cellulases (CBHI, CBHII, EGI, EGII). The transformations were performed as in Penttilä *et al.* (1987) with the modifications described in Karhunen *et al.* (1993), selecting acetamidase as a sole nitrogen source (amdS marker gene). The transformants were purified on selection plates through single conidia prior to sporulating them on PD.

**Table 8. The expression cassettes constructed to produce Man6, Man7 and Man14 recombinant proteins from Bacillus clausii, Bacillus hemicellulosilyticus and Virgibacillus soli in Trichoderma reesei. The overall structure of the expression cassettes was as described in Fig. 2.**

| **Mannanase (GH5) protein** | **Expression plasmid** | **Expression cassette ^{(a}** |
|---|---|---|
| Man6 | pALK4274 | 7.0 kb *Not*I |
| Man7 | pALK4273 | 7.5 kb *Not*I |
| Man14 | pALK4414 | 7.6 kb *Not*I |

| | | |
|---|---|---|
| ^{(a} The expression cassette for *T*. *reesei* transformation was isolated from vector backbone by using *Not*I digestion. | | |

The mannanase production of the transformants was analyzed from the culture supernatants of the shake flask cultivations. The transformants were inoculated from the PD slants to shake flasks containing 50 ml of complex lactose-based cellulase inducing medium (Joutsjoki *at al.* 1993) buffered with 5% KH₂PO₄. The GH5 protein production of the transformants was analyzed from culture supernatants after growing them for 7 days at 30 °C, 250 rpm. Heterologous production of recombinant proteins was analyzed by SDS-PAGE with subsequent Coomassie staining. The best producing transformants were chosen to be cultivated in laboratory scale bioreactors. The transformants were cultivated in bioreactors either on batch or by additional feeding type of process under protein inducing conditions at a typical mesophilic fungal cultivation temperature and slightly acidic conditions. The cultivation was continued until depletion of the medium sugars or until suitable yield was reached. The supernatants were recovered for application tests by centrifugation or by filtration.

### Example 7: Assay of galactomannanase activity by DNS -method

Mannanase activity (MNU) was measured as the release of reducing sugars from galactomannan (0.3 w/w-%) at 50°C and pH 7.0 in 5 min. The amount of released reducing carbohydrates was determined spectrophotometrically using dinitrosalicylic acid. Substrate (0,3 w/w-%) used in the assay was prepared as follows: 0.6 g of locust bean gum (Sigma G-0753) was in 50 mM sodium citrate buffer pH 7 (or citrate phosphate buffer pH 7) at about 80 °C using a heating magnetic stirrer and heated up to boiling point. The solution was cooled and let to dissolve overnight in a cold room (2 - 8 °C) with continuous stirring and insoluble residues were removed by centrifugation. After that solution was filled up to 200 ml by buffer. Substrate was stored as frozen and melted by heating in a boiling water bath to about 80 °C, cooled to room temperature and mixed carefully before use. DNS reagent used in the assay was prepared by dissolving 50 g of 3.5- dinitrosalisylic acid (Sigma D-550) in about 4 liter of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution that might have been cautiously warmed to a maximum temperature of 45 °C., was cooled to room temperature and filled up to 5000 ml. After that it was filtered through Whatman 1 filter paper and stored in a dark bottle at room temperature. The reaction was first started by adding 1.8 ml of substrate solution to each of the two test tubes and let to equilibrate at 50 °C for 5 minutes, after which 200 µl of suitably diluted enzyme solution was added to one of the tubes, mixed well with vortex mixer and incubated exactly for 5 min at 50 °C. Enzyme blanks didn't need to be equilibrated or incubated. The reaction was stopped by adding 3.0 ml of DNS reagent into both tubes and mixed. 200 µl of sample solution was added to the enzyme blank tubes. Both tubes were placed in a boiling water bath. After boiling for exactly 5 minutes, the tubes were placed in a cooling water bath and allow them to cool to room temperature. The absorbance of sample was measured against the enzyme blank at 540 nm and activity was read from the calibration curve and multiplied by the dilution factor. A suitable diluted sample yielded an absorbance difference of 0.15 - 0.4. Standard curve was prepared 20 mM from mannose stock solution by dissolving 360 mg of mannose (SigmaM-6020, stored in a desiccator) in assay buffer and diluted to solutions containing 3, 6, 10 and 14 µmol/ml of mannose. Standards were handled like the samples except for incubating at 50°C. The absorbances were measured against the reagent blank (containing buffer instead of standard dilution of mannose) at 540nm. Calibration curve was constructed for every series of assays. One mannanase unit (MNU) was defined as the amount of enzyme that produces reductive carbohydrates having a reductive power corresponding to one nmol of mannose from galactomannan in one second under the assay conditions (1 MNU = 1nkat).

### Example 8: Purification of Man6 mannanase

Cells and solids were removed from the fermentation culture medium by centrifugation for 10 min, 4000 g at 4°C. The supernatant of 10 ml was used for protein purification. The sample was filtered through 0.44 µm PVDF membrane (Millex-HV, Merck Millipore Ltd,Carrigtwohill, IRL). The filtrate was loaded onto a HiPrep 26/10 Desalting column (GE Healthcare, Uppsala, Sweden) equilibrated in 20 mM HEPES pH 7. The desalted sample was then loaded onto a 5 ml HiTrap Q HP column (GE Healthcare, Uppsala, Sweden) pre-equilibrated with 20 mM HEPES pH 7. After sample loading, the column was washed with the same buffer for 20 ml. Proteins were eluted with linear salt gradient 20 mM HEPES, 500 mM NaCl pH 7 in 15 CVs. Fractions of 5 ml were collected and analyzed on SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gel-filtration column equilibrated with 20 mM MES, 200 mM NaCl pH 6,5. Fractions of 2 ml were collected and analyzed by SDS-PAGE. Fractions containing pure mannanase were combined. Other mannanases were purified using the same protocol but changing the buffer composition in desalting and ion exchange steps. Buffer compositions are shown in Table 9.

**Table 9. Buffers used in ion exchange chromatography**

| Mannanase | Buffers used in ion exchange chromatography |
|---|---|
| Man6 | 20 mM HEPES pH 7 |
| Man7 | 20 mM HEPES pH 7 |
| Man14 | 20 mM MES pH 6 |

| | |
|---|---|
| Purified samples were above 95% pure. | |

Enzyme content of the purified sample was determined using UV absorbance 280 nm measurements. Excitation coefficients for each mannanases were calculated on the bases of amino acid sequence of the enzyme by using ExPASy Server http://web.expasy.org/protparam/. (Gasteiger E et al 2005). The enzyme activity (MNU) of purified samples was measured as release of reducing sugars as described in Example 7. The specific activity (MNU/mg) of mannanases was calculated by dividing MNU activity of purified sample with the amount of purified enzyme. Obtained values were used for calculating enzyme dosages used in Examples 10 and 11.

### pH profiles of mannanases

The pH profiles of purified mannanases were determined using the beta-mannazyme tablet assay Azurine-crosslinked carob galactomannan (T-MNZ 11/14) from Megazyme with minor modifications to the suggested protocol. The linearity of the assay has been checked with each purified enzymes. The assay was performed in 40 mM Britton-Robinson buffer adjusted to pH values between 4 and 11. The enzyme solution was diluted into the assay buffer and 500µl of enzyme solution was equilibrated at 50C water bath for 5 min before adding one substrate tablet. After 10 minutes, the reaction was stopped by adding 10 ml 2% Tris pH 12. The reaction tubes were left at room temperature for 5 min, stirred and the liquid filtered through a Whatman No.1 paper filter. Release of blue dye from the substrate was quantified by measuring the absorbance at 595 nm. Enzyme activity at each pH was reported as relative activity where the activity at the pH optimum was set to 100%. The pH profiles were shown in Figure 3.

Relative activity (%) of mannanase is calculated by dividing mannanase activity of a sample by the mannanase activity of a reference sample. In the case of pH profile, the reference sample is a sample at the optimal pH. In the case of temperature profile the reference sample is a sample at the optimal temperature.

### Temperature profiles of mannanases

The temperature optimum of purified mannanases was determined using the beta-mannazyme tablet assay Azurine-crosslinked carob galactomannan (T-MNZ 11/14) from Megazyme with minor modifications to suggested protocol. The assay was performed at temperatures varying between 30-90°C for 10 minutes in 40 mM Britton-Robinson buffer pH7. Enzyme activity was reported as relative activity where the activity at temperature optimum was set to 100%. The temperature profiles were shown in Figure 4.

### Temperature and pH characteristics of mannanases

Man6 has a molecular mass between 30-35 kDa. The optimal temperature of the enzyme at pH 7 is from 50°C to 70°C. Said enzyme has pH optimum at the pH range of at least pH 6 to pH 9 at 50°C. The optimal temperature and pH optimum were determined using 10 min reaction time and Azurine-crosslinked carob galactomannan as a substrate.

Man7 has a molecular mass between 50-55 kDa. The optimal temperature of the enzyme at pH 7 is from 50°C to 70°C. Said enzyme has pH optimum at the pH range of at least pH 7 to pH 10 at 50°C. The optimal temperature and pH optimum were determined using 10 min reaction time and Azurine-crosslinked carob galactomannan as a substrate.

Man14 has a molecular mass between 30-40 kDa. The optimal temperature of the enzyme at pH 7 is from 50°C to 70°C. Said enzyme has pH optimum at the pH range of at least pH 7 to pH 8 at 50°C. The optimal temperature and pH optimum were determined using 10 min reaction time and Azurine-crosslinked carob galactomannan as a substrate.

### Example 9: Stain removal performance of Man6 and Man7 mannanases with commercial detergents without bleaching agents

Man6 and Man7 mannanases produced in *Bacillus* (as described in Example 5) and in *Trichoderma* (as described in Example 6), were tested for their ability to remove mannanase sensitive standard stains at 40 °C and water hardness of 16°dH with commercial detergents without bleaching agents and compared to commercial mannanase preparation Mannaway® 4,0 L (Novozymes). The following artificially soiled test cloths from Center for testmaterial B.V. (the Netherlands) were used: Chocolate pudding mannanase sensitive on cotton (E-165), Locust bean gum, with pigment on cotton (C-S-73) and on polyester/cotton (PC-S-73) and Guar gum with carbon black on cotton (C-S-43). The fabric was cut in 6 cm x 6 cm swatches and 2 pieces of each were used in test.

Commercial heavyduty liquid detergent Acontaining all other enzymes except mannanase was used at concentration of 4.4 g per liter of wash liquor and Commercial Color detergent powder without enzymes was used at 3.8 g/l. Detergent containing wash liquors we prepared in synthetic tap water with hardness of 16 °dH. Protease Savinase® 16 L (0.5 w/w %) and amylase Stainzyme® 12 L (0.4 w/w %) was added into hard water used with commercial Color detergent powder, the liquid detergent already contained amylase and protease. pH of the wash liquor of Color detergent powder was approximately 10 and with the liquid detergent approximately 8.3.

Mannanase dosages were in range 0 - 0.2/0.25% of detergent weight but for the evaluation the dosages were calculated as enzyme activity units (MNU) per ml wash liquor or as mg of active enzyme protein (AEP) per I of wash liquor. Activity was measured as described in Example 7. AEP content of each preparation was calculated by dividing the enzyme activity with specific activity, defined in Example 8. Control sample contained the detergent solution but no mannanase.

For synthetic tap water with hardness of 16 °dH the following stock solutions were prepared in deionized water (Milli-Q or equivalent):
Stock solution with 1000 °d Calcium-hardness: CaCl2 x 2 H2O (1.02382.1000, Merck KGaA, Germany) 26,22 g/l
Stock solution with 200 °d Magnesium-hardness: MgSO4 x 7 H2O (1.05886.1000, Merck KGaA, Germany) 8,79 g/l H2O
NaHCO3 stock solution: NaHCO3 (1.06329.1000 Merck KGaA, Germany) 29,6 g/l
13,3 ml CaCl2 solution, 13,3 ml MgSO4 solution and 10,0 ml of freshly made NaHCO3 solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16 °dH and diluted enzyme (<1,0 ml) were added into 1,2 liter containers. Stains were added and the Launder-Ometer was run at 40°C for 60 min with a rotation speed of 42 rpm. After that the swatches were carefully rinsed under running water and dried overnight at indoor air, on a grid protected against daylight. The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each stains. Color values of each stains were average of 2 swatches. The results obtained with commercial liquid detergent are shown in Figs. 6-7. The mannanases according to the invention have similar (Man6) or considerably better (Man7) stain removal performance with liquid detergent when dosed as activity units or as active enzyme protein compared to commercial mannanase preparation Mannaway® 4,0 L. Similar performance was obtained with Man6 and Man7 regardless of the expression host, *Bacillus* or *Trichoderma* (Fig 6). The results obtained with commercial color detergent powder (Figs. 8-9) show that the mannanases according to the invention have better stain removal performance with color detergent powder when dosed as activity units or as active enzyme protein compared to commercial mannanase preparation Mannaway® 4,0 L.

### Example 10: Stain removal performance Man6 and Man7 mannanases with bleach containing detergent

Man6 and Man7 mannanases produced in *Bacillus* (as described in Example 5) were tested for their ability to remove mannanase sensitive standard stains at 40 °C and water hardness of 16°dH with commercial bleach detergent powder and compared to commercial mannanase preparation Mannaway® 4,0 L (Novozymes). Test system was similar to described in Example 9, except 3 different artificially soiled test cloths from Center for testmaterial B.V. (the Netherlands) were used: Chocolate pudding mannanase sensitive on cotton (E-165), Locust bean gum, with pigment on cotton (C-S-73) and Guar gum with carbon black on cotton (C-S-43). Commercial Color detergent powder was used at concentration of 4,2 g per liter of wash liquor and pH of the wash liquor was approx. 9,5. Protease Savinase® 16 L (0.5 w/w %) and amylase Stainzyme® 12 L (0.4 w/w %) were added into hard water used in test, since the detergent didn't contain any enzymes. The color of the swatches after treatment was measured and results calculated as sum of ΔL* of each 3 stains as described in Example 9. The results (Fig. 10) obtained with commercial bleach containing detergent indicate that the mannanase according to the invention (Man7) has considerably better stain removal performance compared to commercial mannanase Mannaway® 4,0 L when dosed as active enzyme protein. With Man6 at least similar performance compared to a commercial bacterial mannanase is obtained.

### Example 11: Stain removal performance Man14 mannanase with commercial liquid detergent

Man14 mannanase produced in Bacillus (as described in Example 5) was tested for their ability to remove mannanase sensitive standard stains at 40 °C and water hardness of 16°dH with commercial heavy duty liquid detergent B and compared to commercial mannanase preparation Mannaway® 4,0 L (Novozymes). Test system was similar to that described in Example 9, except two different artificially soiled test cloths from Center for testmaterial B.V. (the Netherlands) were used: Chocolate pudding mannanase sensitive on cotton (E-165) and Locust bean gum, with pigment on cotton (C-S-73). Commercial heavy duty liquid detergent B was used at concentration of 5 g per liter of wash liquor and pH of the wash liquor was approx. 8.3. Protease Savinase® 16 L (0,5 w/w %) and amylase Stainzyme® 12 L (0,4 w/w %) were added into hard water used in test, since the detergent didn't contain any enzymes. The color of the swatches after treatment was measured and results calculated as sum of ΔL* of each 2 stains as described in Example 9. The results (Figs. 11-12) obtained with commercial liquid containing detergent indicate Man14 had good performance in a liquid detergent, comparable to commercial product, when dosed either as activity units or as active enzyme protein.

### Example 12: Stability of Man6 and Man7 mannanases in commercial liquid detergents

The stability of Man6 and Man7 mannanase preparations produced in *Bacillus* were tested in OMO Color liquid obtained from local super market and compared to commercial mannanase preparation Mannaway® 4,0 L. Mannanase preparations were added 0,5 % w/w-% in detergents and samples were incubated in plastic tubes with caps at 37°C for 5 weeks. The activity was measured at certain intervals by activity assay described in Example 7 except using 30 min incubation time. Results were calculated as residual activity (%), which was obtained by dividing the activity of a sample taken at certain time point by initial activity of the sample. The stability of Man7 produced both in *Bacillus* and *Trichoderma* and Man6 produced in *Trichoderma* were tested against Mannaway® 4,0 L also in commercial liquid heavyduty detergent A containing protease but no mannanase. In this test 1 % -(w/w) of mannanases were used and samples incubated for 37°C for 12 weeks. The results in Omo Color (Fig. 13) show that Man6 had considerably better and Man7 similar stability compared to Mannaway® 4,0 L Both Man7 and especially Man6 were more stable than Mannaway® 4,0 L with another commercial liquid detergent A, as shown in Fig 14. Results obtained in another test at same conditions showed that Man6 had similar stability regardless of the expression host, *Bacillus* or *Trichoderma* (data not shown). The results of the stability experiments show that the mannanase according to the invention is stabile in detergents for several weeks even when stored at high temperature like 37°C. The stability of the mannanases according to the invention (Man6 and Man7) is improved compared to a commercial bacterial mannanase in liquid detergent.

### Example 13: Wash performance of liquid detergent compositions according to the invention

The wash performance of liquid detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A liquid washing agent with the following composition was used as base formulation (all values in weight percent):

| Chemical name | Active substance raw material [%] | Active substance detergent formulation [%] |
|---|---|---|
| Water demin. | 100 | Rest |
| Alkyl benzene sulfonic acid | 96 | 2-7 |
| Anionic surfactants | 70 | 6-10 |
| C12-C18 Fatty acid sodium salt | 30 | 1-4 |
| Nonionic surfactants | 100 | 4-7 |
| Phosphonates | 40 | 0,1-2 |
| Citric acid | 100 | 1-3 |
| NaOH | 50 | 1-4 |
| Boronic acid | 100 | 0,1-2 |
| Antifoaming agent | 100 | 0,01-1 |
| Glycerol | 100 | 1-3 |
| Enzymes | 100 | 0,1-2 |
| Preserving agent | 100 | 0,05-1 |
| Ethanol | 93 | 0,5-2 |
| Optical brightener | 90 | 0,01-1 |
| Perfume | 100 | 0,1-1 |
| Dye | 100 | 0,001-0,1 |

| | | |
|---|---|---|
| The pH of the detergent composition was between 8,2-8,6. | | |

Based on this base formulation, liquid detergent composition 2 was prepared by adding respective enzyme as indicated below:

### Composition 2: Enzyme according to SEQ ID NO:16 (Man7)

The wash was performed as follows according to the AISE Method: 3,5 kg Clean ballast cloth, 4 SBL Cloths, Miele washing machine, 20°C and 40°C Short program.

All mannanases were added in the same amounts based on total protein content.

The dosing ratio of the liquid washing agent was 4.0 grams per liter of washing liquor. The washing procedure was performed for 60 minutes at a temperature of 20°C and 40°C, the water having a water hardness between 15.5 and 16.5° (German degrees of hardness).

The results obtained are the difference values between the remission units obtained with the detergents and the remission units obtained with the detergent containing the commercially available reference mannanase (Mannaway 4.0L, obtained from Novozymes). A positive value therefore indicates an improved wash performance of the detergent compositions comprising the mannanases of present invention compared to the same detergent composition comprising the reference mannanase. Within the washing test a large range of stains were tested.

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the enzyme combinations. A positive value therefore indicates an improved wash performance due to the enzyme combinations present in the detergent. Mannanases of the invention in detergent compositions show improved performance on a variety of mannan comprising stains.

| | 20°C | 40°C |
|---|---|---|
| Stain | Comp.2 | Comp.2 |
| Chocolate Ice Cream (Equest) | 4.2 | n.d. |
| Carte Dor Chocolate Ice Cream (Equest) | 3.3 | 0.7 |
| Cocoa [CO] (Equest) | 2.3 | n.d. |
| Mayonnaise/Carbon black color (CFT CS05S [CO]) | 4.3 | 2.2 |
| Salad dressing, with natural black (CFT CS06 [CO]) | 3.5 | 2,6 |
| Lipstick, diluted, Red (CFT CS216 [CO]) | 1.5 | 0.7 |

### Example 14: Wash performance of powder detergent compositions according to the invention

The wash performance of powder detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") Warwick Equest Ltd Unit 55, Consett Business Park, Consett, County Durham ("Equest").

A solid washing agent with the following composition was used as base formulation (all values in weight percent):

| Chemical Name | Active substance raw material [%] | Active substance detergent formulation [%] |
|---|---|---|
| Water demin. | 100 | 1-4 |
| Alkyl benzene sulfonic acid | 97 | 9-13 |
| Nonionic surfactants | 100 | 4-7 |
| Percarbonates | 88 | 9-13 |
| TAED | 92 | 1-5 |
| Phosphonates | 60 | 0,1-3 |
| Polyacrylates | 45 | 1-4 |
| Soduim silicate | 40 | 5-10 |
| Sodium carbonate | 100 | 18-22 |
| Carboxymethylcellulose | 69 | 1-4 |
| Soil release polymer | 100 | 0,1-1 |
| Optical brightener | 70 | 0,1-1 |
| Antifoaming agent | t.q. | 0,01-1 |
| Sodium sulfate | 100 | 22-30 |
| Enzymes | 100 | 0,1-1 |
| Perfume | 100 | 0,1-1 |
| NaOH | 100 | 0,1-1 |
| Rest | - | 1-4 |

Based on this base formulation, solid detergent composition 4 was prepared by adding respective enzymes as indicated below:

### Composition 4: Enzyme according to SEQ ID NO:16 (Man7)

The wash was performed as follows according to the AISE Method: 3,5 kg Clean ballast cloth, 4 SBL Cloths, Miele washing machine, 20°C and 40°C Short program. All mannanases were added in the same amounts based on total protein content.

The dosing ratio of the powder washing agent was 3.8 grams per liter of washing liquor. The washing procedure was performed for 60 minutes at a temperature of 20°C and 40°C, the water having a water hardness between 15.5 and 16.5° (German degrees of hardness).

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the reference mannanase (Mannaway 4.0L, obtained from Novozymes). A positive value therefore indicates an improved wash performance of the variants in the detergent. Mannanases of the invention show improved performance on several stains. Therefore, it is evident that mannanases according to the invention show improved wash performance compared to Mannaway.

| | 20°C | 40°C |
|---|---|---|
| Stain | Comp.4 | Comp.4 |
| Carte Dór Chocolate Ice Cream (Equest) | 2,8 | 0,5 |
| Vienetta (Equest) | 0,8 | n.d. |
| Chocolate Icecream L [CO] (Equest) | 0,9 | n.d. |
| Porridge (EMPA 163 [CO]) | n.d. | 5,1 |
| Cocoa (CFT CS02 [CO]) | 3,1 | n.d. |
| Mayonnaise/Carbon black color (CFT CS05S [CO]) | 1,0 | 2,7 |
| Salad dressing, with natural black (CFT CS06 [CO]) | 4,8 | 5,1 |
| Sebum BEY with carbon black (CFT CS32 [CO]) | 1,4 | 0,7 |
| Chocolate drink, pure (CFT CS44 [CO]) | 1,4 | 0,8 |

Without limiting the scope and interpretation of the patent claims, certain technical effects of one or more of the aspects or embodiments disclosed herein are listed in the following: A technical effect is degradation or modification of mannan. Another technical effect is provision of mannanase which has good storage stability. The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

Furthermore, some of the features of the above-disclosed aspects and embodiments of this invention may be used to advantage without the corresponding use of other features. As such, the foregoing description should be considered as merely illustrative of the principles of the present invention, and not in limitation thereof. Hence, the scope of the invention is only restricted by the appended patent claims. In an embodiment at least one component of the compositions of the invention has a different chemical, structural or physical characteristic compared to the corresponding natural component from which the at least one component is derived from. In an embodiment said characteristic is at least one of uniform size, homogeneous dispersion, different isoform, different codon degeneracy, different post-translational modification, different methylation, different tertiary or quaternary structure, different enzyme activity, different affinity, different binding activity, and different immunogenicity.

## Claims

1. Detergent composition comprising at least one enzyme having an amino acid sequence having at least 74% sequence identity to the amino acid sequence of SEQ ID NO: 16, wherein the at least one enzyme has mannan degrading activity.

2. Detergent composition of claim 1, wherein the at least one enzyme has an amino acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 16.

3. The detergent composition of any one of claims 1 to 2, the composition further comprising one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase.

4. The detergent composition according to any one of claims 1 to 3, wherein the composition is in form of a bar, a homogenous tablet, a tablet having two or more layers, a pouch having one or more compartments, a regular or compact powder, a granule, a paste, a gel, or a regular, compact or concentrated liquid.

5. The detergent composition of any one of claims 1 to 4, wherein the detergent composition is a laundry detergent composition, preferably a liquid or solid laundry detergent composition.

6. Use of a detergent composition of any of claims 1 to 5for degrading mannan.

7. Use of a detergent composition according to any one of claims 1 to 5 in a laundry process.

8. A method for removing a stain from a surface, comprising contacting the surface with a detergent composition according to any one of claims 1 to 5.

9. A method for degrading mannan comprising applying a detergent composition according to any of claims 1 to 5 to mannan, preferably wherein the mannan is on the surface of a textile.

## Patentansprüche

1. Reinigungsmittelzusammensetzung, umfassend wenigstens ein Enzym mit einer Aminosäuresequenz, die wenigstens 74 % Sequenzidentität zu der Aminosäuresequenz aus SEQ ID NO: 16 aufweist, wobei das wenigstens eine Enzym Mannan-Abbauaktivität aufweist.

2. Reinigungsmittelzusammensetzung nach Anspruch 1, wobei das wenigstens eine Enzym eine Aminosäuresequenz mit wenigstens 75 %, wenigstens 76 %, wenigstens 77 %, wenigstens 78 %, wenigstens 79 %, wenigstens 80 %, wenigstens 81 %, wenigstens 82 %, wenigstens 83 %, wenigstens 84 %, wenigstens 85 %, wenigstens 86 %, wenigstens 87 %, wenigstens 88 %, wenigstens 89 %, wenigstens 90 %, wenigstens 91 %, wenigstens 92 %, wenigstens 93 %, wenigstens 94 %, wenigstens 95 %, wenigstens 96 %, wenigstens 97 %, wenigstens 98 % oder wenigstens 99 % Sequenzidentität zu der Aminosäuresequenz der SEQ ID NO: 16 aufweist.

3. Reinigungsmittelzusammensetzung nach einem der Ansprüche 1 bis 2, wobei die Zusammensetzung ferner ein oder mehrere zusätzliche Enzyme umfasst, ausgewählt aus der Gruppe bestehend aus Protease, Lipase, Cutinase, Amylase, Carbohydrase, Cellulase, Pectinase, Pectatlyase, Mannanase, Arabinase, Galactanase, Xylanase, Oxidase, Xanthanase, Laccase und/oder Peroxidase.

4. Reinigungsmittelzusammensetzung nach einem der Ansprüche 1 bis 3, wobei die Zusammensetzung in der Form eines Riegels, eines homogenen Tabs, eines Tabs mit zwei oder mehr Schichten, eines Beutels mit einem oder mehreren Fächern, eines herkömmlichen Kompaktpulvers, eines Granulats, einer Paste, eines Gels oder einer herkömmlichen, kompakten oder konzentrierten Flüssigkeit vorliegt.

5. Reinigungsmittelzusammensetzung nach einem der Ansprüche 1 bis 4, wobei die Reinigungsmittelzusammensetzung eine Waschmittelzusammensetzung ist, vorzugsweise eine flüssige oder feste Waschmittelzusammensetzung.

6. Verwendung einer Reinigungsmittelzusammensetzung nach einem der Ansprüche 1 bis 5 zum Abbauen von Mannan.

7. Verwendung einer Reinigungsmittelzusammensetzung nach einem der Ansprüche 1 bis 5 in einem Wäschewaschvorgang.

8. Verfahren zum Entfernen eines Flecks von einer Oberfläche, umfassend das Inberührungbringen der Oberfläche mit einer Reinigungsmittelzusammensetzung nach einem der Ansprüche 1 bis 5.

9. Verfahren zum Abbauen von Mannan, umfassend das Auftragen einer Reinigungsmittelzusammensetzung nach einem der Ansprüche 1 bis 5 auf Mannan, wobei sich das Mannan vorzugsweise auf der Oberfläche einer Textilie befindet.

## Revendications

1. Composition de détergent comprenant au moins une enzyme ayant une séquence d'acides aminés ayant au moins 74 % d'identité de séquences avec la séquence d'acides aminés SEQ ID N°16, l'au moins une enzyme exerçant une activité de dégradation du mannane.

2. Composition de détergent selon la revendication 1, dans laquelle l'au moins une enzyme a une séquence d'acides aminés ayant au moins 75 %, au moins 76 %, au moins 77 %, au moins 78 %, au moins 79 %, au moins 80 %, au moins 81 %, au moins 82 %, au moins 83 %, au moins 84 %, au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 % ou au moins 99 % d'identité de séquence avec la séquence d'acides aminés SEQ ID N°16.

3. Composition de détergent selon l'une quelconque des revendications 1 à 2, la composition comprenant en outre au moins une enzyme supplémentaire choisie dans le groupe constitué de la protéase, la lipase, la cutinase, l'amylase, la carbohydrase, la cellulase, la pectinase, la pectatlyase, la mannanase, l'arabinase, la galactanase, la xylanase, l'oxydase, la xanthanase, la laccase et/ou la peroxydase.

4. Composition de détergent selon l'une quelconque des revendications 1 à 3, la composition étant sous la forme d'une barre, d'un comprimé homogène, d'un comprimé comportant au moins deux couches, d'une poche comportant au moins un compartiment, d'une poudre régulière ou compacte, d'une granule, d'une pâte, d'un gel ou d'un liquide régulier, compact ou concentré.

5. Composition de détergent selon l'une quelconque des revendications 1 à 4, la composition de détergent étant une composition de détergent de blanchisserie, de préférence une composition liquide ou solide de détergent de blanchisserie.

6. Utilisation d'une composition de détergent selon l'une quelconque des revendications 1 à 5 destinée à la dégradation du mannane.

7. Utilisation d'une composition de détergent selon l'une quelconque des revendications 1 à 5 dans un processus de blanchisserie.

8. Procédé d'élimination d'une tache d'une surface, comprenant la mise en contact de la surface avec une composition de détergent selon l'une quelconque des revendications 1 à 5.

9. Procédé de dégradation du mannane comprenant l'application d'une composition de détergent selon l'une quelconque des revendications 1 à 5 sur du mannane, le mannane étant de préférence à la surface d'un textile.


### SEQUENCE LISTING

<110> Henkel AG & Co. KGaA
<120> Detergent compositions comprising Bacterial mannanases
<130> PT034268EP
<160> 48
<170> PatentIn version 3.5
<210> 1
   <211> 39
   <212> DNA
   <213> Artificial Sequence
<220>
   <223> primer
<400> 1
   caaccgcctc tgcagcttat gcacaaaacg gatttcacg 39
<210> 2
   <211> 39
   <212> DNA
   <213> Artificial Sequence
<220>
   <223> primer
<400> 2
   cggtatatct ctgtcttaat cactcttaag cccattttc 39
<210> 3
   <211> 37
   <212> DNA
   <213> Artificial Sequence
<220>
   <223> primer
<400> 3
   caaccgcctc tgcagcttct gatggtcata gccaaac 37
<210> 4
   <211> 36
   <212> DNA
   <213> Artificial Sequence
<220>
   <223> primer
<400> 4
   cggtatatct ctgtcttatt ggattgttac atgatc 36
<210> 5
   <211> 40
   <212> DNA
   <213> Artificial Sequence
<220>
   <223> primer
<400> 5
   caaccgcctc tgcagctgca agcgggtttt atgtaaacgg 40
<210> 6
   <211> 39
   <212> DNA
   <213> Artificial Sequence
<220>
   <223> primer
<400> 6
   cggtatatct ctgtcttatt taatggtaac gttatcaac 39
<210> 7
   <211> 17
   <212> DNA
   <213> Artificial Sequence
<220>
   <223> primer
<400> 7
   agctgcagag gcggttg 17
<210> 8
   <211> 21
   <212> DNA
   <213> Artificial Sequence
<220>
   <223> primer
<400> 8
   gacagagata taccgacagt g 21
<210> 9
   <211> 975
   <212> DNA
   <213> Bacillus clausii
<400> 9
   atgaagaggg aggacatgga tcaaatgaaa agaaagcggt tacaattgtt tggaacacta      60
   gtggtattgg ttttgttcgt gtacggtagc ggttcggcat atgcacaaaa cggatttcac     120
   gtatccggta cagagttgtt ggacaaaaat ggcgatcctt atgttatgcg tggcgtcaac     180
   catggacact cttggtttaa gcaagatctg gaggaagcaa tccctgccat agcagaaaca     240
   ggggcgaaca cggtgagaat ggtcttatcc aatggacagc aatgggaaaa agatgatgcc     300
   tctgagcttg cccgtgtgct ggctgccaca gaaacatatg gattgacaac tgtgctggaa     360
   gtccatgacg ctacaggaag tgacgatcct gctgatttag agaaagcagt cgattattgg     420
   atcgaaatgg ctgatgttct caaggggaca gaagaccgag taatcattaa cgttgccaat     480
   gaatggtatg ggtcgtggag gagcgacgtt tgggcagaag catacgcaca agcgatcccg     540
   cgcttgcgca gcgctggcct ctcccataca ttaatggttg atgcggcagg ttggggccag     600
   taccctgcct ccatccacga gcggggagcc gatgtgtttg cgtccgatcc attaaaaaac     660
   acgatgtttt cgatccatat gtacgaatat gcaggagctg atagggcgac aattgcctat     720
   aacattgatc gtgtgcttgc tgaaaatctt gctgtggtga tcggtgaatt tggccatagg     780
   catcatgatg gcgatgtcga tgaagatgcg attttggcct atacagcaga gcggcaagtg     840
   ggctggctgg cctggtcatg gtatggcaac agcgggggtg ttgaatactt ggatttagct     900
   gaaggcccat caggcccatt gacgagttgg ggcaaacgaa ttgtttatgg tgaaaatggg     960
   cttaagagtg attaa                                                      975
<210> 10
   <211> 870
   <212> DNA
   <213> Bacillus clausii
<400> 10
   cagaacggct tccacgtctc cggcacggag ctcctggaca agaacggcga cccttacgtc       60
   atgcgcggcg tcaaccacgg ccacagctgg ttcaagcagg acctcgagga agccatccct      120
   gctatcgctg agacgggcgc taacacggtc cgcatggtcc tgagcaacgg ccagcagtgg      180
   gagaaggacg acgctagcga gctggctcgc gtcctcgctg ctacggagac gtacggcctc      240
   accacggtcc tggaggtcca cgacgctacg ggctcggacg accccgccga cctcgagaag      300
   gccgtcgact actggatcga gatggctgac gtcctgaagg gcaccgagga ccgcgtcatc      360
   atcaacgtcg ccaacgagtg gtacggctcc tggcgcagcg acgtctgggc cgaggcctac      420
   gctcaggcta tccctcgcct ccgctcggcc ggcctctccc acacgctcat ggtcgacgct      480
   gccggctggg gccagtaccc tgcttccatc cacgagcgcg gcgctgacgt ctttgcttcg      540
   gaccccctga agaacaccat gttctccatc cacatgtacg agtacgctgg cgctgaccgc      600
   gctaccatcg cctacaacat cgaccgcgtc ctggctgaga acctggctgt cgtcatcggc      660
   gagtttggcc accgccacca cgacggcgac gtcgacgagg acgctatcct ggcttacacc      720
   gccgagcgcc aggtcggctg gctggcttgg tcgtggtacg gcaactcggg cggcgtcgag      780
   tacctggacc tggctgaggg cccttcgggc cctctcacga gctggggcaa gcgcatcgtc      840
   tacggcgaga acggcctgaa gagcgactaa                                        870
<210> 11
   <211> 324
   <212> PRT
   <213> Bacillus clausii
<400> 11
   Met Lys Arg Glu Asp Met Asp Gln Met Lys Arg Lys Arg Leu Gln Leu
   1 5 10 15
   Phe Gly Thr Leu Val Val Leu Val Leu Phe Val Tyr Gly Ser Gly Ser
   20 25 30
   Ala Tyr Ala Gln Asn Gly Phe His Val Ser Gly Thr Glu Leu Leu Asp
   35 40 45
   Lys Asn Gly Asp Pro Tyr Val Met Arg Gly Val Asn His Gly His Ser
   50 55 60
   Trp Phe Lys Gln Asp Leu Glu Glu Ala Ile Pro Ala Ile Ala Glu Thr
   65 70 75 80
   Gly Ala Asn Thr Val Arg Met Val Leu Ser Asn Gly Gln Gln Trp Glu
   85 90 95
   Lys Asp Asp Ala Ser Glu Leu Ala Arg Val Leu Ala Ala Thr Glu Thr
   100 105 110
   Tyr Gly Leu Thr Thr Val Leu Glu Val His Asp Ala Thr Gly Ser Asp
   115 120 125
   Asp Pro Ala Asp Leu Glu Lys Ala Val Asp Tyr Trp Ile Glu Met Ala
   130 135 140
   Asp Val Leu Lys Gly Thr Glu Asp Arg Val Ile Ile Asn Val Ala Asn
   145 150 155 160
   Glu Trp Tyr Gly Ser Trp Arg Ser Asp Val Trp Ala Glu Ala Tyr Ala
   165 170 175
   Gln Ala Ile Pro Arg Leu Arg Ser Ala Gly Leu Ser His Thr Leu Met
   180 185 190
   Val Asp Ala Ala Gly Trp Gly Gln Tyr Pro Ala Ser Ile His Glu Arg
   195 200 205
   Gly Ala Asp Val Phe Ala Ser Asp Pro Leu Lys Asn Thr Met Phe Ser
   210 215 220
   Ile His Met Tyr Glu Tyr Ala Gly Ala Asp Arg Ala Thr Ile Ala Tyr
   225 230 235 240
   Asn Ile Asp Arg Val Leu Ala Glu Asn Leu Ala Val Val Ile Gly Glu
   245 250 255
   Phe Gly His Arg His His Asp Gly Asp Val Asp Glu Asp Ala Ile Leu
   260 265 270
   Ala Tyr Thr Ala Glu Arg Gln Val Gly Trp Leu Ala Trp Ser Trp Tyr
   275 280 285
   Gly Asn Ser Gly Gly Val Glu Tyr Leu Asp Leu Ala Glu Gly Pro Ser
   290 295 300
   Gly Pro Leu Thr Ser Trp Gly Lys Arg Ile Val Tyr Gly Glu Asn Gly
   305 310 315 320
   Leu Lys Ser Asp
<210> 12
   <211> 289
   <212> PRT
   <213> Bacillus clausii
<400> 12
   Gln Asn Gly Phe His Val Ser Gly Thr Glu Leu Leu Asp Lys Asn Gly
   1 5 10 15
   Asp Pro Tyr Val Met Arg Gly Val Asn His Gly His Ser Trp Phe Lys
   20 25 30
   Gln Asp Leu Glu Glu Ala Ile Pro Ala Ile Ala Glu Thr Gly Ala Asn
   35 40 45
   Thr Val Arg Met Val Leu Ser Asn Gly Gln Gln Trp Glu Lys Asp Asp
   50 55 60
   Ala Ser Glu Leu Ala Arg Val Leu Ala Ala Thr Glu Thr Tyr Gly Leu
   65 70 75 80
   Thr Thr Val Leu Glu Val His Asp Ala Thr Gly Ser Asp Asp Pro Ala
   85 90 95
   Asp Leu Glu Lys Ala Val Asp Tyr Trp Ile Glu Met Ala Asp Val Leu
   100 105 110
   Lys Gly Thr Glu Asp Arg Val Ile Ile Asn Val Ala Asn Glu Trp Tyr
   115 120 125
   Gly Ser Trp Arg Ser Asp Val Trp Ala Glu Ala Tyr Ala Gln Ala Ile
   130 135 140
   Pro Arg Leu Arg Ser Ala Gly Leu Ser His Thr Leu Met Val Asp Ala
   145 150 155 160
   Ala Gly Trp Gly Gln Tyr Pro Ala Ser Ile His Glu Arg Gly Ala Asp
   165 170 175
   Val Phe Ala Ser Asp Pro Leu Lys Asn Thr Met Phe Ser Ile His Met
   180 185 190
   Tyr Glu Tyr Ala Gly Ala Asp Arg Ala Thr Ile Ala Tyr Asn Ile Asp
   195 200 205
   Arg Val Leu Ala Glu Asn Leu Ala Val Val Ile Gly Glu Phe Gly His
   210 215 220
   Arg His His Asp Gly Asp Val Asp Glu Asp Ala Ile Leu Ala Tyr Thr
   225 230 235 240
   Ala Glu Arg Gln Val Gly Trp Leu Ala Trp Ser Trp Tyr Gly Asn Ser
   245 250 255
   Gly Gly Val Glu Tyr Leu Asp Leu Ala Glu Gly Pro Ser Gly Pro Leu
   260 265 270
   Thr Ser Trp Gly Lys Arg Ile Val Tyr Gly Glu Asn Gly Leu Lys Ser
   275 280 285
   Asp
<210> 13
   <211> 1473
   <212> DNA
   <213> Bacillus hemicellulosilyticus
<400> 13
   atgagaaatt tcggtaagtt aattgtcagt tcttgtcttc tattcagttt ttttcttttt 60
   gcctctgatg gtcatagcca aacacattct ggtttttata tcgaaggttc aaccctttat 120
   gacgccaacg gagagccctt tgtaatgaga ggtatcaatc atggacatgc ctggtataaa 180
   catgattcta acgtcgctat accagctatt gctaatcaag gagcaaatac aattcgtatt 240
   gttctgtcag atggtggtca atgggcaaaa gatgatataa acacattaaa tcaagtgctc 300
   gatttagcag aggaacatga gatgattgct gttgttgagg ttcacgatgc aacaggatct 360
   aattctatgg ctgacttaaa tcgtgctgtc gattattgga ttgaaatgaa agacgcttta      420
   attggaaaag aagatcgcgt cataattaac attgccaatg aatggtatgg agcatgggac      480
   ggacaaggct gggcaaatgg ctataaggag gttattccac gtttacgaaa tgctggcttc      540
   actcatacat taatggtaga tgcagctggt tggggacaat accctcaatc gattcatgat      600
   tatggtcaag aggtatttaa tgctgatcct ttagcaaata cgatgttttc catccatatg      660
   tatgaatatg ctggcggaaa tgcttcaatg gtacaatcta atatcgatgg tgtcgtcgat      720
   caagggttag ctcttgtaat aggagaattt gggcatatgc atacggacgg agatgttgat      780
   gaagcaacga tattgagcta ctcgcaacaa agaggagtcg gttggctagc ttggtcttgg      840
   aaaggcaatg ggactcaatg ggaatatcta gatttatctt atgattggca aggaacaaac      900
   ttaacttctt ggggaaatac cattgtccac gggcctaatg gattactcga aacatccatt      960
   ccaagctcga ttttccatac cgctccaaac aatggagatc cccctcctca taacggtaat     1020
   gaaacgatct tatatgattt cgaacatggc actcaaggct ggtcaggttc ttcacttctt     1080
   ggaggacctt ggacgacgaa tgaatggagt acaaatggta accattcatt aaaggccgat     1140
   attttcttat cagctaactc caaacatgaa ttagcaaaag ttgaaaatcg aaatttatca     1200
   ggctactcta ctttacaagc cactgtccgc catgcacatt ggggaaatgt tggtaattta     1260
   acggcgagaa tgtatgtaaa aacgggctca aactatagct ggtttaatgg tgatcctatc     1320
   ccagtaaact cagcaaatgg tacgactgtc actttgcctc tttcatctat tccaaaccta     1380
   aatgacgtaa aagaaattgg cgttgaattt attggagctt caaatagcaa tggacaaacc     1440
   gccatttatt tagatcatgt aacaatccaa taa                                  1473
<210> 14
   <211> 1395
   <212> DNA
   <213> Bacillus hemicellulosilyticus
<400> 14
   cagacccact cgggcttcta catcgagggc tcgacgctct acgacgctaa cggcgagcct      60
   tttgtcatgc gcggcatcaa ccacggccac gcctggtaca agcacgactc caacgtcgct     120
   atccctgcta tcgctaacca gggcgctaac accatccgca tcgtcctcag cgacggtggc     180
   cagtgggcca aggacgacat caacacgctg aaccaggtcc tcgacctggc cgaggagcac     240
   gagatgatcg ctgtcgtcga ggtccacgac gctaccggct ccaacagcat ggccgacctc     300
   aaccgcgccg tcgactactg gatcgagatg aaggacgccc tgatcggcaa ggaagaccgc     360
   gtcatcatca acatcgctaa cgagtggtac ggcgcttggg acggccaggg ctgggccaac     420
   ggctacaagg aagtcatccc tcgcctgcgc aacgctggct tcacccacac cctcatggtc     480
   gacgctgccg gctggggcca gtaccctcag agcatccacg actacggcca agaggtcttc     540
   aacgccgacc ctctggccaa caccatgttc tccatccaca tgtacgagta cgctggcggc     600
   aacgcctcca tggtccagag caacatcgac ggcgtcgtcg accagggcct cgctctggtc     660
   atcggcgagt tcggccacat gcacacggac ggcgacgtcg acgaggctac catcctgagc     720
   tactcgcagc agcgcggcgt cggctggctg gcctggtcgt ggaagggcaa cggcacccag     780
   tgggagtacc tcgacctgag ctacgactgg cagggcacca acctcacgtc gtggggcaac     840
   acgatcgtcc acggccctaa cggcctcctg gagacgtcca tcccttccag catctttcac     900
   accgctccta acaacggcga ccctcctccc cacaacggca acgagacgat cctgtacgac     960
   ttcgagcacg gcacgcaggg ctggtcgggc tcgtccctgc tgggcggccc ttggaccacc    1020
   aacgagtggt cgaccaacgg caaccactcc ctcaaggccg acatcttcct gtccgccaac    1080
   agcaagcacg agctcgccaa ggtcgagaac cgcaacctca gcggctactc gacgctgcag    1140
   gctaccgtcc gccacgctca ctggggcaac gtcggcaacc tgacggctcg catgtacgtc    1200
   aagacgggca gcaactactc gtggttcaac ggcgacccca tccctgtcaa ctcggctaac    1260
   ggcaccaccg tcaccctccc tctgagctcg atccccaacc tcaacgacgt caaggagatc    1320
   ggcgtcgagt tcatcggcgc tagcaacagc aacggccaga ccgccatcta cctggaccac    1380
   gtcacgatcc agtaa                                                     1395
<210> 15
   <211> 490
   <212> PRT
   <213> Bacillus hemicellulosilyticus
<400> 15
   Met Arg Asn Phe Gly Lys Leu Ile Val Ser Ser Cys Leu Leu Phe Ser
   1               5                   10                  15
   Phe Phe Leu Phe Ala Ser Asp Gly His Ser Gln Thr His Ser Gly Phe
               20                  25                  30
   Tyr Ile Glu Gly Ser Thr Leu Tyr Asp Ala Asn Gly Glu Pro Phe Val
           35                  40                  45
   Met Arg Gly Ile Asn His Gly His Ala Trp Tyr Lys His Asp Ser Asn
       50                  55                  60
   Val Ala Ile Pro Ala Ile Ala Asn Gln Gly Ala Asn Thr Ile Arg Ile
   65                  70                  75                  80
   Val Leu Ser Asp Gly Gly Gln Trp Ala Lys Asp Asp Ile Asn Thr Leu
                   85                  90                  95
   Asn Gln Val Leu Asp Leu Ala Glu Glu His Glu Met Ile Ala Val Val
               100                 105                 110
   Glu Val His Asp Ala Thr Gly Ser Asn Ser Met Ala Asp Leu Asn Arg
           115                 120                 125
   Ala Val Asp Tyr Trp Ile Glu Met Lys Asp Ala Leu Ile Gly Lys Glu
       130                 135                 140
   Asp Arg Val Ile Ile Asn Ile Ala Asn Glu Trp Tyr Gly Ala Trp Asp
   145                 150                 155                 160
   Gly Gln Gly Trp Ala Asn Gly Tyr Lys Glu Val Ile Pro Arg Leu Arg
                   165                 170                 175
   Asn Ala Gly Phe Thr His Thr Leu Met Val Asp Ala Ala Gly Trp Gly
               180                 185                 190
   Gln Tyr Pro Gln Ser Ile His Asp Tyr Gly Gln Glu Val Phe Asn Ala
           195                 200                 205
   Asp Pro Leu Ala Asn Thr Met Phe Ser Ile His Met Tyr Glu Tyr Ala
       210                 215                 220
   Gly Gly Asn Ala Ser Met Val Gln Ser Asn Ile Asp Gly Val Val Asp
   225                 230                 235                 240
   Gln Gly Leu Ala Leu Val Ile Gly Glu Phe Gly His Met His Thr Asp
                   245                 250                 255
   Gly Asp Val Asp Glu Ala Thr Ile Leu Ser Tyr Ser Gln Gln Arg Gly
               260                 265                 270
   Val Gly Trp Leu Ala Trp Ser Trp Lys Gly Asn Gly Thr Gln Trp Glu
           275                 280                 285
   Tyr Leu Asp Leu Ser Tyr Asp Trp Gln Gly Thr Asn Leu Thr Ser Trp
       290                 295                 300
   Gly Asn Thr Ile Val His Gly Pro Asn Gly Leu Leu Glu Thr Ser Ile
   305                 310                 315                 320
   Pro Ser Ser Ile Phe His Thr Ala Pro Asn Asn Gly Asp Pro Pro Pro
                   325                 330                 335
   His Asn Gly Asn Glu Thr Ile Leu Tyr Asp Phe Glu His Gly Thr Gln
               340                 345                 350
   Gly Trp Ser Gly Ser Ser Leu Leu Gly Gly Pro Trp Thr Thr Asn Glu
   355 360 365
   Trp Ser Thr Asn Gly Asn His Ser Leu Lys Ala Asp Ile Phe Leu Ser
   370 375 380
   Ala Asn Ser Lys His Glu Leu Ala Lys Val Glu Asn Arg Asn Leu Ser
   385 390 395 400
   Gly Tyr Ser Thr Leu Gln Ala Thr Val Arg His Ala His Trp Gly Asn
   405 410 415
   Val Gly Asn Leu Thr Ala Arg Met Tyr Val Lys Thr Gly Ser Asn Tyr
   420 425 430
   Ser Trp Phe Asn Gly Asp Pro Ile Pro Val Asn Ser Ala Asn Gly Thr
   435 440 445
   Thr Val Thr Leu Pro Leu Ser Ser Ile Pro Asn Leu Asn Asp Val Lys
   450 455 460
   Glu Ile Gly Val Glu Phe Ile Gly Ala Ser Asn Ser Asn Gly Gln Thr
   465 470 475 480
   Ala Ile Tyr Leu Asp His Val Thr Ile Gln
   485 490
<210> 16
   <211> 464
   <212> PRT
   <213> Bacillus hemicellulosilyticus
<400> 16
   Gln Thr His Ser Gly Phe Tyr Ile Glu Gly Ser Thr Leu Tyr Asp Ala
   1 5 10 15
   Asn Gly Glu Pro Phe Val Met Arg Gly Ile Asn His Gly His Ala Trp
   20 25 30
   Tyr Lys His Asp Ser Asn Val Ala Ile Pro Ala Ile Ala Asn Gln Gly
   35 40 45
   Ala Asn Thr Ile Arg Ile Val Leu Ser Asp Gly Gly Gln Trp Ala Lys
   50 55 60
   Asp Asp Ile Asn Thr Leu Asn Gln Val Leu Asp Leu Ala Glu Glu His
   65 70 75 80
   Glu Met Ile Ala Val Val Glu Val His Asp Ala Thr Gly Ser Asn Ser
                   85                  90                  95
   Met Ala Asp Leu Asn Arg Ala Val Asp Tyr Trp Ile Glu Met Lys Asp
               100                 105                 110
   Ala Leu Ile Gly Lys Glu Asp Arg Val Ile Ile Asn Ile Ala Asn Glu
           115                 120                 125
   Trp Tyr Gly Ala Trp Asp Gly Gln Gly Trp Ala Asn Gly Tyr Lys Glu
       130                 135                 140
   Val Ile Pro Arg Leu Arg Asn Ala Gly Phe Thr His Thr Leu Met Val
   145                 150                 155                 160
   Asp Ala Ala Gly Trp Gly Gln Tyr Pro Gln Ser Ile His Asp Tyr Gly
                   165                 170                 175
   Gln Glu Val Phe Asn Ala Asp Pro Leu Ala Asn Thr Met Phe Ser Ile
               180                 185                 190
   His Met Tyr Glu Tyr Ala Gly Gly Asn Ala Ser Met Val Gln Ser Asn
           195                 200                 205
   Ile Asp Gly Val Val Asp Gln Gly Leu Ala Leu Val Ile Gly Glu Phe
       210                 215                 220
   Gly His Met His Thr Asp Gly Asp Val Asp Glu Ala Thr Ile Leu Ser
   225                 230                 235                 240
   Tyr Ser Gln Gln Arg Gly Val Gly Trp Leu Ala Trp Ser Trp Lys Gly
                   245                 250                 255
   Asn Gly Thr Gln Trp Glu Tyr Leu Asp Leu Ser Tyr Asp Trp Gln Gly
               260                 265                 270
   Thr Asn Leu Thr Ser Trp Gly Asn Thr Ile Val His Gly Pro Asn Gly
           275                 280                 285
   Leu Leu Glu Thr Ser Ile Pro Ser Ser Ile Phe His Thr Ala Pro Asn
       290                 295                 300
   Asn Gly Asp Pro Pro Pro His Asn Gly Asn Glu Thr Ile Leu Tyr Asp
   305                 310                 315                 320
   Phe Glu His Gly Thr Gln Gly Trp Ser Gly Ser Ser Leu Leu Gly Gly
                   325                 330                 335
   Pro Trp Thr Thr Asn Glu Trp Ser Thr Asn Gly Asn His Ser Leu Lys
               340                 345                 350
   Ala Asp Ile Phe Leu Ser Ala Asn Ser Lys His Glu Leu Ala Lys Val
           355                 360                 365
   Glu Asn Arg Asn Leu Ser Gly Tyr Ser Thr Leu Gln Ala Thr Val Arg
       370                 375                 380
   His Ala His Trp Gly Asn Val Gly Asn Leu Thr Ala Arg Met Tyr Val
   385                 390                 395                 400
   Lys Thr Gly Ser Asn Tyr Ser Trp Phe Asn Gly Asp Pro Ile Pro Val
                   405                 410                 415
   Asn Ser Ala Asn Gly Thr Thr Val Thr Leu Pro Leu Ser Ser Ile Pro
               420                 425                 430
   Asn Leu Asn Asp Val Lys Glu Ile Gly Val Glu Phe Ile Gly Ala Ser
           435                 440                 445
   Asn Ser Asn Gly Gln Thr Ala Ile Tyr Leu Asp His Val Thr Ile Gln
       450                 455                 460
<210> 17
   <211> 1449
   <212> DNA
   <213> Virgibacillus soli
<400> 17
   atgttattct ctacttcact gtttacttct acttcaaaag cgaatgcagc aagcgggttt      60
   tatgtaaacg gaaacacact ctatgacgca acaggtaccc cttttgtgat aagaggaatc     120
   aatcatgctc actcttggtt taaagacgac acagcaaccg caatacctgc cattgcagca     180
   actggggcga atactattag aatcgtatta tcggatggca gccaatatag tcgggatgat     240
   attgatggcg tgaggaatct aatatcattg gctgaggaaa ataatctaat tgctatgtta     300
   gaggtccacg atgctactgg aaaagatgat atcagctcat tagatagtgc ggcagattat     360
   tggattagta taaaagaagc acttatcggc aaggaagaca aagtcctaat aaacatcgca     420
   aatgaatggt acggtacttg ggatggggct agttgggcgg atggctacaa acaagtgatt     480
   cccaaattaa gaaatgcagg acttaaccac acactaatag tagactctgc tggctggggg     540
   caatttccgg agtccattca caattacgga aaagaagtat tcaatgctga ccccctacaa     600
   aatacaatgt tctctattca tatgtatgaa tatgctggtg gggacgcttc tactgtcaaa     660
   gcaaatattg acggtgtatt aaatcaaggt ctagccgtaa tcattggaga atttggacat     720
   aggcatacag acggagatgt agatgaagca acaattatga attattccca agagaaaaat     780
   gttggctggc tcgcatggtc gtggaaaggt aatggcatgg aatgggatta tttagactta     840
   tcctatgatt gggccggaaa taacctaacc gactggggaa ataccattgt aaatagtaca     900
   aacggcttaa aagctacatc tgaaataagt ccagtatttg gagatggaga tgacggtgta     960
   ggcgacggtg gtcctgggga ttctaacgga actgaaacta cgctttataa cttcgaaacc    1020
   gggacagaag gatggagcgg cgaaaatata gaaactggac cttggtcagt gaatgagtgg    1080
   gcagcaaaag gtaaccactc tttaaaagct gatgttaatt tgggtgataa ctctgaacat    1140
   tatctatacc taactcaaaa cctaaatttt agcggaaagt cacaactcac agcgactgta    1200
   aagcatgctg attggggaaa cttcggggat gaaataaatg caaagttata tgtaaaaaca    1260
   gaatcagatt ggcaatggtt tgatggagga attgaaaaga tcaattcttc aattggaact    1320
   attataacct tagatttatc atcgctctca aacccaagtg atattaaaga agttggtgtt    1380
   cagtttacgg gttcttcaaa tagttatggc ctaacagctt tatatgttga taacgttacc    1440
   attaaataa                                                            1449
<210> 18
   <211> 1401
   <212> DNA
   <213> Virgibacillus soli
<400> 18
   gcctcgggct tctacgtcaa cggcaacact ctctacgacg ccacgggcac cccatttgtc 60
   atccgcggca tcaaccacgc tcactcgtgg ttcaaggacg acactgccac cgctatccct 120
   gctatcgctg ctacgggcgc caacacgatc cgcatcgtcc tcagcgacgg ctcgcagtac 180
   tcccgcgacg acatcgacgg cgtccgcaac ctcatctccc tggccgagga gaacaacctc 240
   atcgccatgc tggaggtcca cgacgctacc ggcaaggacg acatcagctc gctggacagc 300
   gccgccgact actggatctc gatcaaggaa gccctcatcg gcaaggaaga caaggtcctg 360
   atcaacatcg ccaacgagtg gtacggcacc tgggacggcg ctagctgggc tgacggctac 420
   aagcaggtca tccctaagct ccgcaacgcc ggcctcaacc acacgctcat cgtcgactcg 480
   gctggctggg gccagttccc ggagagcatc cacaactacg gcaaggaagt cttcaacgcc 540
   gaccccctgc agaacacgat gttctcgatc cacatgtacg agtacgccgg cggcgacgct 600
   tccacggtca aggccaacat cgacggcgtc ctcaaccagg gcctggctgt catcatcggc 660
   gagtttggcc accgccacac cgacggcgac gtcgacgagg ccaccatcat gaactacagc 720
   caggagaaga acgtcggctg gctggcttgg agctggaagg gcaacggcat ggagtgggac 780
   tacctcgacc tgagctacga ctgggccggc aacaacctca ccgactgggg caacacgatc 840
   gtcaactcga ccaacggcct gaaggccacc tcggagatca gccctgtctt tggcgacggc 900
   gacgacggcg tcggcgacgg tggccccggc gacagcaacg gcaccgagac gacgctgtac 960
   aactttgaga cgggcaccga gggctggagc ggcgagaaca tcgagacggg cccttggtcg 1020
   gtcaacgagt gggctgccaa gggcaaccac tccctcaagg ccgacgtcaa cctgggcgac 1080
   aacagcgagc actacctcta cctgacgcag aacctcaact tctccggcaa gtcgcagctg 1140
   acggctaccg tcaagcacgc tgactggggc aacttcggcg acgagatcaa cgccaagctc 1200
   tacgtcaaga ccgagagcga ctggcagtgg ttcgacggtg gcatcgagaa gatcaactcc 1260
   agcatcggca ccatcatcac gctcgacctg tcgtccctgt cgaacccgtc cgacatcaag 1320
   gaagtcggcg tccagttcac tggctcgtct aactcttacg gcctcactgc tctttacgtc 1380
   gacaacgtca ctatcaagta g 1401
<210> 19
   <211> 482
   <212> PRT
   <213> Virgibacillus soli
<400> 19
   Met Leu Phe Ser Thr Ser Leu Phe Thr Ser Thr Ser Lys Ala Asn Ala
   1               5                   10                  15
   Ala Ser Gly Phe Tyr Val Asn Gly Asn Thr Leu Tyr Asp Ala Thr Gly
               20                  25                  30
   Thr Pro Phe Val Ile Arg Gly Ile Asn His Ala His Ser Trp Phe Lys
           35                  40                  45
   Asp Asp Thr Ala Thr Ala Ile Pro Ala Ile Ala Ala Thr Gly Ala Asn
       50                  55                  60
   Thr Ile Arg Ile Val Leu Ser Asp Gly Ser Gln Tyr Ser Arg Asp Asp
   65                  70                  75                  80
   Ile Asp Gly Val Arg Asn Leu Ile Ser Leu Ala Glu Glu Asn Asn Leu
                   85                  90                  95
   Ile Ala Met Leu Glu Val His Asp Ala Thr Gly Lys Asp Asp Ile Ser
               100                 105                 110
   Ser Leu Asp Ser Ala Ala Asp Tyr Trp Ile Ser Ile Lys Glu Ala Leu
           115                 120                 125
   Ile Gly Lys Glu Asp Lys Val Leu Ile Asn Ile Ala Asn Glu Trp Tyr
       130                 135                 140
   Gly Thr Trp Asp Gly Ala Ser Trp Ala Asp Gly Tyr Lys Gln Val Ile
   145                 150                 155                 160
   Pro Lys Leu Arg Asn Ala Gly Leu Asn His Thr Leu Ile Val Asp Ser
                   165                 170                 175
   Ala Gly Trp Gly Gln Phe Pro Glu Ser Ile His Asn Tyr Gly Lys Glu
               180                 185                 190
   Val Phe Asn Ala Asp Pro Leu Gln Asn Thr Met Phe Ser Ile His Met
           195                 200                 205
   Tyr Glu Tyr Ala Gly Gly Asp Ala Ser Thr Val Lys Ala Asn Ile Asp
       210                 215                 220
   Gly Val Leu Asn Gln Gly Leu Ala Val Ile Ile Gly Glu Phe Gly His
   225                 230                 235                 240
   Arg His Thr Asp Gly Asp Val Asp Glu Ala Thr Ile Met Asn Tyr Ser
                   245                 250                 255
   Gln Glu Lys Asn Val Gly Trp Leu Ala Trp Ser Trp Lys Gly Asn Gly
               260                 265                 270
   Met Glu Trp Asp Tyr Leu Asp Leu Ser Tyr Asp Trp Ala Gly Asn Asn
           275                 280                 285
   Leu Thr Asp Trp Gly Asn Thr Ile Val Asn Ser Thr Asn Gly Leu Lys
       290                 295                 300
   Ala Thr Ser Glu Ile Ser Pro Val Phe Gly Asp Gly Asp Asp Gly Val
   305                 310                 315                 320
   Gly Asp Gly Gly Pro Gly Asp Ser Asn Gly Thr Glu Thr Thr Leu Tyr
                   325                 330                 335
   Asn Phe Glu Thr Gly Thr Glu Gly Trp Ser Gly Glu Asn Ile Glu Thr
               340                 345                 350
   Gly Pro Trp Ser Val Asn Glu Trp Ala Ala Lys Gly Asn His Ser Leu
           355                 360                 365
   Lys Ala Asp Val Asn Leu Gly Asp Asn Ser Glu His Tyr Leu Tyr Leu
       370                 375                 380
   Thr Gln Asn Leu Asn Phe Ser Gly Lys Ser Gln Leu Thr Ala Thr Val
   385                 390                 395                 400
   Lys His Ala Asp Trp Gly Asn Phe Gly Asp Glu Ile Asn Ala Lys Leu
   405 410 415
   Tyr Val Lys Thr Glu Ser Asp Trp Gln Trp Phe Asp Gly Gly Ile Glu
   420 425 430
   Lys Ile Asn Ser Ser Ile Gly Thr Ile Ile Thr Leu Asp Leu Ser Ser
   435 440 445
   Leu Ser Asn Pro Ser Asp Ile Lys Glu Val Gly Val Gln Phe Thr Gly
   450 455 460
   Ser Ser Asn Ser Tyr Gly Leu Thr Ala Leu Tyr Val Asp Asn Val Thr
   465 470 475 480
   Ile Lys
<210> 20
   <211> 466
   <212> PRT
   <213> Virgibacillus soli
<400> 20
   Ala Ser Gly Phe Tyr Val Asn Gly Asn Thr Leu Tyr Asp Ala Thr Gly
   1 5 10 15
   Thr Pro Phe Val Ile Arg Gly Ile Asn His Ala His Ser Trp Phe Lys
   20 25 30
   Asp Asp Thr Ala Thr Ala Ile Pro Ala Ile Ala Ala Thr Gly Ala Asn
   35 40 45
   Thr Ile Arg Ile Val Leu Ser Asp Gly Ser Gln Tyr Ser Arg Asp Asp
   50 55 60
   Ile Asp Gly Val Arg Asn Leu Ile Ser Leu Ala Glu Glu Asn Asn Leu
   65 70 75 80
   Ile Ala Met Leu Glu Val His Asp Ala Thr Gly Lys Asp Asp Ile Ser
   85 90 95
   Ser Leu Asp Ser Ala Ala Asp Tyr Trp Ile Ser Ile Lys Glu Ala Leu
   100 105 110
   Ile Gly Lys Glu Asp Lys Val Leu Ile Asn Ile Ala Asn Glu Trp Tyr
   115 120 125
   Gly Thr Trp Asp Gly Ala Ser Trp Ala Asp Gly Tyr Lys Gln Val Ile
       130                 135                 140
   Pro Lys Leu Arg Asn Ala Gly Leu Asn His Thr Leu Ile Val Asp Ser
   145                 150                 155                 160
   Ala Gly Trp Gly Gln Phe Pro Glu Ser Ile His Asn Tyr Gly Lys Glu
                   165                 170                 175
   Val Phe Asn Ala Asp Pro Leu Gln Asn Thr Met Phe Ser Ile His Met
               180                 185                 190
   Tyr Glu Tyr Ala Gly Gly Asp Ala Ser Thr Val Lys Ala Asn Ile Asp
           195                 200                 205
   Gly Val Leu Asn Gln Gly Leu Ala Val Ile Ile Gly Glu Phe Gly His
       210                 215                 220
   Arg His Thr Asp Gly Asp Val Asp Glu Ala Thr Ile Met Asn Tyr Ser
   225                 230                 235                 240
   Gln Glu Lys Asn Val Gly Trp Leu Ala Trp Ser Trp Lys Gly Asn Gly
                   245                 250                 255
   Met Glu Trp Asp Tyr Leu Asp Leu Ser Tyr Asp Trp Ala Gly Asn Asn
               260                 265                 270
   Leu Thr Asp Trp Gly Asn Thr Ile Val Asn Ser Thr Asn Gly Leu Lys
           275                 280                 285
   Ala Thr Ser Glu Ile Ser Pro Val Phe Gly Asp Gly Asp Asp Gly Val
       290                 295                 300
   Gly Asp Gly Gly Pro Gly Asp Ser Asn Gly Thr Glu Thr Thr Leu Tyr
   305                 310                 315                 320
   Asn Phe Glu Thr Gly Thr Glu Gly Trp Ser Gly Glu Asn Ile Glu Thr
                   325                 330                 335
   Gly Pro Trp Ser Val Asn Glu Trp Ala Ala Lys Gly Asn His Ser Leu
               340                 345                 350
   Lys Ala Asp Val Asn Leu Gly Asp Asn Ser Glu His Tyr Leu Tyr Leu
           355                 360                 365
   Thr Gln Asn Leu Asn Phe Ser Gly Lys Ser Gln Leu Thr Ala Thr Val
       370                 375                 380
   Lys His Ala Asp Trp Gly Asn Phe Gly Asp Glu Ile Asn Ala Lys Leu
   385 390 395 400
   Tyr Val Lys Thr Glu Ser Asp Trp Gln Trp Phe Asp Gly Gly Ile Glu
   405 410 415
   Lys Ile Asn Ser Ser Ile Gly Thr Ile Ile Thr Leu Asp Leu Ser Ser
   420 425 430
   Leu Ser Asn Pro Ser Asp Ile Lys Glu Val Gly Val Gln Phe Thr Gly
   435 440 445
   Ser Ser Asn Ser Tyr Gly Leu Thr Ala Leu Tyr Val Asp Asn Val Thr
   450 455 460
   Ile Lys
   465
<210> 21
   <211> 60
   <212> DNA
   <213> Artificial Sequence
<220>
   <223> primer
<400> 21
   agtcaatcgc gacaagcgcc agacccactc gggcttctac atcgagggct cgacgctcta 60
<210> 22
   <211> 46
   <212> DNA
   <213> Artificial Sequence
<220>
   <223> primer
<400> 22
   cgcgccggat ccttactgga tcgtgacgtg gtccaggtag atggcg 46
<210> 23
   <211> 60
   <212> DNA
   <213> Artificial Sequence
<220>
   <223> primer
<400> 23
   agtcaatcgc gacaagcgcc agaacggctt ccacgtctcc ggcacggagc tcctggacaa 60
<210> 24
   <211> 51
   <212> DNA
   <213> Artificial Sequence
<220>
   <223> primer
<400> 24
   cgcgccggat ccttagtcgc tcttcaggcc gttctcgccg tagacgatgc g 51
<210> 25
   <211> 1846
   <212> DNA
   <213> Bacillus pumilus
<400> 25
   atgaaaaaat gggttcaacg ggtggcttgt tttatgctgc tgatcacttt atgggcgggt      60
   tggttcactc tgaccgtaaa ggcctcctcc tatgtgcaaa catctggtac acattttgta     120
   ttgaacaacc acccatttta ctttgctggc acaaataatt attatttcca ttacaaatca     180
   aaaaagatgg tagatgctgt ttttgacgat atgaaggcaa tggatttaaa ggttattcgt     240
   atttggggat ttcacgatgg tacccctcaa gaaaactcag tcttacaatc tcgtccaggt     300
   gtttatgaag aatccggttt tcaaaaacta gactatgcga tttataaagc agggcaggaa     360
   ggaatcaagc tggtcatacc gctcgtgaac aattgggatg actttggcgg gatgaatcaa     420
   tatgtgaagt ggtttcaggc aggatcacat gatcactttt atacagattc tcggattaaa     480
   acagcttaca aaaactatgt gcgctatgta ttagagagaa ccaatacgta ctcaggtgtt     540
   caatataaag atgaccctgc tattatgaca tgggagctcg ccaatgagcc gcgcgctcag     600
   tcagaccctt cgggagatat actagtaaac tgggcagatg aaatgagtgc atggatcaaa     660
   tcaattgact cgaatcatct tgttgctgta ggagacgaag ggttctttcg catgacaggt     720
   catgatgatt ggttttacag tggaggagaa ggtgttgatt gggatcgttt gactgctctc     780
   cctcatattg attatggaac ctatcattta tacccggatc actggaatca gtctgctgca     840
   tggggagtga aatggatcaa agatcatatc acccgaggaa acgcaatcgg aaaacctgtt     900
   gtattagaag agtttggcta tcaaaatcaa gcagcccgtc ctgatgtata tgatagctgg     960
   ctgaagacaa ttgaacagct cggaggcgca ggtagccaat tttggatttt aacaagcatt    1020
   caagacgatg attccctcta cccggattat gatggttttc gagttttaaa ggagagccgg    1080
   gaggcaggaa ttattcgtga acacgccaaa agaatgaatg aaaagaactg atgaagaatg    1140
   cctgtttata aggaacttca tttgcataaa aaaattggat atggtatagt ttttatggaa    1200
   atgctaacga ttaccgagac aagagtgggg aaacccgctc ttttgtattg aacaggcaat    1260
   ttttgtctcg acattattca tccgttttct gctccccctg ctcacaataa agcagggttt    1320
   ttatgcagaa tgattgataa gagcgtttat cgaaagcaca aggaggaaga gaatgagcaa    1380
   aaaagtagtg gatatcgtaa gcgacatggt gcagccaatt ttagatggct tacagcttga    1440
   actcgttgat gttgaatttg tcaaagaggg tcaaaactgg ttccttcgcg tatttattga     1500
   ctctgataaa ggcgtcgata tcgaggagtg tgccaaagtg agcgaagcct tgagcgaaaa     1560
   gcttgatgag gcagatccaa ttagccaaaa ctactttctt gaagtgtcct ctcctggagc     1620
   ggagcgccca ttaaagaaaa aagctgattt tgaaaaagca cttggaaaaa atgttttcat     1680
   gaaaacatac gaaccaattg atggtgaaaa ggcatttgaa ggtgagctta caagctttga     1740
   tggtgagatt gcaacagtga cagtgaagat caagacaaga aagaaagaga tcaatattcc     1800
   atacgaaaaa attgctaacg caagattagc agtttcgttc aattaa                    1846
<210> 26
   <211> 376
   <212> PRT
   <213> Bacillus pumilus
<400> 26
   Met Lys Lys Trp Val Gln Arg Val Ala Cys Phe Met Leu Leu Ile Thr
   1               5                   10                  15
   Leu Trp Ala Gly Trp Phe Thr Leu Thr Val Lys Ala Ser Ser Tyr Val
               20                  25                  30
   Gln Thr Ser Gly Thr His Phe Val Leu Asn Asn His Pro Phe Tyr Phe
           35                  40                  45
   Ala Gly Thr Asn Asn Tyr Tyr Phe His Tyr Lys Ser Lys Lys Met Val
       50                  55                  60
   Asp Ala Val Phe Asp Asp Met Lys Ala Met Asp Leu Lys Val Ile Arg
   65                  70                  75                  80
   Ile Trp Gly Phe His Asp Gly Thr Pro Gln Glu Asn Ser Val Leu Gln
                   85                  90                  95
   Ser Arg Pro Gly Val Tyr Glu Glu Ser Gly Phe Gln Lys Leu Asp Tyr
               100                 105                 110
   Ala Ile Tyr Lys Ala Gly Gln Glu Gly Ile Lys Leu Val Ile Pro Leu
           115                 120                 125
   Val Asn Asn Trp Asp Asp Phe Gly Gly Met Asn Gln Tyr Val Lys Trp
       130                 135                 140
   Phe Gln Ala Gly Ser His Asp His Phe Tyr Thr Asp Ser Arg Ile Lys
   145                 150                 155                 160
   Thr Ala Tyr Lys Asn Tyr Val Arg Tyr Val Leu Glu Arg Thr Asn Thr
                   165                 170                 175
   Tyr Ser Gly Val Gln Tyr Lys Asp Asp Pro Ala Ile Met Thr Trp Glu
               180                 185                 190
   Leu Ala Asn Glu Pro Arg Ala Gln Ser Asp Pro Ser Gly Asp Ile Leu
           195                 200                 205
   Val Asn Trp Ala Asp Glu Met Ser Ala Trp Ile Lys Ser Ile Asp Ser
       210                 215                 220
   Asn His Leu Val Ala Val Gly Asp Glu Gly Phe Phe Arg Met Thr Gly
   225                 230                 235                 240
   His Asp Asp Trp Phe Tyr Ser Gly Gly Glu Gly Val Asp Trp Asp Arg
                   245                 250                 255
   Leu Thr Ala Leu Pro His Ile Asp Tyr Gly Thr Tyr His Leu Tyr Pro
               260                 265                 270
   Asp His Trp Asn Gln Ser Ala Ala Trp Gly Val Lys Trp Ile Lys Asp
           275                 280                 285
   His Ile Thr Arg Gly Asn Ala Ile Gly Lys Pro Val Val Leu Glu Glu
       290                 295                 300
   Phe Gly Tyr Gln Asn Gln Ala Ala Arg Pro Asp Val Tyr Asp Ser Trp
   305                 310                 315                 320
   Leu Lys Thr Ile Glu Gln Leu Gly Gly Ala Gly Ser Gln Phe Trp Ile
                   325                 330                 335
   Leu Thr Ser Ile Gln Asp Asp Asp Ser Leu Tyr Pro Asp Tyr Asp Gly
               340                 345                 350
   Phe Arg Val Leu Lys Glu Ser Arg Glu Ala Gly Ile Ile Arg Glu His
           355                 360                 365
   Ala Lys Arg Met Asn Glu Lys Asn
       370                 375
<210> 27
   <211> 1083
   <212> DNA
   <213> Bacillus amyloliquefaciens
<400> 27
   atgctcaaaa agttcgcagt ctgtctgtct atcattttat tactcatctc agccgcccgt       60
   ccgatatcgg ctcacaccgt ttaccctgtc aatcccaatg cccagcagac gacaaaagac      120
   gtcatgaact ggctggcgca tttgcccaac cgttcagaaa acagggtcat gtccggtgca      180
   ttcggcgggt acagcgatgt caccttttca atgacggagg aaaaccgctt gaaaaacgcg      240
   acgggacagt ctcccgccat ctacggctgt gattatggga gagggtggct ggaaacatcg      300
   gatatcaccg attctatcga ctacagctgc aacagcagcc tcatttcgta ctggaaaagc      360
   ggcggcctcc ctcaggtcag cctgcatctc gcaaatccgg cctttccatc aggacactat      420
   aaaacggcca tttcaaacag ccagtataaa aatatcctga acccttcaac tgttgaagga      480
   cggcggcttg aggccttgct cagcaaaatc gccgacggcc ttactcagct gaaaaatcaa      540
   ggcgtcaccg ttctgttcag gccgctgcat gagatgaacg gtgaatggtt ctggtggggg      600
   ctgacaggct acaaccaaaa agacactgag agaatctcgc tgtacaaaga gctttacaag      660
   aagatatacc gctatatgac agagacaaga ggattggata atcttttgtg ggtgtattcg      720
   cctgatgcca acagagactt caaaacagac ttctacccag gctcatctta tgtggatatt      780
   accggactgg atgcttactt caccgacccg tatgcgatat caggctatga tgaaatgctg      840
   tctctgaaaa aaccgtttgc ctttgccgag accggtccgt ccggtaatat cggaagcttt      900
   gattacgctg tttttatcaa tgcgatcagg caaaagtatc ccgagacaac ctactttttg      960
   acatgggatg aacaattaag cccggcagcc aatcaaggcg cgcaaagcct ttatcaaaac     1020
   agctggacgc tgaacaaggg cgaaatgtgg aatggcggaa ccttgacgcc gatcgcggaa     1080
   taa                                                                   1083
<210> 28
   <211> 360
   <212> PRT
   <213> Bacillus amyloliquefaciens
<400> 28
   Met Leu Lys Lys Phe Ala Val Cys Leu Ser Ile Ile Leu Leu Leu Ile
   1               5                   10                  15
   Ser Ala Ala Arg Pro Ile Ser Ala His Thr Val Tyr Pro Val Asn Pro
               20                  25                  30
   Asn Ala Gln Gln Thr Thr Lys Asp Val Met Asn Trp Leu Ala His Leu
           35                  40                  45
   Pro Asn Arg Ser Glu Asn Arg Val Met Ser Gly Ala Phe Gly Gly Tyr
       50                  55                  60
   Ser Asp Val Thr Phe Ser Met Thr Glu Glu Asn Arg Leu Lys Asn Ala
   65                  70                  75                  80
   Thr Gly Gln Ser Pro Ala Ile Tyr Gly Cys Asp Tyr Gly Arg Gly Trp
   85 90 95
   Leu Glu Thr Ser Asp Ile Thr Asp Ser Ile Asp Tyr Ser Cys Asn Ser
   100 105 110
   Ser Leu Ile Ser Tyr Trp Lys Ser Gly Gly Leu Pro Gln Val Ser Leu
   115 120 125
   His Leu Ala Asn Pro Ala Phe Pro Ser Gly His Tyr Lys Thr Ala Ile
   130 135 140
   Ser Asn Ser Gln Tyr Lys Asn Ile Leu Asn Pro Ser Thr Val Glu Gly
   145 150 155 160
   Arg Arg Leu Glu Ala Leu Leu Ser Lys Ile Ala Asp Gly Leu Thr Gln
   165 170 175
   Leu Lys Asn Gln Gly Val Thr Val Leu Phe Arg Pro Leu His Glu Met
   180 185 190
   Asn Gly Glu Trp Phe Trp Trp Gly Leu Thr Gly Tyr Asn Gln Lys Asp
   195 200 205
   Thr Glu Arg Ile Ser Leu Tyr Lys Glu Leu Tyr Lys Lys Ile Tyr Arg
   210 215 220
   Tyr Met Thr Glu Thr Arg Gly Leu Asp Asn Leu Leu Trp Val Tyr Ser
   225 230 235 240
   Pro Asp Ala Asn Arg Asp Phe Lys Thr Asp Phe Tyr Pro Gly Ser Ser
   245 250 255
   Tyr Val Asp Ile Thr Gly Leu Asp Ala Tyr Phe Thr Asp Pro Tyr Ala
   260 265 270
   Ile Ser Gly Tyr Asp Glu Met Leu Ser Leu Lys Lys Pro Phe Ala Phe
   275 280 285
   Ala Glu Thr Gly Pro Ser Gly Asn Ile Gly Ser Phe Asp Tyr Ala Val
   290 295 300
   Phe Ile Asn Ala Ile Arg Gln Lys Tyr Pro Glu Thr Thr Tyr Phe Leu
   305 310 315 320
   Thr Trp Asp Glu Gln Leu Ser Pro Ala Ala Asn Gln Gly Ala Gln Ser
                       325                 330                 335
   Leu Tyr Gln Asn Ser Trp Thr Leu Asn Lys Gly Glu Met Trp Asn Gly
               340                 345                 350
   Gly Thr Leu Thr Pro Ile Ala Glu
           355                 360
<210> 29
   <211> 1494
   <212> DNA
   <213> Amphibacillus xylanus
<400> 29
   gtgaagttaa ctaaactaaa actattgagt agtgtatttt ttgttgtatt aactgtgtta      60
   atgttgtttg tccctgggaa tattgtgaat gtaaaagctg ctaacggctt ttatgtaagc     120
   gattccaatc tgtatgatgc aaatggaaat caatttgtta tgcgtggggt taatcatgcc     180
   cattcatggt ataaggacac gtataccgag gcaattcctg caattgcggc tacaggagcg     240
   aatactatcc gaattgtatt atctgatgga gggcaatacc aaaaagatga tataaacata     300
   gtcagaaatt tgattgaaac cgcagaagcc aataatttag tcgctgtact tgaggttcat     360
   gatgctactg ggtcggattc attatcggat ttgaaccggg ctgtagatta ttggattgaa     420
   attaaagatg cgttaattgg taaagaagat acggtgatca taaacattgt caatgaatgg     480
   tatggcactt gggatggtcg tctctgggca gatggttata aacaggcgat accgagatta     540
   agagatgctg gattaacaca tacgttgatg attgatgcag caggttgggg gcaatttcct     600
   agctcgatcc atcaatatgg tagagaagta tttaatgcag atcgtttagg gaatacaatg     660
   ttttcgattc atatgtatga atatgctggc ggtgatgatc aaatggttag agataatatt     720
   aacggtgtga tcaatcaaga cttagctcta gtgattggtg aatttggtca ttatcacaca     780
   gatggcgatg ttgatgaaga tacgattttg agttacgcgg agcagacagg tgttggttgg     840
   ttagcatggt catggaaagg caatggaact gagtgggagt atcttgatct atcaaatgat     900
   tggggaggaa attatttaac atcttggggt gacaggattg taaatggagc aaatggatta     960
   agagaaacga gtcaaattgc ttctgttttt tcaggaaaca atggcgggac tcctggaaat    1020
   ggtgaggaag agactcctgg tgatgtaagt catttcgcaa acttcgagaa tggtactgaa    1080
   ggttgggaag caagcaatgt atctggtgga ccttgggcaa caaatgaatg gagtgctagt    1140
   ggttcatatg ctttaaaagc cgatgcgcaa ttagcatctg gaagagaaca ctatttatat    1200
   cgaatcggtc cctttaattt atctgggtca acattaaacg caacggtaag gggtgctaat     1260
   tgggggaatt atggatctgg tatcgacgtg aagctatacg ttaagtacgg agatggctgg    1320
   acgtggagag atagtggtgt acagacaatt agagcgggag aatctattga tctatcacta    1380
   gatttatcaa atgttgatcg ctcaaacatt agagaagttg gtatccagtt tattggtgga    1440
   aatcattcat ctggaaaaac cgctttttat gttgatcatg tttattcaca ttag          1494
<210> 30
   <211> 497
   <212> PRT
   <213> Amphibacillus xylanus
<400> 30
   Val Lys Leu Thr Lys Leu Lys Leu Leu Ser Ser Val Phe Phe Val Val
   1               5                   10                  15
   Leu Thr Val Leu Met Leu Phe Val Pro Gly Asn Ile Val Asn Val Lys
               20                  25                  30
   Ala Ala Asn Gly Phe Tyr Val Ser Asp Ser Asn Leu Tyr Asp Ala Asn
           35                  40                  45
   Gly Asn Gln Phe Val Met Arg Gly Val Asn His Ala His Ser Trp Tyr
       50                  55                  60
   Lys Asp Thr Tyr Thr Glu Ala Ile Pro Ala Ile Ala Ala Thr Gly Ala
   65                  70                  75                  80
   Asn Thr Ile Arg Ile Val Leu Ser Asp Gly Gly Gln Tyr Gln Lys Asp
                   85                  90                  95
   Asp Ile Asn Ile Val Arg Asn Leu Ile Glu Thr Ala Glu Ala Asn Asn
               100                 105                 110
   Leu Val Ala Val Leu Glu Val His Asp Ala Thr Gly Ser Asp Ser Leu
           115                 120                 125
   Ser Asp Leu Asn Arg Ala Val Asp Tyr Trp Ile Glu Ile Lys Asp Ala
       130                 135                 140
   Leu Ile Gly Lys Glu Asp Thr Val Ile Ile Asn Ile Val Asn Glu Trp
   145                 150                 155                 160
   Tyr Gly Thr Trp Asp Gly Arg Leu Trp Ala Asp Gly Tyr Lys Gln Ala
                   165                 170                 175
   Ile Pro Arg Leu Arg Asp Ala Gly Leu Thr His Thr Leu Met Ile Asp
               180                 185                 190
   Ala Ala Gly Trp Gly Gln Phe Pro Ser Ser Ile His Gln Tyr Gly Arg
           195                 200                 205
   Glu Val Phe Asn Ala Asp Arg Leu Gly Asn Thr Met Phe Ser Ile His
       210                 215                 220
   Met Tyr Glu Tyr Ala Gly Gly Asp Asp Gln Met Val Arg Asp Asn Ile
   225                 230                 235                 240
   Asn Gly Val Ile Asn Gln Asp Leu Ala Leu Val Ile Gly Glu Phe Gly
                   245                 250                 255
   His Tyr His Thr Asp Gly Asp Val Asp Glu Asp Thr Ile Leu Ser Tyr
               260                 265                 270
   Ala Glu Gln Thr Gly Val Gly Trp Leu Ala Trp Ser Trp Lys Gly Asn
           275                 280                 285
   Gly Thr Glu Trp Glu Tyr Leu Asp Leu Ser Asn Asp Trp Gly Gly Asn
       290                 295                 300
   Tyr Leu Thr Ser Trp Gly Asp Arg Ile Val Asn Gly Ala Asn Gly Leu
   305                 310                 315                 320
   Arg Glu Thr Ser Gln Ile Ala Ser Val Phe Ser Gly Asn Asn Gly Gly
                   325                 330                 335
   Thr Pro Gly Asn Gly Glu Glu Glu Thr Pro Gly Asp Val Ser His Phe
               340                 345                 350
   Ala Asn Phe Glu Asn Gly Thr Glu Gly Trp Glu Ala Ser Asn Val Ser
           355                 360                 365
   Gly Gly Pro Trp Ala Thr Asn Glu Trp Ser Ala Ser Gly Ser Tyr Ala
       370                 375                 380
   Leu Lys Ala Asp Ala Gln Leu Ala Ser Gly Arg Glu His Tyr Leu Tyr
   385                 390                 395                 400
   Arg Ile Gly Pro Phe Asn Leu Ser Gly Ser Thr Leu Asn Ala Thr Val
                   405                 410                 415
   Arg Gly Ala Asn Trp Gly Asn Tyr Gly Ser Gly Ile Asp Val Lys Leu
               420                 425                 430
   Tyr Val Lys Tyr Gly Asp Gly Trp Thr Trp Arg Asp Ser Gly Val Gln
           435                 440                 445
   Thr Ile Arg Ala Gly Glu Ser Ile Asp Leu Ser Leu Asp Leu Ser Asn
       450                 455                 460
   Val Asp Arg Ser Asn Ile Arg Glu Val Gly Ile Gln Phe Ile Gly Gly
   465 470 475 480
   Asn His Ser Ser Gly Lys Thr Ala Phe Tyr Val Asp His Val Tyr Ser
   485 490 495
   His
<210> 31
   <211> 1767
   <212> DNA
   <213> Paenibacillus polymyxa
<400> 31
   atgaaaaaac tactgtcttg tctcatttcg ctgtcaatgc ttgtgtatat cttaccgaca 60
   atgatagtgt ccgctaacaa tgatggcgta acgaaccttg ctcttgattc aacacctagt 120
   gcgcaaagtg atattatttc tgatgctgtc tacaaaatca cagctcagca ttcaggaaaa 180
   agccttgagg ttgaaggcgg ttctaaagat gacggcgcga atgttcaaca atggacagat 240
   aacgggaaag aacagcagaa atggagagtt gtggacgtcg gtggcggata ttacaagctc 300
   atcagtcaat ctagcggaaa agcactggat gtggcaggtg gtaatacaca tgatggtgcc 360
   aatgtgcaac agtggacgga caacggaaat gctcagcaaa agtggaagat catcgatgta 420
   ggaggaggct attataagtt gatctcacaa agctctggaa aggcactcga cgtcgttggt 480
   ggttatacgc acgacggggc caatgtgcag caatgggcag acaatggatc tgctcaacag 540
   cgctggcgtt tcacacaaat tgatacaacc acggatacga cgccgccaac agcaccaacg 600
   aatttacaat catcatcgaa aacaagtacc tctgtaacat tgacttggac cacaagcatt 660
   gataatgtag gtgtgacagg ctatgtcatt tataatggaa cagatttggt cgggacttct 720
   acaactacat cttatattgt tacaggatta acagcgaaca cttcctataa cttcactgtc 780
   aaagcgaagg atgccgctgg gaatatttca gaaccatcaa atgtcttgaa agtcacaacg 840
   agttcagatt cttctcaaaa cacaggtttt tatgtgaagg gcacaacatt atatgatgga 900
   aacggtaatc catttgtgat gagaggaatc aatcatgcat acacatggta taaagggcaa 960
   gaatcagtag caattcctgc gattgcgaaa acgggtgcaa acaccatccg gattgtctta 1020
   tctgacggac agcagtggac aaaagatgat ttaagcgcgc ttcaaaattt gattacactc 1080
   agtgagcaaa acaaacttgt agtgatttta gaggtgcacg acggtactgg caatgacaat 1140
   gccgcagttt taaataaaat tgctgattat tggattgaaa tgaagtcagc tttaattggg 1200
   aaggaaaata cagttatttt aaacatcgca aatgaatggt ttggtacatg ggatggaaac 1260
   ggctgggcgc agggctacaa atcagtcata ccaaagctgc gaaatgcggg catcaaaaac 1320
   acgattatgg tggatgcggc tggatgggga caatatccaa aatcgatttt tgattacgga     1380
   acgcaagtgt tcgatgcaga tccgctcaag aatacgatgt tttccattca tatgtatgaa     1440
   tacgcaggcg gcaacgcaga aacagtgaaa agtaatatcg acaacgtcct gaataaaaat     1500
   cttgcactca tcattggaga atttggaatt aaacatacaa acggagatgt tgatgaagca     1560
   acgatcatgt catacgcaca gcaaaaaggt gttgggtatc ttggctggtc atggaaagga     1620
   aatggttcag gtcttgaata tttagatatg agtaacgatt gggctggcag cagttataca     1680
   gagcaaggac atgccattat cgaaggacca aatggcattc gtgcaacatc aaaattatca     1740
   accatttaca gcaatgggaa acaataa                                         1767
<210> 32
   <211> 588
   <212> PRT
   <213> Paenibacillus polymyxa
<400> 32
   Met Lys Lys Leu Leu Ser Cys Leu Ile Ser Leu Ser Met Leu Val Tyr
   1               5                   10                  15
   Ile Leu Pro Thr Met Ile Val Ser Ala Asn Asn Asp Gly Val Thr Asn
               20                  25                  30
   Leu Ala Leu Asp Ser Thr Pro Ser Ala Gln Ser Asp Ile Ile Ser Asp
           35                  40                  45
   Ala Val Tyr Lys Ile Thr Ala Gln His Ser Gly Lys Ser Leu Glu Val
       50                  55                  60
   Glu Gly Gly Ser Lys Asp Asp Gly Ala Asn Val Gln Gln Trp Thr Asp
   65                  70                  75                  80
   Asn Gly Lys Glu Gln Gln Lys Trp Arg Val Val Asp Val Gly Gly Gly
                   85                  90                  95
   Tyr Tyr Lys Leu Ile Ser Gln Ser Ser Gly Lys Ala Leu Asp Val Ala
               100                 105                 110
   Gly Gly Asn Thr His Asp Gly Ala Asn Val Gln Gln Trp Thr Asp Asn
           115                 120                 125
   Gly Asn Ala Gln Gln Lys Trp Lys Ile Ile Asp Val Gly Gly Gly Tyr
       130                 135                 140
   Tyr Lys Leu Ile Ser Gln Ser Ser Gly Lys Ala Leu Asp Val Val Gly
   145                 150                 155                 160
   Gly Tyr Thr His Asp Gly Ala Asn Val Gln Gln Trp Ala Asp Asn Gly
                   165                 170                 175
   Ser Ala Gln Gln Arg Trp Arg Phe Thr Gln Ile Asp Thr Thr Thr Asp
               180                 185                 190
   Thr Thr Pro Pro Thr Ala Pro Thr Asn Leu Gln Ser Ser Ser Lys Thr
           195                 200                 205
   Ser Thr Ser Val Thr Leu Thr Trp Thr Thr Ser Ile Asp Asn Val Gly
       210                 215                 220
   Val Thr Gly Tyr Val Ile Tyr Asn Gly Thr Asp Leu Val Gly Thr Ser
   225                 230                 235                 240
   Thr Thr Thr Ser Tyr Ile Val Thr Gly Leu Thr Ala Asn Thr Ser Tyr
                   245                 250                 255
   Asn Phe Thr Val Lys Ala Lys Asp Ala Ala Gly Asn Ile Ser Glu Pro
               260                 265                 270
   Ser Asn Val Leu Lys Val Thr Thr Ser Ser Asp Ser Ser Gln Asn Thr
           275                 280                 285
   Gly Phe Tyr Val Lys Gly Thr Thr Leu Tyr Asp Gly Asn Gly Asn Pro
       290                 295                 300
   Phe Val Met Arg Gly Ile Asn His Ala Tyr Thr Trp Tyr Lys Gly Gln
   305                 310                 315                 320
   Glu Ser Val Ala Ile Pro Ala Ile Ala Lys Thr Gly Ala Asn Thr Ile
                   325                 330                 335
   Arg Ile Val Leu Ser Asp Gly Gln Gln Trp Thr Lys Asp Asp Leu Ser
               340                 345                 350
   Ala Leu Gln Asn Leu Ile Thr Leu Ser Glu Gln Asn Lys Leu Val Val
           355                 360                 365
   Ile Leu Glu Val His Asp Gly Thr Gly Asn Asp Asn Ala Ala Val Leu
       370                 375                 380
   Asn Lys Ile Ala Asp Tyr Trp Ile Glu Met Lys Ser Ala Leu Ile Gly
   385                 390                 395                 400
   Lys Glu Asn Thr Val Ile Leu Asn Ile Ala Asn Glu Trp Phe Gly Thr
                   405                 410                 415
   Trp Asp Gly Asn Gly Trp Ala Gln Gly Tyr Lys Ser Val Ile Pro Lys
               420                 425                 430
   Leu Arg Asn Ala Gly Ile Lys Asn Thr Ile Met Val Asp Ala Ala Gly
           435                 440                 445
   Trp Gly Gln Tyr Pro Lys Ser Ile Phe Asp Tyr Gly Thr Gln Val Phe
       450                 455                 460
   Asp Ala Asp Pro Leu Lys Asn Thr Met Phe Ser Ile His Met Tyr Glu
   465                 470                 475                 480
   Tyr Ala Gly Gly Asn Ala Glu Thr Val Lys Ser Asn Ile Asp Asn Val
                   485                 490                 495
   Leu Asn Lys Asn Leu Ala Leu Ile Ile Gly Glu Phe Gly Ile Lys His
               500                 505                 510
   Thr Asn Gly Asp Val Asp Glu Ala Thr Ile Met Ser Tyr Ala Gln Gln
           515                 520                 525
   Lys Gly Val Gly Tyr Leu Gly Trp Ser Trp Lys Gly Asn Gly Ser Gly
       530                 535                 540
   Leu Glu Tyr Leu Asp Met Ser Asn Asp Trp Ala Gly Ser Ser Tyr Thr
   545                 550                 555                 560
   Glu Gln Gly His Ala Ile Ile Glu Gly Pro Asn Gly Ile Arg Ala Thr
                   565                 570                 575
   Ser Lys Leu Ser Thr Ile Tyr Ser Asn Gly Lys Gln
               580                 585
<210> 33
   <211> 1470
   <212> DNA
   <213> Bacillus hemicellulosilyticus
<400> 33
   atggatatat taagaaagtg tgtacttgta ctattggcct tactattgtt gttacctacg      60
   acatcaacgg cattttctga aagcgcttct actaatgaga gagtgctaaa tttatctgat     120
   ccgaatgcga cacgctatac gaaggaattg tttgcgtttc ttcaagacgt gagtggtgag     180
   caagtgttgt tcgggcaaca gcatgcaaca gatgaagggt tgactctgac aggtgaagga     240
   aatcgaattg gttcaactga gtcggaggtg aagaatgcag taggtgatta tccagctgtt     300
   tttgggtggg atacgaacag cttggatggt cgtgaaaagc caggtacaga tgtggaaagt     360
   caagagcaac gaattttaaa tacagcagaa tcgatgaaag tggcacatga attaggaggg     420
   atcatcacat taagtatgca tccggataac tttgttaccg gtcattacta tggcgatacg     480
   gatggtaacg tcgttcaaga aatattgcca ggtggctcca agcacaatga atttaacgct     540
   tggctagata atattgctgc cctagcacat gaattagttg atgataatgg agagcctatt     600
   ccggttatct tccgtccatt ccatgagcaa acaggttcgt ggttttggtg gggtgcgagc     660
   acaacaactc ctgagcaata caaagcgatt tttcgatata cagtcgaata cttaagagat     720
   gcaaagggtg ttcataactt tttatatgga ttctcccctg gtgcgggtcc tgctggcgat     780
   ctagatcgat atttagaaac gtacccaggt gataattatg tcgatatctt aggtattgat     840
   aattatgata gtaagtcaaa tgcggggtca gacgcttggt tatctggaat ggtaaaagat     900
   ttagcgatga tctcgaaatt agcagaggaa agagggaagg tatcagcctt tactgaattt     960
   ggatacagcg ctgaagggat gagtcaaacg ggtgatgcgt tagattggta tacacgtgtg    1020
   ttaaatgcga taaaagcaga tgaagatgcg cgaaacatat cctacatgct aacgtgggct    1080
   aactttgggt ggcctaataa tatatttgtt ccgtatcgtg atgtgaatgg ggatttaggt    1140
   ggagatcatg agttattacc tgactttgta cagttttatg aagatgaata ctcagcattt    1200
   cgtgaagata taaatgaaag tgtttacaat cgtaatgaga gttatattgt tgcggatcat    1260
   gagccattta tgtatgttgt ttcccctacg acaggtacat atataacagg ctcgtctgtt    1320
   gtcttacgag cgaaagtagt taacgatgag gatccgtccg ttacgtatca agtggcgggt    1380
   tctgaagaag tctatgagat gactttagat gaaaatgggt attactctgc tgattatatt    1440
   cctactgctc ctaagaatgg agctctgtag                                     1470
<210> 34
   <211> 489
   <212> PRT
   <213> Bacillus hemicellulosilyticus
<400> 34
   Met Asp Ile Leu Arg Lys Cys Val Leu Val Leu Leu Ala Leu Leu Leu
   1               5                   10                  15
   Leu Leu Pro Thr Thr Ser Thr Ala Phe Ser Glu Ser Ala Ser Thr Asn
               20                  25                  30
   Glu Arg Val Leu Asn Leu Ser Asp Pro Asn Ala Thr Arg Tyr Thr Lys
           35                  40                  45
   Glu Leu Phe Ala Phe Leu Gln Asp Val Ser Gly Glu Gln Val Leu Phe
       50                  55                  60
   Gly Gln Gln His Ala Thr Asp Glu Gly Leu Thr Leu Thr Gly Glu Gly
   65                  70                  75                  80
   Asn Arg Ile Gly Ser Thr Glu Ser Glu Val Lys Asn Ala Val Gly Asp
                   85                  90                  95
   Tyr Pro Ala Val Phe Gly Trp Asp Thr Asn Ser Leu Asp Gly Arg Glu
               100                 105                 110
   Lys Pro Gly Thr Asp Val Glu Ser Gln Glu Gln Arg Ile Leu Asn Thr
           115                 120                 125
   Ala Glu Ser Met Lys Val Ala His Glu Leu Gly Gly Ile Ile Thr Leu
       130                 135                 140
   Ser Met His Pro Asp Asn Phe Val Thr Gly His Tyr Tyr Gly Asp Thr
   145                 150                 155                 160
   Asp Gly Asn Val Val Gln Glu Ile Leu Pro Gly Gly Ser Lys His Asn
                   165                 170                 175
   Glu Phe Asn Ala Trp Leu Asp Asn Ile Ala Ala Leu Ala His Glu Leu
               180                 185                 190
   Val Asp Asp Asn Gly Glu Pro Ile Pro Val Ile Phe Arg Pro Phe His
           195                 200                 205
   Glu Gln Thr Gly Ser Trp Phe Trp Trp Gly Ala Ser Thr Thr Thr Pro
       210                 215                 220
   Glu Gln Tyr Lys Ala Ile Phe Arg Tyr Thr Val Glu Tyr Leu Arg Asp
   225                 230                 235                 240
   Ala Lys Gly Val His Asn Phe Leu Tyr Gly Phe Ser Pro Gly Ala Gly
                   245                 250                 255
   Pro Ala Gly Asp Leu Asp Arg Tyr Leu Glu Thr Tyr Pro Gly Asp Asn
               260                 265                 270
   Tyr Val Asp Ile Leu Gly Ile Asp Asn Tyr Asp Ser Lys Ser Asn Ala
           275                 280                 285
   Gly Ser Asp Ala Trp Leu Ser Gly Met Val Lys Asp Leu Ala Met Ile
       290                 295                 300
   Ser Lys Leu Ala Glu Glu Arg Gly Lys Val Ser Ala Phe Thr Glu Phe
   305                 310                 315                 320
   Gly Tyr Ser Ala Glu Gly Met Ser Gln Thr Gly Asp Ala Leu Asp Trp
                   325                 330                 335
   Tyr Thr Arg Val Leu Asn Ala Ile Lys Ala Asp Glu Asp Ala Arg Asn
               340                 345                 350
   Ile Ser Tyr Met Leu Thr Trp Ala Asn Phe Gly Trp Pro Asn Asn Ile
           355                 360                 365
   Phe Val Pro Tyr Arg Asp Val Asn Gly Asp Leu Gly Gly Asp His Glu
       370                 375                 380
   Leu Leu Pro Asp Phe Val Gln Phe Tyr Glu Asp Glu Tyr Ser Ala Phe
   385                 390                 395                 400
   Arg Glu Asp Ile Asn Glu Ser Val Tyr Asn Arg Asn Glu Ser Tyr Ile
                   405                 410                 415
   Val Ala Asp His Glu Pro Phe Met Tyr Val Val Ser Pro Thr Thr Gly
               420                 425                 430
   Thr Tyr Ile Thr Gly Ser Ser Val Val Leu Arg Ala Lys Val Val Asn
           435                 440                 445
   Asp Glu Asp Pro Ser Val Thr Tyr Gln Val Ala Gly Ser Glu Glu Val
       450                 455                 460
   Tyr Glu Met Thr Leu Asp Glu Asn Gly Tyr Tyr Ser Ala Asp Tyr Ile
   465                 470                 475                 480
   Pro Thr Ala Pro Lys Asn Gly Ala Leu
                   485
<210> 35
   <211> 1110
   <212> DNA
   <213> Bacillus alcalophilus
<400> 35
   atgagaagta tgaagctttt atttgctatg tttattttag ttttttcctc ttttactttt      60
   aacttagtag ttgcgcaagc tagtggacat ggacaaatgc ataaagtacc ttgggcaccc     120
   caagctgaag cacctggaaa aacggctgag aatggagtct gggataaagt tagaaataat     180
   cctggaaaag ccaatcctcc agcaggaaaa gtcaatggtt tttatataga tggaacaacc     240
   ttatatgatg caaatggtaa gccatttgtg atgcgcggaa ttaaccacgc tcattcctgg     300
   tacaagcctc acatagaaac cgcgatggag gcaattgctg atactggagc aaactccatt     360
   cgtgtagttc tctcagatgg acaacagtgg accaaagatg atgttgacga agtagcaaaa     420
   attatatctt tagcagaaaa acattcttta gttgctgttc ttgaggtaca tgatgcactc     480
   ggaacagatg atattgaacc attacttaaa acagtcgatt actggattga gatcaaagat     540
   gctttaatcg gaaaagagga caaagtaatt attaacattt ctaatgaatg gtttggttct     600
   tggagcagtg aaggttgggc agaaggatat aaaaaagcaa ttcctttact aagagaggcg     660
   ggtctaaaac atacacttat ggttgacgca gctgggtggg gacaatttcc tagatctatt     720
   catgaaaaag gattagacgt ttttaactca gacccattaa agaatacaat gttttccatt     780
   catatgtatg aatgggcagc gggtaatcct caacaagtaa aagacaatat tgacggtgtt     840
   cttgaaaaga atttagctgt agtaattggt gagttcggtc atcatcacta cggaagagat     900
   gttgctgttg atacgatctt aagtcattca gagaagtatg atgtaggttg gcttgcctgg     960
   tcttggcacg gaaatagtgg tggtgtagag tatcttgact tagcaacaga tttttcaggg    1020
   acgcaactaa ctgaatgggg agaaagaatt gtgtacggtc cgaatggttt aaaagaaact    1080
   tctgaaatcg ttagtgtata caaaaaataa                                     1110
<210> 36
   <211> 369
   <212> PRT
   <213> Bacillus alcalophilus
<400> 36
   Met Arg Ser Met Lys Leu Leu Phe Ala Met Phe Ile Leu Val Phe Ser
   1               5                   10                  15
   Ser Phe Thr Phe Asn Leu Val Val Ala Gln Ala Ser Gly His Gly Gln
               20                  25                  30
   Met His Lys Val Pro Trp Ala Pro Gln Ala Glu Ala Pro Gly Lys Thr
           35                  40                  45
   Ala Glu Asn Gly Val Trp Asp Lys Val Arg Asn Asn Pro Gly Lys Ala
       50                  55                  60
   Asn Pro Pro Ala Gly Lys Val Asn Gly Phe Tyr Ile Asp Gly Thr Thr
   65                  70                  75                  80
   Leu Tyr Asp Ala Asn Gly Lys Pro Phe Val Met Arg Gly Ile Asn His
                   85                  90                  95
   Ala His Ser Trp Tyr Lys Pro His Ile Glu Thr Ala Met Glu Ala Ile
               100                 105                 110
   Ala Asp Thr Gly Ala Asn Ser Ile Arg Val Val Leu Ser Asp Gly Gln
           115                 120                 125
   Gln Trp Thr Lys Asp Asp Val Asp Glu Val Ala Lys Ile Ile Ser Leu
       130                 135                 140
   Ala Glu Lys His Ser Leu Val Ala Val Leu Glu Val His Asp Ala Leu
   145                 150                 155                 160
   Gly Thr Asp Asp Ile Glu Pro Leu Leu Lys Thr Val Asp Tyr Trp Ile
                   165                 170                 175
   Glu Ile Lys Asp Ala Leu Ile Gly Lys Glu Asp Lys Val Ile Ile Asn
               180                 185                 190
   Ile Ser Asn Glu Trp Phe Gly Ser Trp Ser Ser Glu Gly Trp Ala Glu
           195                 200                 205
   Gly Tyr Lys Lys Ala Ile Pro Leu Leu Arg Glu Ala Gly Leu Lys His
       210                 215                 220
   Thr Leu Met Val Asp Ala Ala Gly Trp Gly Gln Phe Pro Arg Ser Ile
   225                 230                 235                 240
   His Glu Lys Gly Leu Asp Val Phe Asn Ser Asp Pro Leu Lys Asn Thr
                   245                 250                 255
   Met Phe Ser Ile His Met Tyr Glu Trp Ala Ala Gly Asn Pro Gln Gln
               260                 265                 270
   Val Lys Asp Asn Ile Asp Gly Val Leu Glu Lys Asn Leu Ala Val Val
           275                 280                 285
   Ile Gly Glu Phe Gly His His His Tyr Gly Arg Asp Val Ala Val Asp
       290                 295                 300
   Thr Ile Leu Ser His Ser Glu Lys Tyr Asp Val Gly Trp Leu Ala Trp
   305                 310                 315                 320
   Ser Trp His Gly Asn Ser Gly Gly Val Glu Tyr Leu Asp Leu Ala Thr
                   325                 330                 335
   Asp Phe Ser Gly Thr Gln Leu Thr Glu Trp Gly Glu Arg Ile Val Tyr
               340                 345                 350
   Gly Pro Asn Gly Leu Lys Glu Thr Ser Glu Ile Val Ser Val Tyr Lys
           355                 360                 365
   Lys
<210> 37
   <211> 1482
   <212> DNA
   <213> Bacillus sp.
<400> 37
   atgaaaaaaa agttatcaca gatttatcat ttaattattt gcacacttat aataagtgtg      60
   ggaataatgg ggattacaac gtccccatca gaagcaagtt caggctttta tgttgatggc     120
   aatacgttat atgacgcaaa cgggcaacca tttgtcatga aaggcattaa ccatggacat     180
   gcttggtata aagacaccgc ttcaacagct attcctgcca ttgcagagca aggcgcgaac     240
   acgatacgta ttgttttatc agatggcggt caatgggaaa aagacgacat tgacaccgtt     300
   cgtgaagtta ttgagcttgc ggagcaaaat aaaatggtgg ctgtcgttga agttcatgat     360
   gccacgggcc gtgattcacg cagtgattta gatcgggcag tcgattattg gatagagatg     420
   aaagatgcac ttatcggcaa agaggatact gtcattatta acattgcaaa cgaatggtat     480
   ggcagttggg atggcgccgc ttgggctgat ggctacattg atgtcattcc gaagcttcgc     540
   gatgccggct taacacacac cttaatggtt gatgcagcag gatgggggca atatccgcaa     600
   tctattcatg attacggaca agatgtgttt aatgcagatc cgttaaaaaa tacgatattc     660
   tccatccata tgtatgagta tgctggtggt gatgctaaca ctgttagatc aaatattgat     720
   agagtcatag atcaagacct tgctctcgta ataggtgagt tcggtcatag acacactgat     780
   ggcgatgttg atgaagatac aatccttagt tattctgaag aaactggcac aggatggctc     840
   gcttggtctt ggaaaggcaa cagtgccgaa tgggattatt tagacctttc agaagattgg     900
   gctggtaacc atttaactga ttggggaaat aggattgtcc acggggcaaa tggcttgcag     960
   gaaacctcca aaccatccac cgtatttaca gatgataacg gtggtgcccc tgaaccgcca    1020
   actactacta ccttgtatga ctttgaagga agcacacaag ggtggcatgg aagcaacgtg    1080
   atgggtggcc cttggtccgt aacagaatgg ggtgcgtcag gcaactactc tttaaagggc    1140
   gatgtcaatt taagctcaaa ttcttcacat gaactgtata gtgaacaaag tcgtaatcta    1200
   cacggatact ctcagctaaa tgcaaccgtt cgccatgcca attggggaaa tcccggtaat    1260
   ggcatgaatg caagacttta cgtgaaaacg ggctctgatt atacatggta tagcggtcct    1320
   tttacacgta tcaatagctc caactcaggt acaacgttat cttttgattt aaacaacatc    1380
   gaaaatagtc atcatgttag ggaaataggt gtgcaatttt cagctgcaga taatagcagc    1440
   ggtcaaactg ctctatacgt tgataatgtt actttaagat aa                       1482
<210> 38
   <211> 493
   <212> PRT
   <213> Bacillus sp.
<400> 38
   Met Lys Lys Lys Leu Ser Gln Ile Tyr His Leu Ile Ile Cys Thr Leu
   1               5                   10                  15
   Ile Ile Ser Val Gly Ile Met Gly Ile Thr Thr Ser Pro Ser Glu Ala
               20                  25                  30
   Ser Ser Gly Phe Tyr Val Asp Gly Asn Thr Leu Tyr Asp Ala Asn Gly
           35                  40                  45
   Gln Pro Phe Val Met Lys Gly Ile Asn His Gly His Ala Trp Tyr Lys
       50                  55                  60
   Asp Thr Ala Ser Thr Ala Ile Pro Ala Ile Ala Glu Gln Gly Ala Asn
   65                  70                  75                  80
   Thr Ile Arg Ile Val Leu Ser Asp Gly Gly Gln Trp Glu Lys Asp Asp
                   85                  90                  95
   Ile Asp Thr Val Arg Glu Val Ile Glu Leu Ala Glu Gln Asn Lys Met
               100                 105                 110
   Val Ala Val Val Glu Val His Asp Ala Thr Gly Arg Asp Ser Arg Ser
           115                 120                 125
   Asp Leu Asp Arg Ala Val Asp Tyr Trp Ile Glu Met Lys Asp Ala Leu
       130                 135                 140
   Ile Gly Lys Glu Asp Thr Val Ile Ile Asn Ile Ala Asn Glu Trp Tyr
   145                 150                 155                 160
   Gly Ser Trp Asp Gly Ala Ala Trp Ala Asp Gly Tyr Ile Asp Val Ile
                   165                 170                 175
   Pro Lys Leu Arg Asp Ala Gly Leu Thr His Thr Leu Met Val Asp Ala
               180                 185                 190
   Ala Gly Trp Gly Gln Tyr Pro Gln Ser Ile His Asp Tyr Gly Gln Asp
           195                 200                 205
   Val Phe Asn Ala Asp Pro Leu Lys Asn Thr Ile Phe Ser Ile His Met
       210                 215                 220
   Tyr Glu Tyr Ala Gly Gly Asp Ala Asn Thr Val Arg Ser Asn Ile Asp
   225 230 235 240
   Arg Val Ile Asp Gln Asp Leu Ala Leu Val Ile Gly Glu Phe Gly His
   245 250 255
   Arg His Thr Asp Gly Asp Val Asp Glu Asp Thr Ile Leu Ser Tyr Ser
   260 265 270
   Glu Glu Thr Gly Thr Gly Trp Leu Ala Trp Ser Trp Lys Gly Asn Ser
   275 280 285
   Ala Glu Trp Asp Tyr Leu Asp Leu Ser Glu Asp Trp Ala Gly Asn His
   290 295 300
   Leu Thr Asp Trp Gly Asn Arg Ile Val His Gly Ala Asn Gly Leu Gln
   305 310 315 320
   Glu Thr Ser Lys Pro Ser Thr Val Phe Thr Asp Asp Asn Gly Gly Ala
   325 330 335
   Pro Glu Pro Pro Thr Thr Thr Thr Leu Tyr Asp Phe Glu Gly Ser Thr
   340 345 350
   Gln Gly Trp His Gly Ser Asn Val Met Gly Gly Pro Trp Ser Val Thr
   355 360 365
   Glu Trp Gly Ala Ser Gly Asn Tyr Ser Leu Lys Gly Asp Val Asn Leu
   370 375 380
   Ser Ser Asn Ser Ser His Glu Leu Tyr Ser Glu Gln Ser Arg Asn Leu
   385 390 395 400
   His Gly Tyr Ser Gln Leu Asn Ala Thr Val Arg His Ala Asn Trp Gly
   405 410 415
   Asn Pro Gly Asn Gly Met Asn Ala Arg Leu Tyr Val Lys Thr Gly Ser
   420 425 430
   Asp Tyr Thr Trp Tyr Ser Gly Pro Phe Thr Arg Ile Asn Ser Ser Asn
   435 440 445
   Ser Gly Thr Thr Leu Ser Phe Asp Leu Asn Asn Ile Glu Asn Ser His
   450 455 460
   His Val Arg Glu Ile Gly Val Gln Phe Ser Ala Ala Asp Asn Ser Ser
   465 470 475 480
   Gly Gln Thr Ala Leu Tyr Val Asp Asn Val Thr Leu Arg
   485 490
<210> 39
   <211> 1551
   <212> DNA
   <213> Bacillus circulans
<400> 39
   atggggtggt ttttagtgat tttacgcaag tggttgattg cttttgtcgc atttttactg 60
   atgttctcgt ggactggaca acttacgaac aaagcacatg ctgcaagcgg attttatgta 120
   agcggtacca aattattgga tgctacagga caaccatttg tgatgcgagg agtcaatcat 180
   gcgcacacat ggtataaaga tcaactatcc accgcaatac cagccattgc taaaacaggt 240
   gccaacacga tacgtattgt actggcgaat ggacacaaat ggacgcttga tgatgtaaac 300
   accgtcaaca atattctcac cctctgtgaa caaaacaaac taattgccgt tttggaagta 360
   catgacgcta caggaagcga tagtctttcc gatttagaca acgccgttaa ttactggatt 420
   ggtattaaaa gcgcgttgat cggcaaggaa gaccgtgtaa tcattaatat agctaacgag 480
   tggtacggaa catgggatgg agtcgcctgg gctaatggtt ataagcaagc catacccaaa 540
   ctgcgtaatg ctggtctaac tcatacgctg attgttgact ccgctggatg gggacaatat 600
   ccagattcgg tcaaaaatta tgggacagaa gtactgaatg cagacccgtt aaaaaacaca 660
   gtattctcta tccatatgta tgaatatgct gggggcaatg caagtaccgt caaatccaat 720
   attgacggtg tgctgaacaa gaatcttgca ctgattatcg gcgaatttgg tggacaacat 780
   acaaacggtg atgtggatga agccaccatt atgagttatt cccaagagaa gggagtcggc 840
   tggttggctt ggtcctggaa gggaaatagc agtgatttgg cttatctcga tatgacaaat 900
   gattgggctg gtaactccct cacctcgttc ggtaataccg tagtgaatgg cagtaacggc 960
   attaaagcaa cttctgtgtt atccggcatt tttggaggtg ttacgccaac ctcaagccct 1020
   acttctacac ctacatctac gccaacctca actcctactc ctacgccaag tccgaccccg 1080
   agtccaggta ataacgggac gatcttatat gatttcgaaa caggaactca aggctggtcg 1140
   ggaaacaata tttcgggagg cccatgggtc accaatgaat ggaaagcaac gggagcgcaa 1200
   actctcaaag ccgatgtctc cttacaatcc aattccacgc atagtctata tataacctct 1260
   aatcaaaatc tgtctggaaa aagcagtctg aaagcaacgg ttaagcatgc gaactggggc 1320
   aatatcggca acgggattta tgcaaaacta tacgtaaaga ccgggtccgg gtggacatgg 1380
   tacgattccg gagagaatct gattcagtca aacgacggta ccattttgac actatccctc 1440
   agcggcattt cgaatttgtc ctcagtcaaa gaaattgggg tagaattccg cgcctcctca 1500
   aacagtagtg gccaatcagc tatttatgta gatagtgtta gtctgcaata a 1551
<210> 40
   <211> 516
   <212> PRT
   <213> Bacillus circulans
<400> 40
   Met Gly Trp Phe Leu Val Ile Leu Arg Lys Trp Leu Ile Ala Phe Val
   1               5                   10                  15
   Ala Phe Leu Leu Met Phe Ser Trp Thr Gly Gln Leu Thr Asn Lys Ala
               20                  25                  30
   His Ala Ala Ser Gly Phe Tyr Val Ser Gly Thr Lys Leu Leu Asp Ala
           35                  40                  45
   Thr Gly Gln Pro Phe Val Met Arg Gly Val Asn His Ala His Thr Trp
       50                  55                  60
   Tyr Lys Asp Gln Leu Ser Thr Ala Ile Pro Ala Ile Ala Lys Thr Gly
   65                  70                  75                  80
   Ala Asn Thr Ile Arg Ile Val Leu Ala Asn Gly His Lys Trp Thr Leu
                   85                  90                  95
   Asp Asp Val Asn Thr Val Asn Asn Ile Leu Thr Leu Cys Glu Gln Asn
               100                 105                 110
   Lys Leu Ile Ala Val Leu Glu Val His Asp Ala Thr Gly Ser Asp Ser
           115                 120                 125
   Leu Ser Asp Leu Asp Asn Ala Val Asn Tyr Trp Ile Gly Ile Lys Ser
       130                 135                 140
   Ala Leu Ile Gly Lys Glu Asp Arg Val Ile Ile Asn Ile Ala Asn Glu
   145                 150                 155                 160
   Trp Tyr Gly Thr Trp Asp Gly Val Ala Trp Ala Asn Gly Tyr Lys Gln
                   165                 170                 175
   Ala Ile Pro Lys Leu Arg Asn Ala Gly Leu Thr His Thr Leu Ile Val
               180                 185                 190
   Asp Ser Ala Gly Trp Gly Gln Tyr Pro Asp Ser Val Lys Asn Tyr Gly
           195                 200                 205
   Thr Glu Val Leu Asn Ala Asp Pro Leu Lys Asn Thr Val Phe Ser Ile
       210                 215                 220
   His Met Tyr Glu Tyr Ala Gly Gly Asn Ala Ser Thr Val Lys Ser Asn
   225 230 235 240
   Ile Asp Gly Val Leu Asn Lys Asn Leu Ala Leu Ile Ile Gly Glu Phe
   245 250 255
   Gly Gly Gln His Thr Asn Gly Asp Val Asp Glu Ala Thr Ile Met Ser
   260 265 270
   Tyr Ser Gln Glu Lys Gly Val Gly Trp Leu Ala Trp Ser Trp Lys Gly
   275 280 285
   Asn Ser Ser Asp Leu Ala Tyr Leu Asp Met Thr Asn Asp Trp Ala Gly
   290 295 300
   Asn Ser Leu Thr Ser Phe Gly Asn Thr Val Val Asn Gly Ser Asn Gly
   305 310 315 320
   Ile Lys Ala Thr Ser Val Leu Ser Gly Ile Phe Gly Gly Val Thr Pro
   325 330 335
   Thr Ser Ser Pro Thr Ser Thr Pro Thr Ser Thr Pro Thr Ser Thr Pro
   340 345 350
   Thr Pro Thr Pro Ser Pro Thr Pro Ser Pro Gly Asn Asn Gly Thr Ile
   355 360 365
   Leu Tyr Asp Phe Glu Thr Gly Thr Gln Gly Trp Ser Gly Asn Asn Ile
   370 375 380
   Ser Gly Gly Pro Trp Val Thr Asn Glu Trp Lys Ala Thr Gly Ala Gln
   385 390 395 400
   Thr Leu Lys Ala Asp Val Ser Leu Gln Ser Asn Ser Thr His Ser Leu
   405 410 415
   Tyr Ile Thr Ser Asn Gln Asn Leu Ser Gly Lys Ser Ser Leu Lys Ala
   420 425 430
   Thr Val Lys His Ala Asn Trp Gly Asn Ile Gly Asn Gly Ile Tyr Ala
   435 440 445
   Lys Leu Tyr Val Lys Thr Gly Ser Gly Trp Thr Trp Tyr Asp Ser Gly
   450 455 460
   Glu Asn Leu Ile Gln Ser Asn Asp Gly Thr Ile Leu Thr Leu Ser Leu
   465 470 475 480
   Ser Gly Ile Ser Asn Leu Ser Ser Val Lys Glu Ile Gly Val Glu Phe
   485 490 495
   Arg Ala Ser Ser Asn Ser Ser Gly Gln Ser Ala Ile Tyr Val Asp Ser
   500 505 510
   Val Ser Leu Gln
   515
<210> 41
   <211> 984
   <212> DNA
   <213> Paenibacillus sp.
<400> 41
   atgagacaac ttttagcaaa aggtatttta gctgcactgg tcatgatgtt agcgatgtat 60
   ggattgggga atctctcttc taaagcttcg gctgcaacag gtttttatgt aagcggtacc 120
   actctatatg attctactgg taaacctttt gtaatgcgcg gtgtcaatca ttcgcatacc 180
   tggttcaaaa atgatctaaa tgcagccatc cctgctattg ccaaaacagg tgcaaataca 240
   gtacgtatcg ttttatctaa tggtgttcag tatactagag atgatgtaaa ctcagtcaaa 300
   aatattattt ccctggttaa ccaaaacaaa atgattgctg ttcttgaggt gcatgatgct 360
   accggtaaag acgattacgc ttctcttgat gccgctgtaa actactggat cagcatcaaa 420
   gatgccttga ttggcaagga agatcgagtc attgttaata ttgccaatga atggtacggt 480
   acatggaatg gcagtgcttg ggcagatggt tataagcagg ctattcccaa actaagaaat 540
   gcaggcatca aaaacacttt aatcgttgat gccgccggct ggggacaatg tcctcaatcg 600
   atcgttgatt acgggcaaag tgtatttgca gcagattcgc ttaaaaatac aattttctct 660
   attcacatgt atgaatatgc aggcggtaca gatgcgatcg tcaaaagcaa tatggaaaat 720
   gtactgaaca aaggacttcc tttgatcatc ggtgaatttg gcgggcagca tacaaacggc 780
   gatgtagatg aacatgcaat tatgcgttat ggtcagcaaa aaggtgtagg ttggctggca 840
   tggtcgtggt atggcaacaa tagtgaactc agttatctgg atttggctac aggtcccgcc 900
   ggtagtctga caagtatcgg caatacgatt gtaaatgatc catatggtat caaagctacc 960
   tcgaaaaaag cgggtatctt ctaa 984
<210> 42
   <211> 327
   <212> PRT
   <213> Paenibacillus sp.
<400> 42
   Met Arg Gln Leu Leu Ala Lys Gly Ile Leu Ala Ala Leu Val Met Met
   1               5                   10                  15
   Leu Ala Met Tyr Gly Leu Gly Asn Leu Ser Ser Lys Ala Ser Ala Ala
   20 25 30
   Thr Gly Phe Tyr Val Ser Gly Thr Thr Leu Tyr Asp Ser Thr Gly Lys
   35 40 45
   Pro Phe Val Met Arg Gly Val Asn His Ser His Thr Trp Phe Lys Asn
   50 55 60
   Asp Leu Asn Ala Ala Ile Pro Ala Ile Ala Lys Thr Gly Ala Asn Thr
   65 70 75 80
   Val Arg Ile Val Leu Ser Asn Gly Val Gln Tyr Thr Arg Asp Asp Val
   85 90 95
   Asn Ser Val Lys Asn Ile Ile Ser Leu Val Asn Gln Asn Lys Met Ile
   100 105 110
   Ala Val Leu Glu Val His Asp Ala Thr Gly Lys Asp Asp Tyr Ala Ser
   115 120 125
   Leu Asp Ala Ala Val Asn Tyr Trp Ile Ser Ile Lys Asp Ala Leu Ile
   130 135 140
   Gly Lys Glu Asp Arg Val Ile Val Asn Ile Ala Asn Glu Trp Tyr Gly
   145 150 155 160
   Thr Trp Asn Gly Ser Ala Trp Ala Asp Gly Tyr Lys Gln Ala Ile Pro
   165 170 175
   Lys Leu Arg Asn Ala Gly Ile Lys Asn Thr Leu Ile Val Asp Ala Ala
   180 185 190
   Gly Trp Gly Gln Cys Pro Gln Ser Ile Val Asp Tyr Gly Gln Ser Val
   195 200 205
   Phe Ala Ala Asp Ser Leu Lys Asn Thr Ile Phe Ser Ile His Met Tyr
   210 215 220
   Glu Tyr Ala Gly Gly Thr Asp Ala Ile Val Lys Ser Asn Met Glu Asn
   225 230 235 240
   Val Leu Asn Lys Gly Leu Pro Leu Ile Ile Gly Glu Phe Gly Gly Gln
   245 250 255
   His Thr Asn Gly Asp Val Asp Glu His Ala Ile Met Arg Tyr Gly Gln
           260                 265                 270
   Gln Lys Gly Val Gly Trp Leu Ala Trp Ser Trp Tyr Gly Asn Asn Ser
           275                 280                 285
   Glu Leu Ser Tyr Leu Asp Leu Ala Thr Gly Pro Ala Gly Ser Leu Thr
       290                 295                 300
   Ser Ile Gly Asn Thr Ile Val Asn Asp Pro Tyr Gly Ile Lys Ala Thr
   305                 310                 315                 320
   Ser Lys Lys Ala Gly Ile Phe
                   325
<210> 43
   <211> 981
   <212> DNA
   <213> Bacillus circulans
<400> 43
   atggccaagt tgcaaaaggg tacaatctta acagtcattg cagcactgat gtttgtcatt      60
   ttggggagcg cggcgcccaa agccgcagca gctacaggtt tttacgtgaa tggaggcaaa     120
   ttgtacgatt ctacgggtaa accattttac atgaggggta tcaatcatgg gcactcctgg     180
   tttaaaaatg atttgaacac ggctatccct gcgatcgcaa aaacgggtgc caatacggta     240
   cgaattgttt tatcaaacgg tacacaatac accaaggatg atctgaattc cgtaaaaaac     300
   atcattaatg tcgtaaatgc aaacaagatg attgctgtgc ttgaagtaca cgatgccact     360
   gggaaagatg acttcaactc gttggatgca gcggtcaact actggataag catcaaagaa     420
   gcactgatcg ggaaggaaga tcgggttatt gtaaacattg caaacgagtg gtacggaaca     480
   tggaacggaa gcgcgtgggc tgacgggtac aaaaaagcta ttccgaaatt aagagatgcg     540
   ggtattaaaa ataccttgat tgtagatgca gcaggctggg gtcagtaccc tcaatcgatc     600
   gtcgattacg gacaaagcgt attcgccgcg gattcacaga aaaatacggc gttttccatt     660
   cacatgtatg agtatgcagg caaggatgcg gccaccgtca aatccaatat ggaaaatgtg     720
   ctgaataagg ggctggcctt aatcattggt gagttcggag gatatcacac caatggagat     780
   gtcgatgaat atgcaatcat gaaatatggt ctggaaaaag gggtaggatg gcttgcatgg      840
   tcttggtacg gtaatagctc tggattaaac tatcttgatt tggcaacagg acctaacggc     900
   agtttgacga gctatggtaa tacggttgtc aatgatactt acggaattaa aaatacgtcc     960
   caaaaagcgg gaatctttta a                                               981
<210> 44
   <211> 326
   <212> PRT
   <213> Bacillus circulans
<400> 44
   Met Ala Lys Leu Gln Lys Gly Thr Ile Leu Thr Val Ile Ala Ala Leu
   1               5                   10                  15
   Met Phe Val Ile Leu Gly Ser Ala Ala Pro Lys Ala Ala Ala Ala Thr
               20                  25                  30
   Gly Phe Tyr Val Asn Gly Gly Lys Leu Tyr Asp Ser Thr Gly Lys Pro
           35                  40                  45
   Phe Tyr Met Arg Gly Ile Asn His Gly His Ser Trp Phe Lys Asn Asp
       50                  55                  60
   Leu Asn Thr Ala Ile Pro Ala Ile Ala Lys Thr Gly Ala Asn Thr Val
   65                  70                  75                  80
   Arg Ile Val Leu Ser Asn Gly Thr Gln Tyr Thr Lys Asp Asp Leu Asn
                   85                  90                  95
   Ser Val Lys Asn Ile Ile Asn Val Val Asn Ala Asn Lys Met Ile Ala
               100                 105                 110
   Val Leu Glu Val His Asp Ala Thr Gly Lys Asp Asp Phe Asn Ser Leu
           115                 120                 125
   Asp Ala Ala Val Asn Tyr Trp Ile Ser Ile Lys Glu Ala Leu Ile Gly
       130                 135                 140
   Lys Glu Asp Arg Val Ile Val Asn Ile Ala Asn Glu Trp Tyr Gly Thr
   145                 150                 155                 160
   Trp Asn Gly Ser Ala Trp Ala Asp Gly Tyr Lys Lys Ala Ile Pro Lys
                   165                 170                 175
   Leu Arg Asp Ala Gly Ile Lys Asn Thr Leu Ile Val Asp Ala Ala Gly
               180                 185                 190
   Trp Gly Gln Tyr Pro Gln Ser Ile Val Asp Tyr Gly Gln Ser Val Phe
           195                 200                 205
   Ala Ala Asp Ser Gln Lys Asn Thr Ala Phe Ser Ile His Met Tyr Glu
       210                 215                 220
   Tyr Ala Gly Lys Asp Ala Ala Thr Val Lys Ser Asn Met Glu Asn Val
   225                 230                 235                 240
   Leu Asn Lys Gly Leu Ala Leu Ile Ile Gly Glu Phe Gly Gly Tyr His
                   245                 250                 255
   Thr Asn Gly Asp Val Asp Glu Tyr Ala Ile Met Lys Tyr Gly Leu Glu
               260                 265                 270
   Lys Gly Val Gly Trp Leu Ala Trp Ser Trp Tyr Gly Asn Ser Ser Gly
           275                 280                 285
   Leu Asn Tyr Leu Asp Leu Ala Thr Gly Pro Asn Gly Ser Leu Thr Ser
       290                 295                 300
   Tyr Gly Asn Thr Val Val Asn Asp Thr Tyr Gly Ile Lys Asn Thr Ser
   305                 310                 315                 320
   Gln Lys Ala Gly Ile Phe
                   325
<210> 45
   <211> 1110
   <212> DNA
   <213> Bacillus nealsonii
<400> 45
   atggttgtga aaaaattatc aagttttatt ctaattttac tgttagttac ttctgctttg 60
   tttattactg attcaaaagc aagtgctgct tcgggatttt atgtaagcgg taccacttta 120
   tatgatgcaa cgggtaaacc gtttactatg agaggtgtaa atcatgctca ttcttggttt 180
   aaagaagatt cagcagctgc tattccagca atagcagcaa ctggagcaaa cacagtaaga 240
   attgttttat ctgatggtgg acaatacacc aaagatgata ttaatactgt taaaagcctt 300
   ttgtcattgg cagaaaaaat aaacttgcat tctggagtca tgacgcacag aaaagacgat 360
   gtggaatctt taaatcgtgc agtcgattat tggatcagct taaaagacac attgataggc 420
   aaagaagata aagtgataat aaacattgcg aatgaatggt atggtacttg ggatggtgcg 480
   gcatgggcag ctggttataa acaagctatt ccaaagttac ggaatgcagg cttaaatcat 540
   actctaataa ttgattctgc tggatgggga caatacccag cttccattca taattatgga 600
   aaagaggtat ttaatgcgga tccattgaaa aatacaatgt tctccataca tatgtatgag 660
   tacgctggtg gggatgcagc aactgttaag tcaaatattg atggtgtctt aaaccaagga 720
   ttagctttaa taataggaga gtttggacaa aaacatacaa atggagatgt agatgaagca 780
   accatcatga gttattcaca gcaaaaaaat atcggttggc ttgcatggtc ttggaaagga 840
   aatagcacag attggagcta tctggattta agcaacgatt ggtctggtaa cagtttaact 900
   gattggggta atacggttgt taatggggca aatgggttaa aagccacttc aaaactaagc 960
   ggagtattcg gtagctcagc aggaacaaat aatatattgt atgattttga aagcggtaat 1020
   caaaactgga ctggatcaaa tatcgcgggt ggaccttgga acgaattcaa gcttgatatc 1080
   attcaggacg agcctcagac tccagcgtaa 1110
<210> 46
   <211> 369
   <212> PRT
   <213> Bacillus nealsonii
<400> 46
   Met Val Val Lys Lys Leu Ser Ser Phe Ile Leu Ile Leu Leu Leu Val
   1               5                   10                  15
   Thr Ser Ala Leu Phe Ile Thr Asp Ser Lys Ala Ser Ala Ala Ser Gly
               20                  25                  30
   Phe Tyr Val Ser Gly Thr Thr Leu Tyr Asp Ala Thr Gly Lys Pro Phe
           35                  40                  45
   Thr Met Arg Gly Val Asn His Ala His Ser Trp Phe Lys Glu Asp Ser
       50                  55                  60
   Ala Ala Ala Ile Pro Ala Ile Ala Ala Thr Gly Ala Asn Thr Val Arg
   65                  70                  75                  80
   Ile Val Leu Ser Asp Gly Gly Gln Tyr Thr Lys Asp Asp Ile Asn Thr
                   85                  90                  95
   Val Lys Ser Leu Leu Ser Leu Ala Glu Lys Ile Asn Leu His Ser Gly
               100                 105                 110
   Val Met Thr His Arg Lys Asp Asp Val Glu Ser Leu Asn Arg Ala Val
           115                 120                 125
   Asp Tyr Trp Ile Ser Leu Lys Asp Thr Leu Ile Gly Lys Glu Asp Lys
       130                 135                 140
   Val Ile Ile Asn Ile Ala Asn Glu Trp Tyr Gly Thr Trp Asp Gly Ala
   145                 150                 155                 160
   Ala Trp Ala Ala Gly Tyr Lys Gln Ala Ile Pro Lys Leu Arg Asn Ala
                   165                 170                 175
   Gly Leu Asn His Thr Leu Ile Ile Asp Ser Ala Gly Trp Gly Gln Tyr
               180                 185                 190
   Pro Ala Ser Ile His Asn Tyr Gly Lys Glu Val Phe Asn Ala Asp Pro
           195                 200                 205
   Leu Lys Asn Thr Met Phe Ser Ile His Met Tyr Glu Tyr Ala Gly Gly
       210                 215                 220
   Asp Ala Ala Thr Val Lys Ser Asn Ile Asp Gly Val Leu Asn Gln Gly
   225                 230                 235                 240
   Leu Ala Leu Ile Ile Gly Glu Phe Gly Gln Lys His Thr Asn Gly Asp
                   245                 250                 255
   Val Asp Glu Ala Thr Ile Met Ser Tyr Ser Gln Gln Lys Asn Ile Gly
               260                 265                 270
   Trp Leu Ala Trp Ser Trp Lys Gly Asn Ser Thr Asp Trp Ser Tyr Leu
           275                 280                 285
   Asp Leu Ser Asn Asp Trp Ser Gly Asn Ser Leu Thr Asp Trp Gly Asn
       290                 295                 300
   Thr Val Val Asn Gly Ala Asn Gly Leu Lys Ala Thr Ser Lys Leu Ser
   305                 310                 315                 320
   Gly Val Phe Gly Ser Ser Ala Gly Thr Asn Asn Ile Leu Tyr Asp Phe
                   325                 330                 335
   Glu Ser Gly Asn Gln Asn Trp Thr Gly Ser Asn Ile Ala Gly Gly Pro
               340                 345                 350
   Trp Asn Glu Phe Lys Leu Asp Ile Ile Gln Asp Glu Pro Gln Thr Pro
           355                 360                 365
   Ala
<210> 47
   <211> 984
   <212> DNA
   <213> Bacillus circulans
<400> 47
   atgatgttga tatggatgca gggatggaag tctattctag tcgcgatctt ggcgtgtgtg 60
   tcagtaggcg gtgggcttcc tagtccagaa gcagccacag gattttatgt aaacggtacc 120
   aagctgtatg attcaacggg caaggccttt gtgatgaggg gtgtaaatca tccccacacc 180
   tggtacaaga atgatctgaa cgcggctatt ccggctatcg cgcaaacggg agccaatacc 240
   gtacgagtcg tcttgtcgaa cgggtcgcaa tggaccaagg atgacctgaa ctccgtcaac 300
   agtatcatct cgctggtgtc gcagcatcaa atgatagccg ttctggaggt gcatgatgcg 360
   acaggcaaag atgagtatgc ttcccttgaa gcggccgtcg actattggat cagcatcaaa 420
   ggggcattga tcggaaaaga agaccgcgtc atcgtcaata ttgctaatga atggtatgga 480
   aattggaaca gcagcggatg ggccgatggt tataagcagg ccattcccaa attaagaaac 540
   gcgggcatta agaatacgtt gatcgttgat gcagcgggat gggggcaata cccgcaatcc 600
   atcgtggatg agggggccgc ggtatttgct tccgatcaac tgaagaatac ggtattctcc 660
   atccatatgt atgagtatgc cggtaaggat gccgctacgg tgaaaacgaa tatggacgat 720
   gttttaaaca aaggattgcc tttaatcatt ggggagttcg gcggctatca tcaaggtgcc 780
   gatgtcgatg agattgctat tatgaagtac ggacagcaga aggaagtggg ctggctggct 840
   tggtcctggt acggaaacag cccggagctg aacgatttgg atctggctgc agggccaagc 900
   ggaaacctga ccggctgggg aaacacggtg gttcatggaa ccgacgggat tcagcaaacc 960
   tccaagaaag cgggcattta ttaa 984
<210> 48
   <211> 327
   <212> PRT
   <213> Bacillus circulans
<400> 48
   Met Met Leu Ile Trp Met Gln Gly Trp Lys Ser Ile Leu Val Ala Ile
   1               5                   10                  15
   Leu Ala Cys Val Ser Val Gly Gly Gly Leu Pro Ser Pro Glu Ala Ala
               20                  25                  30
   Thr Gly Phe Tyr Val Asn Gly Thr Lys Leu Tyr Asp Ser Thr Gly Lys
           35                  40                  45
   Ala Phe Val Met Arg Gly Val Asn His Pro His Thr Trp Tyr Lys Asn
       50                  55                  60
   Asp Leu Asn Ala Ala Ile Pro Ala Ile Ala Gln Thr Gly Ala Asn Thr
   65                  70                  75                  80
   Val Arg Val Val Leu Ser Asn Gly Ser Gln Trp Thr Lys Asp Asp Leu
                   85                  90                  95
   Asn Ser Val Asn Ser Ile Ile Ser Leu Val Ser Gln His Gln Met Ile
               100                 105                 110
   Ala Val Leu Glu Val His Asp Ala Thr Gly Lys Asp Glu Tyr Ala Ser
           115                 120                 125
   Leu Glu Ala Ala Val Asp Tyr Trp Ile Ser Ile Lys Gly Ala Leu Ile
       130                 135                 140
   Gly Lys Glu Asp Arg Val Ile Val Asn Ile Ala Asn Glu Trp Tyr Gly
   145                 150                 155                 160
   Asn Trp Asn Ser Ser Gly Trp Ala Asp Gly Tyr Lys Gln Ala Ile Pro
                   165                 170                 175
   Lys Leu Arg Asn Ala Gly Ile Lys Asn Thr Leu Ile Val Asp Ala Ala
               180                 185                 190
   Gly Trp Gly Gln Tyr Pro Gln Ser Ile Val Asp Glu Gly Ala Ala Val
           195                 200                 205
   Phe Ala Ser Asp Gln Leu Lys Asn Thr Val Phe Ser Ile His Met Tyr
       210                 215                 220
   Glu Tyr Ala Gly Lys Asp Ala Ala Thr Val Lys Thr Asn Met Asp Asp
   225                 230                 235                 240
   Val Leu Asn Lys Gly Leu Pro Leu Ile Ile Gly Glu Phe Gly Gly Tyr
                   245                 250                 255
   His Gln Gly Ala Asp Val Asp Glu Ile Ala Ile Met Lys Tyr Gly Gln
               260                 265                 270
   Gln Lys Glu Val Gly Trp Leu Ala Trp Ser Trp Tyr Gly Asn Ser Pro
           275                 280                 285
   Glu Leu Asn Asp Leu Asp Leu Ala Ala Gly Pro Ser Gly Asn Leu Thr
       290                 295                 300
   Gly Trp Gly Asn Thr Val Val His Gly Thr Asp Gly Ile Gln Gln Thr
   305                 310                 315                 320
   Ser Lys Lys Ala Gly Ile Tyr
                   325